# EUROPEAN PATENT APPLICATION

(11) **EP 4 841 263 A2**
(43) Date of publication of application: **18.11.2026**
(21) Application number: 26194400.3
(22) Date of filing: 06.10.2021
(51) Int. Cl.: G01N 33/68

(54) **METHODS, COMPOSITIONS, KITS AND SYSTEMS FOR SIGNAL-ON DETECTION OF NEUTRALIZATION TARGETS**

(30) Priority: 06.10.2020 US 202063087960 P; 09.04.2021 US 202163173039 P; 05.10.2021 US 202163252545 P
(62) Document number(s) of the earlier application(s) in accordance with Art. 76 EPC: 21878457.7 / 4 225 946
(71) Applicant: President And Fellows Of Harvard College, Cambridge, Massachusetts 02138 (US)
(72) Inventor: XUAN, Feng, Cambridge, 02138 (US); WANG, Yu, Cambridge, 02138 (US); YIN, Peng, Cambridge, 02138 (US); FAN, Tsz Wing, Cambridge, 02138 (US)
(74) Representative: Keltie LLP

(57) **Abstract**

Provided herein are methods, compositions, kits, and systems for the detection of neutralization targets such as neutralizing antibodies.

## Description

### CROSS-REFERENCE TO RELATED APPLICATIONS

This application claims benefit under 35 U.S.C. § 119(e) of U.S. Provisional Application No. 63/087,960 filed October 6, 2020, U.S. Provisional Application No. 63/173,039 filed April 9, 2021, and U.S. Provisional Application No. 63/252,545 filed October 5, 2021, the contents of each of which are incorporated herein by reference in their entireties.

### TECHNICAL FIELD

The present disclosure relates to methods, compositions, kits and systems for detection of target such as neutralizing antibodies.

### BACKGROUND

Neutralizing antibodies represent one important category of antibody therapeutics, which are intended to alter the bioactivity of target components through forming antigen-antibody complexes. In immunogenicity test, the presence of neutralizing antibodies, or anti-drug antibodies (ADA) often leads to abnormal PK behaviors or efficacy loss of corresponding drugs. On the other hand, antibodies that are capable of neutralizing harmful substances, i.e. viruses and cytokines, can be of great therapeutic potential. Neutralizing antibodies are also important indicators in vaccine evaluation for immunity and resistance to viruses. Protective levels of neutralizing antibodies have been established for many viruses such as dengue, yellow fever and Japanese encephalitis. The current existing methods including functional cell-based assays and competitive ligand binding assays all have limited performance on sensitivity. Thus, there remains a need in the art for methods and compositions for detecting targets such as neutralizing antibodies.

### SUMMARY

Provided herein are methods, compositions and kits for detecting targets, for example neutralizing antibodies from a sample, such as a biological sample. In one aspect, provided here are generalized methods to sensitively detect neutralizing antibodies (against a virus, cytokine, interleukins or other proteins). The methods comprise labeling recombinant proteins and antibodies with specially designed nucleic acid probes which only respond to neutralizing antibody and generating an amplifiable nucleic acid molecule. In principle, the methods, compositions and kits described herein eliminate or inhibit the signal from binding antibodies and can quantify the neutralizing antibody concentration through qPCR or high-throughput sequencing.

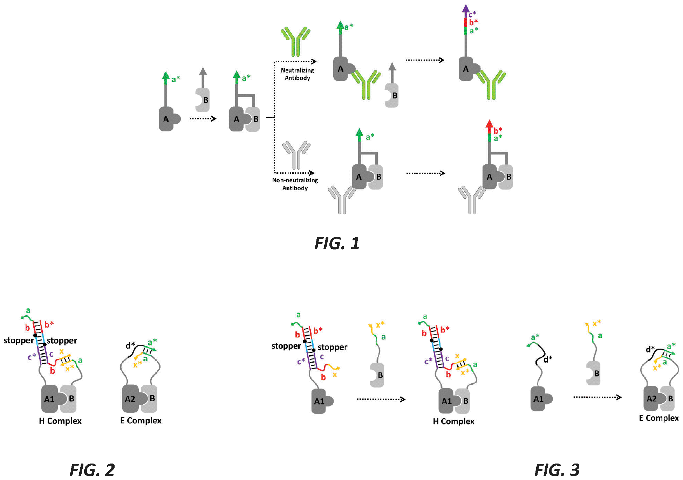

Principles of programming nucleic acid extension to differentiate neutralizing binding event vs non-neutralizing binding event is schematically shown in **FIG. 1**. A pair of binding molecules, e.g., receptor-ligand pair is conjugated with specially-design nucleic acid molecules. When one member of the pair binds with the target, e.g., one member is neutralized by binding to a target, e.g., antibody or other biomolecules, the nucleic acid molecules conjugated to the binding pair are generally prevented from binding to each other. The nucleic acid molecule conjugated to the binding pair member (A) bound to the target can be extended twice or multiple times on an end with a portion of the sequence (a*) of the same nucleic acid molecule, to generate a nucleic acid record, e.g., "target" sequence c*. In contrast, in the absence of the target, the binding pair member A is not "neutralized" and can still bind to the binding pair member B. In this situation, only intermediate sequences (b*) can be extended to a* due to the "interference" of the nucleic acid sequence conjugated on B. Thus, a unique sequence is generated only when the binding pair interaction, e.g., receptor-ligand interaction is disrupted by the presence of the target (e.g., a neutralizing antibody), and in some embodiments over different experimental steps, the generated sequence can be an amplified and quantified by qPCR or next generation sequencing. Targets (e.g., binding antibodies) that are not capable of disrupting the binding pair interaction (e.g., disrupting receptor-ligand interaction) would not generate such unique nucleic acid sequences.

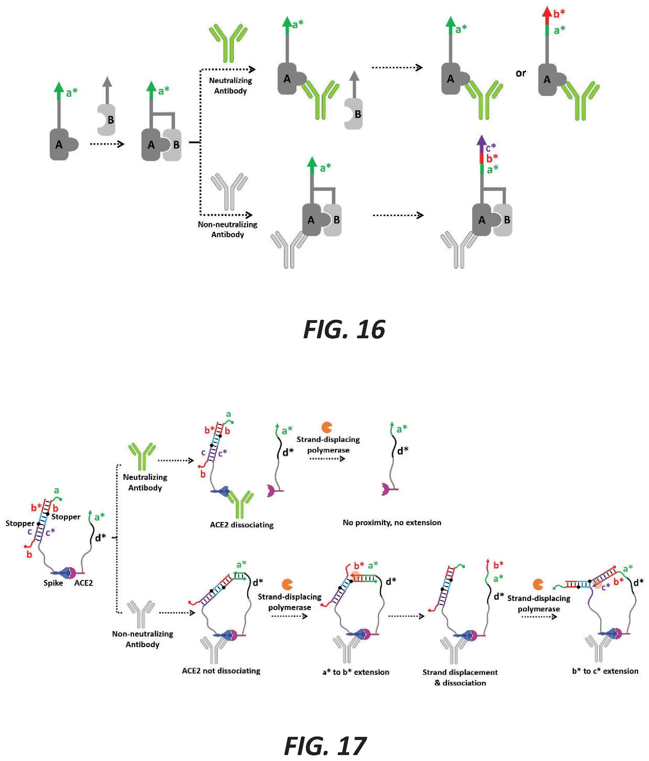

Another principle of programming nucleic acid extension to differentiate neutralizing binding event vs non-neutralizing binding event is schematically shown in **FIG. 16****.** A pair of binding molecules, e.g., receptor-ligand pair is conjugated with specially-design nucleic acid molecules. When one member of the pair binds with the target, e.g., one member is neutralized by binding to a target, e.g., antibody or other biomolecules, the nucleic acid molecules conjugated to the binding pair are generally prevented from binding to each other. In the presence of the target, the binding pair member A is "neutralized" and cannot bind to the binding pair member B. In this situation, a* is generally prevented from being extended or only intermediate sequences (b*) can generally be extended to a* due to the "interference" of the nucleic acid sequence conjugated on B. In contrast, in the absence of the target, the nucleic acid molecule conjugated to the binding pair member (A) can be extended twice or multiple times on an end with a portion of the sequence (a*) of the same nucleic acid molecule, to generate a nucleic acid record, e.g., "target" sequence c*. Thus, a unique sequence is generated only when the binding pair interaction, e.g., receptor-ligand interaction is not disrupted by the presence of the target (e.g., a neutralizing antibody), and in some embodiments over different experimental steps, the generated sequence can be an amplified and quantified by qPCR or next generation sequencing. Targets (e.g., binding antibodies) that are not capable of disrupting the binding pair interaction (e.g., disrupting receptor-ligand interaction) generate such unique nucleic acid sequences.

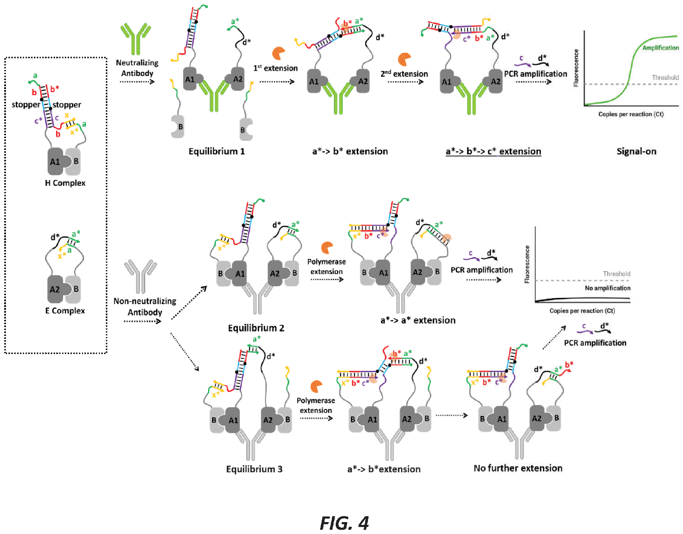

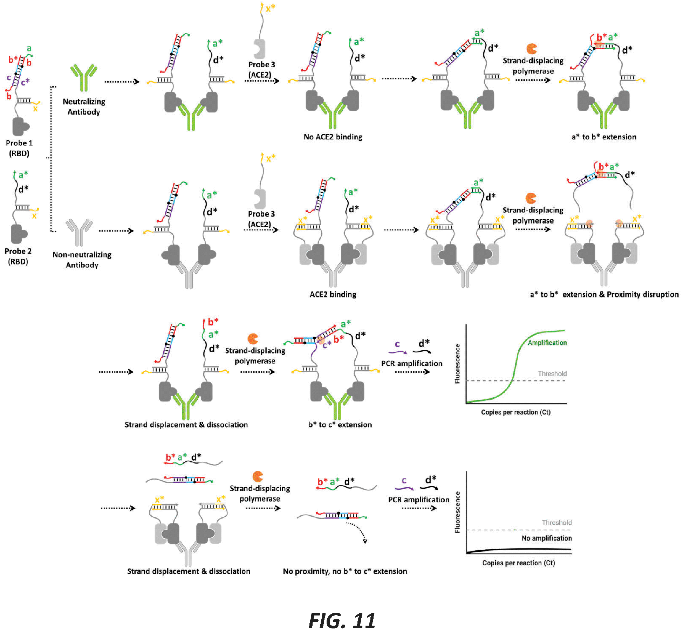

In some embodiments, the methods of the present disclosure use differences in interaction times between binding and non-binding moieties to generate a nucleic acid record molecule only when a pair of reporter probes recognize the same target (**FIGS. 4** **and** **11**).

In some other embodiments, the methods of the present disclosure use differences in interaction times between binding and non-binding moieties to generate a nucleic acid record molecule only in absence of the target (**FIG. 17**).

In some embodiments, the methods disclosed herein make use of a strand displacement mechanism to generate a nucleic acid record molecule only when the first and second reporter probes are brought into close proximity to each other in presence of the target. *See*, for example, **FIGS. 4****,** **11** **and** **16**. "Strand displacement" refers to the mechanism by which two nucleic acid strands with identical sequences, when proximate to a single complementary nucleic acid strand (or segment of a strand), undergo relatively rapid (e.g. , timescale < 1s) competition for that complement strand, 'displacing' each other from the complement presumably by a 'random-walk' mechanism (see, e.g., Yurke et al, Nature 406: 605-608, 2000; and Zhang et al. Nature Chemistry 3: 103-113, 2011, content of each of which is incorporated by reference herein).

Generating the nucleic acid record molecule only in the presence of the target can eliminate the signal from reporter probes and can quantify the target concentration through qPCR or high-throughput sequencing. Generally, the method comprises contacting the sample with a pair of reporter probes conjugated with specially designed nucleic acids. The nucleic acids from the two reporter probes interact with each other in presence of the target generating a nucleic acid record which could be detected, e.g., amplified. *See* **FIGS. 4** **and** **11**.

In some other embodiments, the method comprises contacting the sample with a pair of reporter probe and a blocking probe conjugated with specially designed nucleic acids. The nucleic acids from the reporter probe and the blocking probe interact with each other in absence of the target generating a nucleic acid record which could be detected, e.g., amplified. *See* **FIGS. 16 and 17**.

The methods, compositions and kits include at least a first reporter probe and a blocking probe, and optionally a second reporter probe.

In some embodiments of any one of the aspects, the first reporter probe comprises a first target binding ligand capable of binding with the target. The first target binding ligand is linked to a specially designed double-stranded nucleic acid comprising single-stranded toehold and/or pairing domains at both ends. For example, each strand of the double-stranded nucleic acid comprises at least one single-stranded toehold and/or pairing domains on at least one of its ends. Further each strand comprises a molecule or modification capable of stopping synthesis of a complementary strand by a polymerase.

In some embodiments of any one of the aspects, the second reporter probe comprises a target binding ligand capable of binding with the target. The second reporter probe comprises a second nucleic acid linked to the second target binding ligand and comprising a region or domain that is substantially complementary to one of the single-stranded toehold/pairing domain of the first nucleic acid of the first reporter probe. In some embodiments of any one of the aspects, the second nucleic acid of the second reporter probe further comprises a primer binding domain.

In some embodiments of any one of the aspects, the second reporter probe comprises a double-stranded nucleic acid comprising single-stranded toehold and/or pairing domains at both ends, wherein one of the toehold and/or pairing domain is substantially complementary to one of the single-stranded toehold/pairing domain of the double-stranded nucleic acid of the first reporter probe, and the other of the toehold and/or pairing domain is substantially identical to the other of the single-stranded toehold/pairing domain of the double-stranded nucleic acid of the first reporter probe. In some further embodiments, one of the strands of the double-stranded nucleic acid comprises a primer binding domain.

In some embodiments of any one of the aspects, the blocking probe comprises a blocking ligand capable of binding or forming a complex, directly or indirectly with the first target binding ligand of the first reporter probe and/or the second target binding ligand of the second reporter probe. In other words, the blocking probe is capable of forming a complex with the first report probe and/or the second reporter probe by interactions between the first and/or second target binding ligand and the blocking ligand. Binding of the target to the first report probe inhibits formation of the complex between the first reporter probe and the blocking probe. Similarly, binding of the target to the second report probe inhibits formation of the complex between the second reporter probe and the blocking probe.

Like the reporter probes, the blocking probe comprises a specifically designed blocking probe nucleic acid linked to the blocking ligand and comprising a region or domain that is substantially complementary to one of the single-stranded toehold/pairing domain of the first nucleic acid of the first reporter probe. In some embodiments, the blocking probe nucleic acid of the blocking probe can further comprise a second toehold or pairing domain. The second toehold or pairing domain can comprise a nucleotide sequence substantially identical to the other single-stranded toehold/pairing domain of the first nucleic acid of the first reporter probe.

In some embodiments of any one of the aspects, the first target binding ligand of the first reporter probe and the second target binding ligand of the second reporter probe are the same, i.e., the same molecule/moiety but linked to different nucleic acids.

The first target binding ligand of the first reporter probe and the target binding ligand of the second reporter probe can bind to the same target. For example, the target comprises a binding site for the first target binding ligand of the first reporter probe and the second target binding ligand of the second reporter probe. In other words, the first and second reporter probes can bind to the same target simultaneously.

In some embodiments of any one of the aspects, the first target binding ligand of the first reporter probe and the second target binding ligand of the second reporter probe are different.

In some embodiments of any one of the aspects, the second target binding ligand of the second reporter probe is an antibody. For example, the second target binding ligand of the second reporter probe is a class specific antibody.

In some embodiments of any one of the aspects, one of the single-stranded toehold/pairing domain of the first nucleic acid of the first reporter probe is complementary to the single-stranded toehold/pairing domain of the blocking probe nucleic acid of the blocking probe and the second of the single-stranded toehold/pairing domain of the first nucleic acid of the first reporter probe is complementary to the single-stranded toehold/pairing domain of the second nucleic acid strand of the second reporter probe.

### Design 1

In some embodiments of any one of the aspects, the double-stranded nucleic acid of the first reporter probe comprises: (1) a first nucleic acid first strand linked to the first target binding ligand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second nucleic acid strand comprises a first subdomain (b*) linked to a second subdomain that is linked to a stopper molecule (•) that is linked to a primer domain (c) that is linked to a first toehold domain (b) that is linked to a second toehold domain (x). The primer binding domain (c*) of the first strand and the primer domain (c) of the second strand are substantially complementary to each other. The first subdomain of the first strand and the second subdomain of the second strand are substantially complementary to each other. The second subdomain (b) of the first strand and the first subdomain (b*) of the second strand are substantially complementary to each other. Further, the first subdomain (b*) and the first toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•).

In some embodiments of any one of the aspects, the blocking probe nucleic acid of the blocking probe comprises a first toehold domain (a) that is linked to a second toehold domain (x*). The toehold domain (x*) is substantially complementary with the second toehold domain (x) of the double-stranded nucleic acid of the first reporter probe. The first toehold domain (a) is substantially identical to the toehold domain (a) of the double-stranded nucleic acid of the first reporter probe.

In some embodiments of any one of the aspects, the second nucleic acid of the second reporter probe comprises a primer binding domain (d*) linked to a toehold domain (a*). The toehold domain (a*) is substantially complementary to the toehold domain (a) of the double-stranded nucleic acid strand of the first reporter probe and/or to the first toehold domain (a) of the blocking probe nucleic acid of the blocking probe.

### Design 2

In some embodiments of any one of the aspects, the double-stranded nucleic acid of the first reporter probe comprises: (1) a first nucleic acid first strand linked to the first target binding ligand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second nucleic acid strand comprises a first subdomain (b*) linked to a second subdomain that is linked to a stopper molecule (•) that is linked to a primer domain (c) that is linked to a first toehold domain (b) that is linked to a second toehold domain (x). The primer binding domain (c*) of the first strand and the primer domain (c) of the second strand are substantially complementary to each other. The first subdomain of the first strand and the second subdomain and second strand are substantially complementary to each other. The second subdomain (b) of the first strand and the first subdomain (b*) of the second strand are substantially complementary to each other. Further, the first subdomain (b*) and the first toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•).

In some embodiments of any one of the aspects, the blocking probe nucleic acid of the blocking probe comprises a toehold domain (x*). The toehold domain (x*) is substantially complementary with the second toehold domain (x) of the double-stranded nucleic acid of the first reporter probe. The blocking probe nucleic acid of the blocking probe does not comprise a domain or region that is substantially identical to the toe-hold domain (a) of the first nucleic acid of the first reporter probe.

In some embodiments of any one of the aspects, the second reporter probe comprises: (1) a second nucleic acid first strand linked to the target binding ligand and (b) a second nucleic acid second strand substantially complementary to the first strand, forming a double-stranded region (duplex region) with the first strand. The first strand comprises a toehold domain (x). The toehold domain (x) is substantially identical to the second toehold domain (x) of the double-stranded nucleic acid of the first reporter probe. It is noted that the toehold domain (x) is substantially complementary to the toehold domain (x*) of the blocking probe. The second reporter probe further comprises (2) a second strand comprises a primer binding domain (d*) linked to a toehold domain (a*). The toehold domain (a*) is substantially complementary to the toehold domain (a) of the double-stranded nucleic acid of the first reporter probe.

### Design 3

In some embodiments of any one of the aspects, the first reporter probe comprises a double stranded nucleic acid hybridized at one end to a linking strand linked to the first target binding ligand. The linking strand linked to the first target binding ligand comprises a first nucleic acid first hybridizing domain for hybridizing with the double-stranded nucleic acid and a toehold domain (x) linked to the first nucleic acid first hybridizing domain. The double-stranded nucleic acid comprises: (1) a first nucleic acid first strand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second strand comprises a first subdomain (b*) linked to a second subdomain that is linked to a stopper molecule (•) that is linked to a primer domain (c) that is linked to a toehold domain (b). The primer binding domain (c*) of the first strand and the primer domain (c) of the second strand are substantially complementary to each other. The first subdomain of the first strand and the second subdomain and second strand are substantially complementary to each other. The second subdomain (b) of the first strand and the first subdomain (b*) of the second strand are substantially complementary to each other. Further, the first subdomain (b*) and the toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•). The primer binding domain (c*) is linked to a first nucleic acid second hybridizing domain for hybridizing with the first nucleic acid first hybridizing domain of the linking strand linked to the molecule/moiety capable of binding with the target (target binding ligand).

In some embodiments of any one of the aspects, the blocking probe nucleic acid of the blocking probe comprises a toehold domain (x*). The toehold domain (x) is substantially complementary with the toehold domain (x*) of the nucleic acid strand linked to the target binding ligand. The blocking probe nucleic acid of the blocking probe does not comprise a domain or region that is substantially identical to the toehold domain (a) of the nucleic acid of the first reporter probe.

In some embodiments of any one of the aspects, the second nucleic acid of the second reporter probe comprises: (1) a second nucleic acid first strand linked to the molecule/moiety capable of binding with the target (the second target binding ligand) and (2) a second nucleic acid second strand substantially complementary to the first strand, forming a double-stranded region (duplex region) with the first strand. The first strand comprises a toehold domain (x). The toehold domain (x) is substantially identical to the toehold domain (x) of the first reporter probe. It is noted that the toehold domain (x) is substantially complementary to the toehold domain (x*) of the blocking probe. The second strand comprises a primer binding domain (d*) linked to a toehold domain (a*). The toehold domain (a*) is substantially complementary to the toehold domain (a) of the double-stranded nucleic acid of the first reporter probe.

### Design 4 (Signal off)

In some embodiments of any one of the aspects, the double-stranded nucleic acid of the first reporter probe comprises: (1) a first nucleic acid first strand linked to the target binding ligand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second strand comprises a first subdomain (b*) linked to a second subdomain that is linked to a stopper molecule (•) that is linked to a primer domain (c) that is linked to a toehold domain (b). The primer binding domain (c*) of the first strand and the primer domain (c) of the second strand are substantially complementary to each other. The first subdomain of the first strand and the second subdomain and second strand are substantially complementary to each other. The second subdomain (b) of the first strand and the first subdomain (b*) of the second strand are substantially complementary to each other. Further, the first subdomain (b*) and the toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•).

In some embodiments of any one of the aspects, the blocking probe nucleic acid of the blocking probe comprises a primer binding domain (d*) linked to a toehold domain (a*). The toehold domain (a*) is substantially complementary to the toehold domain (a) of the double-stranded nucleic acid of the first reporter probe

### Method

In another aspect, the disclosure provides a method for detecting a target in a sample. Generally, the method comprises contacting the sample with at least a first reporter probe and a blocking probe described herein. In some embodiments, the method comprises contacting the sample with a first reporter probe, a second reporter probe, and a blocking probe described herein. As disclosed herein, a signal, i.e., a nucleic acid record is produced only in the presence of the target. As such, the detection is in a signal-on modality.

It is noted that the first reporter probe and the blocking probe can be formed into a complex prior to contacting with the sample. Alternatively, or in addition, the first reporter probe and the blocking probe can be contacted with the sample individually. For example, the sample can be contacted with the first reporter probe followed by contacting with the blocking probe.

Similarly, the second reporter probe and the blocking probe can be formed into a complex prior to contacting with the sample. Alternatively, or in addition, the second reporter probe and the blocking probe can be contacted with the sample individually. For example, the sample can be contacted with the second reporter probe followed by contacting with the blocking probe.

In some embodiments, the sample is contacted with the first and second reporter probes prior to contacting with the blocking probe.

One exemplary strategy for detecting a target is depicted in **FIGS. 4** **and** **11**. When the first and second reporter probes are co-bound to the target, a toehold/pairing domain in the first nucleic acid of the first reporter probe hybridizes with a complementary toehold/pairing domain in the second nucleic acid of the second reporter probe. A polymerase, e.g., a strand displacing polymerase extends the hybridized strands until it reaches a stopper molecule. This extension results in in a newly formed region or domain appended to the second nucleic acid of the second reporter probe. The double-stranded nucleic acid undergoes strand displacement whereby the newly formed region or domain appended to the second nucleic acid of the second reporter probe hybridizes with a complementary region on the other end of the double-stranded region. The polymerase, e.g., a strand displacing polymerase extends the newly hybridized strands until it reaches the second stopper molecule. The resulting nucleic acid comprises a sequence substantially identical to a part of the sequence in the double-stranded nucleic acid of the first reporter probe on one end and a sequence substantially identical to a part of the strand in the second reporter probe on the other end.

In **FIG 4**, in the absence of a target, the first and second reporter probes each remain bound to the blocking probe. The polymerase extends along the strands until it reaches a stopper molecule. The extended nucleic acid strands may separate from each other. However, the separated extended strands from the first and second reporter probes do not hybridize to each other. This deactivates the reporter probes since they are no longer useable for detection. Thus, through hybridization, extension, and strand-displacement mechanisms, a record of interaction between the nucleic acids of the first reporter probe and the second reporter probe is produced only in the presence of the target.

**In** **FIG. 11**, in the absence of a target, the polymerase extends along the strands linked to the blocking ligands. This releases the interacting strands from the first and second reporter probes. However, the released strands from the first reporter probe and the second reporter probe do not hybridize to each other. This deactivates the reporter probes since they are no longer useable for detection. As such, a record of interaction between the nucleic acids of the first reporter probe and the second reporter probe is produced only in the presence of the target through hybridization, extension, and strand-displacement mechanisms.

In another aspect, provided herein is a composition. Generally, the composition comprises at least one of a first reporter probe, a second reporter probe, and a blocking probe described herein. In some embodiments of any one of the aspects, the composition comprises at least two of a first reporter probe, a second reporter probe, and a blocking probe described herein.

In yet another aspect, provided herein is a kit for detecting a target. The kit comprises at least one of a first reporter probe, a second reporter probe, and a blocking probe described herein. In some embodiments of any one of the aspects, the composition comprises at least two of a first reporter probe, a second reporter probe, and a blocking probe described herein.

Another exemplary strategy for detecting a target is depicted in **FIG. 17**. In the absence of the target, the reporter probe and the blocking probe are bound to each other. A toehold/pairing domain in the nucleic acid of the first and/or second reporter probe hybridizes with a complementary toehold/pairing domain in the blocking probe nucleic acid of the blocking probe. A polymerase, e.g., a strand displacing polymerase extends the hybridized strands until it reaches a stopper molecule. This extension results in a newly formed region or domain appended to the blocking probe nucleic acid of the blocking probe. The double-stranded nucleic acid undergoes strand displacement whereby the newly formed region or domain appended to the nucleic acid of the blocking probe hybridizes with a complementary region on the other end of the double-stranded region. The polymerase, e.g., a strand displacing polymerase extends the newly hybridized strands until it reaches the second stopper molecule. The resulting nucleic acid comprises a sequence substantially identical to a part of the sequence in the double-stranded nucleic acid of the first reporter probe on one end and a sequence substantially identical to a part of the blocking probe nucleic acid on the other end. In the presence of the target, the reporter probes and blocking probe are not bound to each other. This deactivates the reporter probes since they are no longer useable for detection. Thus, through hybridization, extension, and strand-displacement mechanisms, a record of interaction, i.e., a nucleic acid record, between the nucleic acids of the first reporter probe and the blocking probe is produced only in the absence of the target.

### Design 5

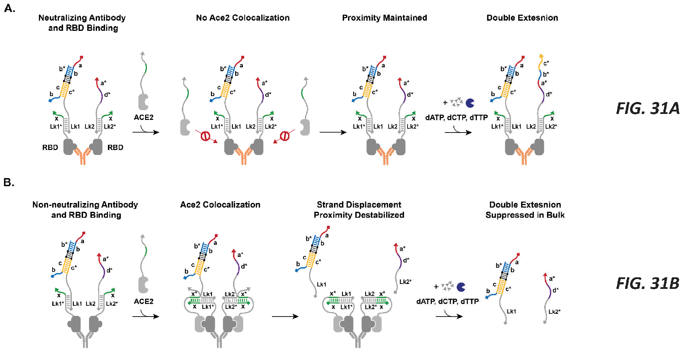

An example of programming nucleic acid extension to differentiate neutralizing binding event vs non-neutralizing binding event with non-enzymatic strand displacement is schematically shown in **FIGS. 31A and 31B**. In **FIG. 31A****,** a neutralizing target, e.g., neutralizing antibody is allowed to incubate with a pair of reporter probes, e.g., RBDs, conjugated with specially-design nucleic acid molecules, in equilibrium and their binding brings the reporter probes into proximity. A blocking probe, e.g., ACE2 conjugated with another specially-design nucleic acid molecule is sequentially added to bind with reporter probes, e.g., RBDs. However, the blocking probe, e.g., ACE2 which binds at the same location on the reporter probes, e.g., RBDs as the target, e.g., neutralizing antibody, optionally at a lower affinity, is generally unable to colocalize with reporter probes, e.g., RBDs which are already bound to the target. Proximity of the two reporter probes is maintained and allows double-extension of the nucleic acid strand comprising the toehold domain (a*) and the primer domain (d*) to generate a nucleic acid reporter comprising the domains (d*), (a*), (b*) and (c*) by the addition of polymerase. In contrast, as shown in **FIG. 31B**, with a non-neutralizing target, the reporter probes are not "neutralized" and can still bind to the blocking probe. The colocalization of the reporter probes with the blocking probes allows the toehold domain (x*) of the blocking probe to bind to the complementary toehold domain (x) of linking strand of the reporter probes. This triggers a toehold mediated strand displacement reaction by hybridization of the hybridizing domain (Lk1) of the blocking probe to the hybridizing domain (Lk1*) of the first reporter probe, and/or hybridization of the hybridizing domain (Lk2) of the blocking probe to the hybridizing domain (Lk2*) of the second reporter probe. This dissociates the nucleic acids from the reporter probes and the generation of a nucleic acid record from double extension is generally prevented. Thus, the nucleic acid record is generated only when the binding between the reporter probes and the blocking probes, e.g., RBD-ACE2 interaction is disrupted by the presence of the target (e.g., a neutralizing antibody).

In some embodiments of any one of the aspects, the method comprises: (A) contacting the sample with a first reporter probe and a second reporter probe; (B) contacting the sample with a first blocking probe and a second blocking probe; (C) providing a polymerase, thereby producing a nucleic acid record of an interaction between the first nucleic acid and the second nucleic acid if said interaction is present; and (D) detecting the presence or absence of the nucleic acid record, and wherein the presence of the nucleic acid record indicates the target is present in the sample, wherein: (i) the first reporter probe comprises a first target binding ligand linked to a first nucleic acid and wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises: (1) a first nucleic acid first strand hybridized to the linking strand linked to the first target binding ligand and comprising a hybridizing domain (Lk1) linked to a single-stranded toehold domain (a), wherein the toehold domain (a) is at an end distal from the hybridizing domain (Lk1) and wherein the hybridizinghybridizing domain (Lk1) is substantially complementary to the hybridizinghybridizing domain (Lk1*); and (2) a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; (ii) the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe, wherein a portion of the first nucleic acid and a portion of the second nucleic acid are capable of hybridizing in the presence of the target; (iii) the first blocking probe comprises a first blocking ligand capable of directly or indirectly forming a complex with the first target binding ligand and linked to a first blocking probe nucleic acid and wherein the first blocking probe nucleic acid comprises a toehold domain (x*) that is linked to a hybridizinghybridizing domain (Lk1), where the toehold domain (x*) is substantially complementary to the toehold domain (x) of the first reporter probe, where the hybridizinghybridizing domain (Lk1) is substantially complementary to the hybridizinghybridizing domain (Lk1*) of the first reporter probe, and wherein binding of the target to the first reporter probe inhibits the first reporter probe and the first blocking probe from forming a complex; and (iv) the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a hybridizinghybridizing domain (Lk2), where the toehold domain (x*) is substantially complementary to the toehold domain (x) of the second reporter probe, where the hybridizinghybridizing domain (Lk2) is substantially complementary to the hybridizinghybridizing domain (Lk2*) of the second reporter probe, and wherein binding of the target to the second reporter probe inhibits the second reporter probe and the second blocking probe from forming a complex.

It is noted that the steps of (A) contacting the sample with a first reporter probe and a second reporter probe and (B) contacting the sample with a first blocking probe and a second blocking probe can be sequential or simultaneous. For example, the sample can be contacted first with the first reporter probe and the second reporter probe followed by contacting with the first blocking probe and the second blocking probe. In other words, the first reporter probe and the second reporter probe can be added to the sample and after a period of time, e.g., 5 seconds, 10 second, 15 seconds, 20 seconds, 25 seconds, 30 seconds, 35 second, 40 second, 45 seconds, 50seconds, 55 seconds, 1 minute, 2 minutes, 3 minutes, 4 minutes, 5 minutes, 10 minutes, 15 minutes, 20 minutes, 25 minutes, 30 minutes, 35 minutes, 40 minutes, 45 minutes, 50 minutes, 55 minutes or more after the first blocking probe and the second blocking probe can be added to the sample.

In some embodiments of any one of the aspects, the first reporter probe comprises a double stranded nucleic acid hybridized at one end to a linking strand linked to a molecule/moiety capable of binding with the target (e.g., the first target binding ligand). The linking strand linked to the first target binding ligand comprises a first nucleic acid first hybridizing domain (Lk1*) for hybridizing with the double-stranded nucleic acid and a toehold domain (x) linked to the first nucleic acid first hybridizing domain (Lk1*). The double-stranded nucleic acid comprises: (1) a first nucleic acid first strand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second strand comprises a first subdomain (b*) linked to a second subdomain that is linked to a stopper molecule (•) that is linked to a primer domain (c) that is linked to a toehold domain (b). The primer binding domain (c*) of the first strand and the primer domain (c) of the second strand are substantially complementary to each other. The first subdomain of the first strand and the second subdomain and second strand are substantially complementary to each other. The second subdomain (b) of the first strand and the first subdomain (b*) of the second strand are substantially complementary to each other. Further, the first subdomain (b*) and the toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•). The primer binding domain (c*) is linked to a first nucleic acid second hybridizing domain (Lk1) for hybridizing with the first nucleic acid first hybridizing domain (Lk1*) of the linking strand linked the first target binding ligand.

In some embodiments of any one of the aspects, the first blocking probe comprises a blocking ligand (e.g., a first blocking ligand) capable of directly or indirectly forming a complex with the target binding ligand of the first reporter probe and linked to a first blocking probe nucleic acid. The first blocking probe nucleic acid comprises a toehold domain (x*) that is linked to a first blocking probe hybridizing domain (Lk1). The toehold domain (x*) of the first blocking probe nucleic acid is substantially complementary to the toehold domain (x) of the first reporter probe. The first blocking probe hybridizing domain (Lk1) is substantially complementary to the hybridizing domain (Lk1*) of the first reporter probe. Binding of the target to the first reporter probe inhibits the first reporter probe and the blocking probe from forming a complex.

In some embodiments of any one of the aspects, the second reporter probe comprises a nucleic acid, wherein the nucleic acid comprises: (1) a second nucleic acid first strand linked to a molecule/moiety capable of binding with the target (e.g., a second target binding ligand) and (2) a second nucleic acid second strand substantially complementary to the first strand, forming a double-stranded region (duplex region) with the first strand. The first strand comprises a hybridizing domain (Lk2*) linked to a toehold domain (x). The toehold domain (x) is substantially identical to the toehold domain (x) of the first reporter probe. The second strand comprises a primer binding domain (d*) linked to a toehold domain (a*). The toehold domain (a*) is substantially complementary to the toehold domain (a) of the first reporter probe.

In some embodiments of any one of the aspects, the second blocking probe comprises a blocking ligand (e.g., a second blocking ligand) capable of directly or indirectly forming a complex with the target binding ligand of the second reporter probe. The blocking ligand is linked to a blocking probe nucleic acid. The blocking probe nucleic acid comprises a toehold domain (x*) that is linked to a hybridizing domain (Lk2). The toehold domain (x*) is substantially complementary to the toehold domain (x) of the second reporter probe. The hybridizing domain (Lk2) is substantially complementary to the hybridizing domain (Lk2*) of the second reporter probe. Binding of the target to the second reporter probe inhibits the second reporter probe and the blocking probe from forming a complex.

### Design 6

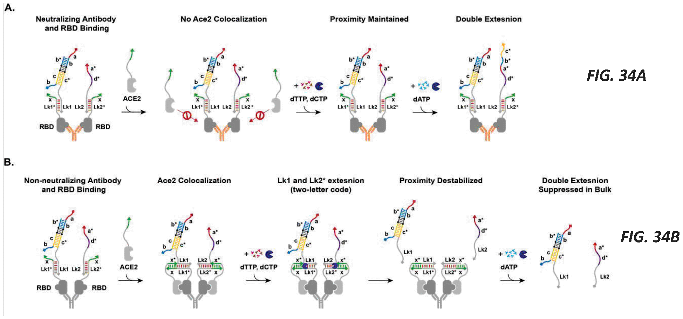

Another example of programming nucleic acid extension to differentiate neutralizing binding event vs non-neutralizing binding event with non-enzymatic strand displacement is schematically shown in **FIGS. 34A and 34B**. In **FIG. 34A**, a neutralizing target, e.g., neutralizing antibody is allowed to incubate with a pair of reporter probes, e.g., RBDs, conjugated with specially-design nucleic acid molecules, in equilibrium and their binding brings the reporter probes into proximity. However, the blocking probe, e.g., ACE2 which binds at the same location on the reporter probes, e.g., RBDs as the target, e.g., neutralizing antibody, optionally at a lower affinity, is generally unable to colocalize with reporter probes, e.g., RBDs which are already bound to the target. Proximity of the two reporter probes is maintained and allows double-extension of the nucleic acid strand comprising the toehold domain (a*) and the primer domain (d*) to generate a nucleic acid reporter comprising the domains (d*), (a*), (b*) and (c*) by the addition of polymerase. In contrast, as shown in **FIG. 34B**, with a non-neutralizing target, the reporter probes are not "neutralized" and can still bind to the blocking probe. The colocalization of the reporter probes with the blocking probes allows the toehold domain (x*) of the blocking probe to bind to the complementary toehold domain (x) of linking strand of the reporter probes. The nucleic acid on the blocking probe is extended with a polymerase in the presence of a dNTP mixture that is substantially free of a dNTP complementary to the nucleotide present in the toehold domain (a) or (b) of the first reporter probe but not in the hybridizinghybridizing domain (Lk1) of the first reporter probe and the hybridizinghybridizing domain (Lk2) of the second probe. This extension dissociates the nucleic acids from the reporter probes. It is noted that in this enzymatic step, polymerase extension of the nucleic acids in the reporter probe is prohibited which needs a dNTP that is not in the dNTP mixture. With proximity of reporter probe nucleic acids being disrupted, polymerase extension of the reporter nucleic acid strands is unfavorable to happen after the addition of the missing dNTP and generation of a nucleic acid record from double extension is generally prevented. In presence of the neutralizing target, the reporter probes remain in close proximity and a nucleic acid record comprising the domains (d*), (a*), (b*) and (c*) is generated by the two-step extension - extension in the presence of a dNTP mixture that is substantially free of a dNTP complementary to the nucleotide present in the toehold domain (a) or (b) of the first reporter probe but not in the hybridizinghybridizing domain (Lk1) of the first reporter probe and the hybridizinghybridizing domain (Lk2) of the second probe, followed by extension in the presence of a dNTP mixture that comprises the dNTP complementary to the nucleotide present in the toehold domain (a) or (b) of the first reporter probe but not in the hybridizinghybridizing domain (Lk1) of the first reporter probe and the hybridizinghybridizing domain (Lk2) of the second probe. Thus, a unique sequence is generated only when the binding between the reporter probes and the blocking probes, e.g., RBD-ACE2 interaction is disrupted by the presence of the target (e.g., a neutralizing antibody).

In some embodiments of any one of the aspects, the method comprises: (A) contacting the sample with a first reporter probe and a second reporter probe; (B) contacting the sample with a first blocking probe and a second blocking probe; (C) providing a polymerase and a first dNTP mix; (D) after a period of time (e.g., 5 seconds, 10 second, 15 seconds, 20 seconds, 25 seconds, 30 seconds, 35 second, 40 second, 45 seconds, 50seconds, 55 seconds, 1 minute, 2 minutes, 3 minutes, 4 minutes, 5 minutes, 10 minutes, 15 minutes, 20 minutes, 25 minutes, 30 minutes, 35 minutes, 40 minutes, 45 minutes, 50 minutes, 55 minutes or more), providing a second dNTP mix and optionally a polymerase, thereby producing a nucleic acid record of an interaction between the first nucleic acid and the second nucleic acid if said interaction is present; and (E) detecting the presence or absence of the nucleic acid record, and wherein the presence of the nucleic acid record indicates the target is present in the sample, wherein: (i) the first reporter probe comprises a first target binding ligand linked to a first nucleic acid and wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises: (1) a first nucleic acid first strand hybridized to the linking strand linked to the first target binding ligand and comprising a hybridizinghybridizing domain (Lk1) linked to a single-stranded toehold domain (a), wherein the toehold domain (a) is at an end distal from the hybridizinghybridizing domain (Lk1) and wherein the hybridizinghybridizing domain (Lk1) is substantially complementary to the hybridizinghybridizing domain (Lk1*); and (2) a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; (ii) the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, wherein a portion of the first nucleic acid and a portion of the second nucleic acid are capable of hybridizing in the presence of the target; (iii) the first blocking probe comprises a first blocking ligand capable of directly or indirectly forming a complex with the first target binding ligand and linked to a first blocking probe nucleic acid and wherein the first blocking probe nucleic acid comprises a toehold domain (x*) that is substantially complementary to the toehold domain (x) of the first reporter probe, and wherein binding of the target to the first reporter probe inhibits the first reporter probe and the first blocking probe from forming a complex; and (iv) the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex, and (iv) wherein the toehold domain (b) of the first reporter probe comprises a nucleotide that is not present in the hybridizinghybridizing domain (Lk1) of the first reporter probe and the hybridizinghybridizing domain (Lk2) of the second reporter probe; wherein the first dNTP mix is substantially free of a dNTP complementary to the nucleotide present in the toehold domain (a) or (b) of the first reporter probe but not in the hybridizing domain (Lk1) of the first reporter probe and/or the hybridizing domain (Lk2) of the second probe; and wherein the second dNTP mix comprises a dTNP complementary to the nucleotide present in the toehold domain (a) or (b) of the first reporter probe but not in the hybridizing domain (Lk1) of the first reporter probe and/or the hybridizing domain (Lk2) of the second probe.

In some embodiments of any one of the aspects, the first reporter probe comprises a double stranded nucleic acid hybridized at one end to a linking strand linked to a molecule/moiety capable of binding with the target (e.g., the first target binding ligand). The linking strand linked to the first target binding ligand comprises a first nucleic acid first hybridizing domain (Lk1*) for hybridizing with the double-stranded nucleic acid and a toehold domain (x) linked to the first nucleic acid first hybridizing domain (Lk1*). The double-stranded nucleic acid comprises: (1) a first nucleic acid first strand and (2) a first nucleic acid second strand substantially complementary to the first strand, forming a double-stranded region (duplex region) with the first strand. The first strand comprises: a primer binding domain (c*) linked to a first subdomain that is linked to a stopper molecule (•) that is linked to a second subdomain (b) that is linked to a toehold domain (a). The second strand comprises a first subdomain (b*) linked to a second subdomain that is linked to a stopper molecule (•) that is linked to a primer domain (c) that is linked to a toehold domain (b). The primer binding domain (c*) of the first strand and the primer domain (c) of the second strand are substantially complementary to each other. The first subdomain of the first strand and the second subdomain and second strand are substantially complementary to each other. The second subdomain (b) of the first strand and the first subdomain (b*) of the second strand are substantially complementary to each other. Further, the first subdomain (b*) and the toehold domain (b) of the second strand are substantially complementary to each other. The primer binding domain (c*), the first subdomain, and the second subdomain (b) of the first strand hybridize with the primer domain (c), the second subdomain, and the first subdomain (b*) of the second strand to form the double-stranded (duplex) region comprising the stopper molecules (•). The primer binding domain (c*) is linked to a first nucleic acid second hybridizing domain (Lk1) for hybridizing with the first nucleic acid first hybridizing domain (Lk1*) of the linking strand linked the first target binding ligand. In some embodiments, the hybridizing domain (Lk1) utilizes a 2- or 3-letter code. For example, in a 2-letter code two of the 4 nucleotides, e.g., two of A, G, C, and T are omitted the nucleotide sequence of the hybridizing domain (Lk1). In a 3-letter code one of the 4 nucleotides, e.g., one of A, G, C, and T is omitted the nucleotide sequence of the hybridizing domain (Lk1). Generally, a nucleotide present in the toehold domain (a) or (b) is omitted in the hybridizing domain (Lk1).

In some embodiments of any one of the aspects, the first blocking probe comprises a blocking ligand (e.g., a first blocking ligand) capable of directly or indirectly forming a complex with the target binding ligand of the first reporter probe and linked to a first blocking probe nucleic acid. The first blocking probe nucleic acid comprises a toehold domain (x*). The toehold domain (x*) of the first blocking probe nucleic acid is substantially complementary to the toehold domain (x) of the first reporter probe. Binding of the target to the first reporter probe inhibits the first reporter probe and the first blocking probe from forming a complex.

In some embodiments of any one of the aspects, the second reporter probe comprises a nucleic acid, wherein the nucleic acid comprises: (1) a second nucleic acid first strand linked to a molecule/moiety capable of binding with the target (e.g., the second target binding ligand) and (2) a second nucleic acid second strand substantially complementary to the first strand, forming a double-stranded region (duplex region) with the first strand. The first strand comprises a hybridizing domain (Lk2*) linked to a toehold domain (x). The toehold domain (x) is substantially identical to the toehold domain (x) of the first reporter probe. The second strand comprises a primer binding domain (d*) linked to a toehold domain (a*). The toehold domain (a*) is substantially complementary to the toehold domain (a) of the first reporter probe. In some embodiments, the hybridizing domain (Lk2) utilizes a 2- or 3-letter code. For example, in a 2-letter code two of the 4 nucleotides, e.g., two of A, G, C, and T are omitted the nucleotide sequence of the hybridizing domain (Lk2). In a 3-letter code one of the 4 nucleotides, e.g., one of A, G, C, and T is omitted the nucleotide sequence of the hybridizing domain (Lk2). Generally, a nucleotide present in the toehold domain (a) or (b) is omitted in the hybridizing domain (Lk1).

In some embodiments of any one of the aspects, the second blocking probe comprises a blocking ligand (e.g., a second blocking ligand) capable of directly or indirectly forming a complex with the target binding ligand of the second reporter probe. The blocking ligand is linked to a blocking probe nucleic acid. The blocking probe nucleic acid comprises a toehold domain (x*). The toehold domain (x*) is substantially complementary to the toehold domain (x) of the second reporter probe. Binding of the target to the second reporter probe inhibits the second reporter probe and the second blocking probe from forming a complex.

By comparing the amount of the nucleic acid record relative to a control or reference sample (e.g., a sample without the target), absence or presence of the target can be determined. For example, an amount less than 95%, less than 90%, less than 85%, less than 80%, less than 75%, less than 70%, less than 65%, less than 60%, less than 65%, less than 60%, less than 45%, less than 40%, less than 35%, less than 30%, less than 25%, less than 20%, less than 15%, less than 10%, or less than 5% relative to the control or reference sample (e.g., a sample without the target) indicates target is present in the sample.

### A BRIEF DESCRIPTION OF THE DRAWINGS

This application file contains at least one drawing executed in color. Copies of this patent application publication with color drawings will be provided by the Office upon request and payment of the necessary fee.
**FIG. 1** is a schematic representation of principles of programming nucleic acid extension to differentiate neutralizing binding event vs non-neutralizing binding event. When A is "neutralized" by antibody or other biomolecules and is generally prevented from binding to B, Sequence a* can be extended twice or multiple times to generate "target" sequence c*. On the other hand, when A is not "neutralized" and can still bind to B, only intermediate sequences (b*) can be extended to a* due to the "interference" of the nucleic acid sequence conjugated on B.
**FIG. 2** is a schematic representation of an exemplary probe design for neutralizing antibody assay that is not specific to antibody class. A1 and A2 are the same receptor protein conjugated to different DNA probes. B binds to A1 and A2 forming receptor-ligand complex. DNA domains with the same color are complementary to each other.
**FIG. 3** is a schematic representation of probe complex forming by receptor-ligand interaction.
**FIG. 4** is a schematic representation of an embodiment of the detection mechanism exemplified by a qPCR readout. The presence of neutralizing antibodies results in the generation of a DNA strand with sequences of domain a*, b*, c* and d*. This DNA sequence can be specifically amplified by a pair of PCR primers designed. The presence of a non-neutralizing antibody results in "deactivation" of the probe and can only produce a sequence with domain a*, b* and d*.

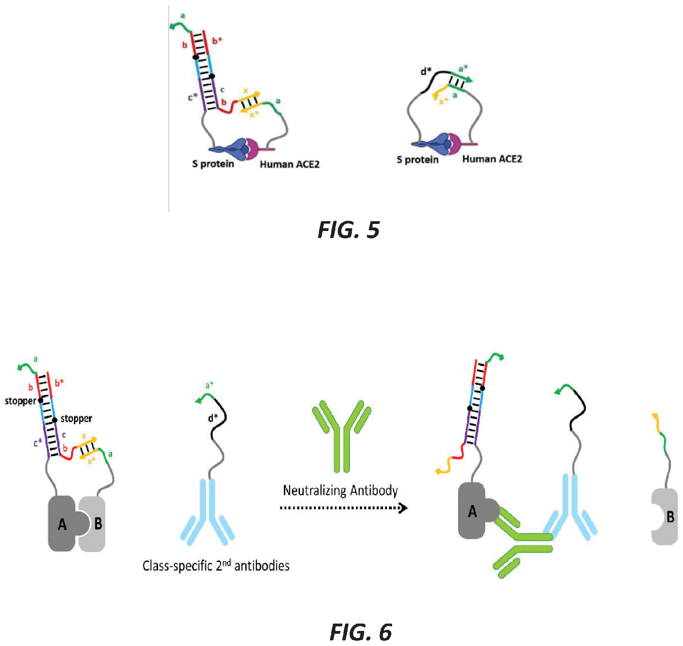

**FIG. 5** is a schematic representation of probe design for SARS-COV-2 neutralizing antibody assay according to an exemplary embodiment of the method.
**FIG. 6** is a schematic representation of probe design and reaction process for class-specific neutralizing antibody detection system according to an exemplary embodiment.

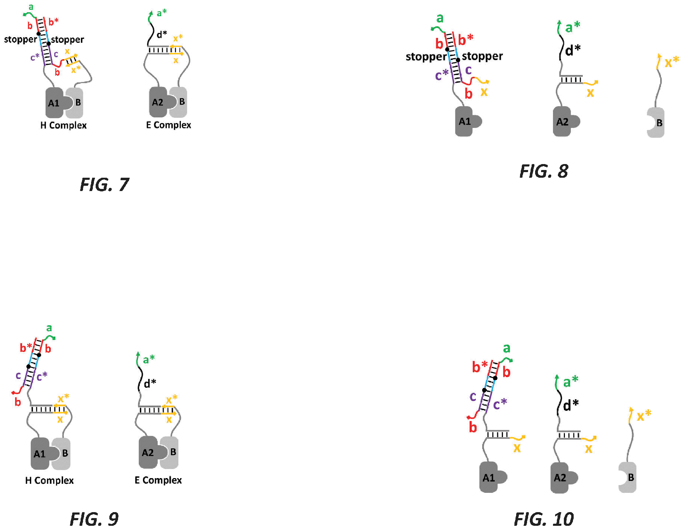

**FIG. 7** is a schematic representation of another exemplary probe design for neutralizing antibody assay that is not specific to antibody class. A1 and A2 are the same receptor protein conjugated to different DNA probes. B binds to A1 and A2 forming receptor-ligand complex. DNA domains with the same color are complementary to each other.
**FIG. 8** is a schematic representation of uncomplexed format of the probe design depicted in the **FIG. 7**.
**FIG. 9** is a schematic representation of yet another exemplary probe design for neutralizing antibody assay that is not specific to antibody class. A1 and A2 are the same receptor protein conjugated to different DNA probes. B binds to A1 and A2 forming receptor-ligand complex. DNA domains with the same color are complementary to each other.
**FIG. 10** is a schematic representation of uncomplexed format of the probe design depicted in the **FIG. 9**.
**FIG. 11** is a schematic representation of an embodiment of the detection mechanism exemplified by a qPCR readout. The presence of neutralizing antibodies results in the generation of a DNA strand with sequences of domain a*, b*, c* and d*. This DNA sequence can be specifically amplified by a pair of PCR primers designed. The presence of a non-neutralizing antibody results in "proximity disruption" of the previously co-localized DNA probe and can only produce a sequence with domain a*, b* and d*.

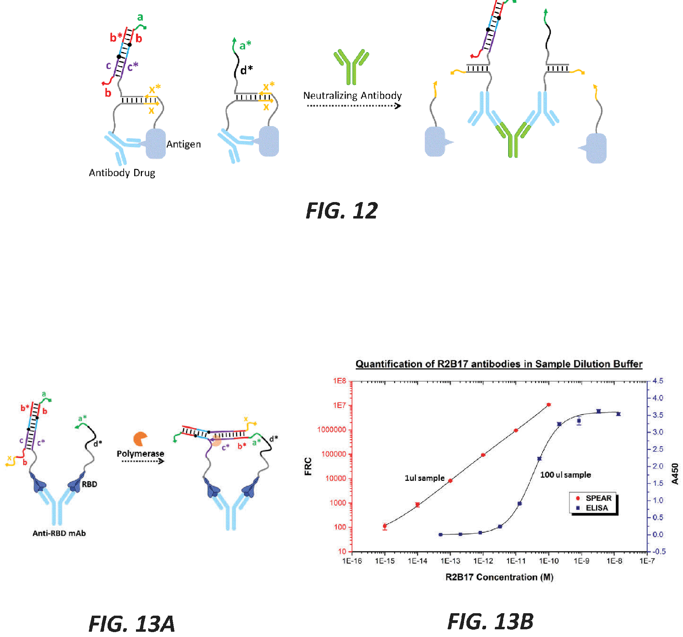

**FIG. 12** is a schematic representation of detection of neutralizing antibodies for monoclonal antibody drugs according to an exemplary embodiment of the method.
**FIG. 13** shows anti-SARS-COV-2-RBD antibody assay demonstrated by a monoclonal antibody R2B17. The basic construct of the assay in which two RBD conjugates bind to an anti-RBD mAb produces a PCR-amplifiable sequence d*a*b*c*. Comparing to a conventional ELISA assay using the same RBD protein as the capture antigen, the SPEAR (Successive Proximity Extension Amplification Reaction) assay has achieved 1,000 times higher sensitivity with only one percent of the sample volume. Particularly, this binding assay shows a linear range of five orders of magnitude and minimum variation between repeats.

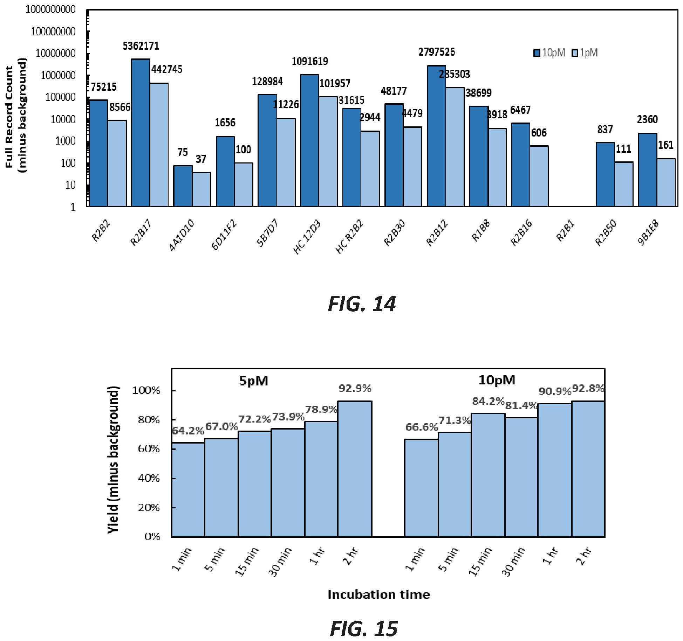

**FIG. 14** is a bar-graph showing anti-SARS-COV-2-RBD antibody assay test by 14 monoclonal antibodies. Only one antibody did not have a response due to the RBD recombinant protein sequence used in the assay.
**FIG. 15** is a bar graph showing high binding yield can be achieved between human ACE2 conjugate (50nM) and SARS-COV-2 RBD conjugate (5pM/10pM) after incubation at room temperature for 2h.
**FIG. 16** is a schematic representation of another principle of programming nucleic acid extension to differentiate neutralizing binding event vs non-neutralizing binding event. When A is "neutralized" by antibody or other biomolecules and is generally prevented from binding to B, Sequence a* is generally prevented from being extended or only intermediate sequences (b*) can generally be extended to a*. On the other hand, when A is not "neutralized" and can still bind to B, sequence a* can be extended twice or multiple times to generate "target" sequence c*.
**FIG. 17** is a schematic representation of an embodiment of the detection mechanism exemplified by probe design and detection mechanism of SARS-COV-2 neutralizing antibody assay. Spike protein binds to human ACE2 receptor forming ligand-receptor complex. DNA domains with the same color are complementary to each other. The presence of neutralizing antibodies facilitates the dissociation of ACE2 from spike protein and result in no extension of a* on ACE2. The presence of a non-neutralizing antibody does not disrupt the ligand-receptor complex and the proximity of the conjugated DNA probe, promoting successive polymerase extension reactions to generate a DNA strand with sequences of domain a*, b*, c* and d*.

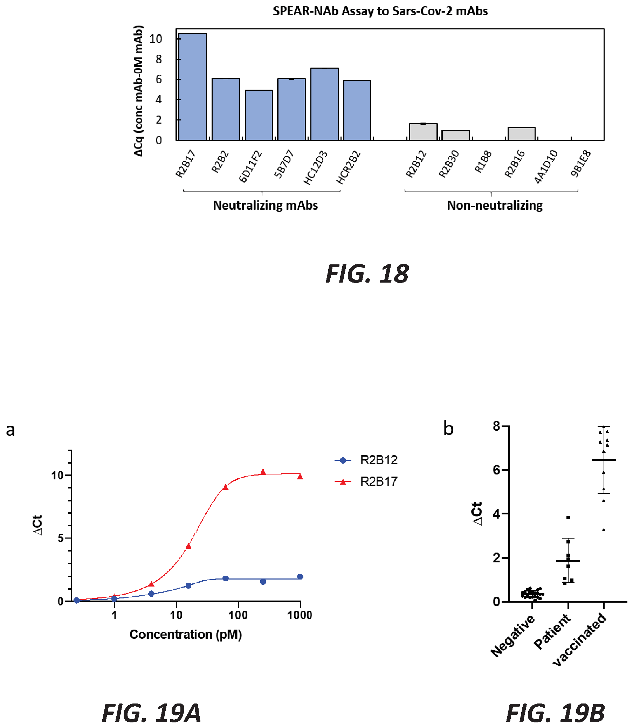

**FIG. 18** is a bar graph showing exemplary assay response to various neutralizing and non-neutralizing antibodies against SARS-Cov-2 spike proteins. In details, 1nM monoclonal antibodies were used to compete with ACE2 proteins to bind SAR-Cov-2 S1 proteins. No antibodies were added in the control samples. The ΔCq refers to the qPCR cycle difference between the antibody-containing samples and control samples. The assay shows high specificity on differentiating neutralization capability of various antibodies.
**FIG. 19A** is a line graph showing measurement of the neutralizing capability of two anti-SARS-Cov2 monoclonal antibodies at different concentrations. R2B17 is a neutralizing antibody and R2B12 is a non-neutralizing antibody. The antibodies were diluted from 1 nM to 244 fM, and the control sample contains no antibodies. The ΔCt refers to the qPCR cycle difference between the antibody-containing samples and control samples.
**FIG. 19B** is a plot showing assay performance according to an embodiment of the disclosure on Dried Blood Spot Samples collected from unvaccinated health donors (i.e. Negative), recovered patients from SARS-Cov2 infection and vaccinated patients (Moderna, two doses). For each dried blood spot sample, a 6 mm spot was punched and eluted using 500 µL elution buffer. The assay is demonstrated to be able to detect neutralizing antibody from all the recovered patient samples and vaccinated donor samples.

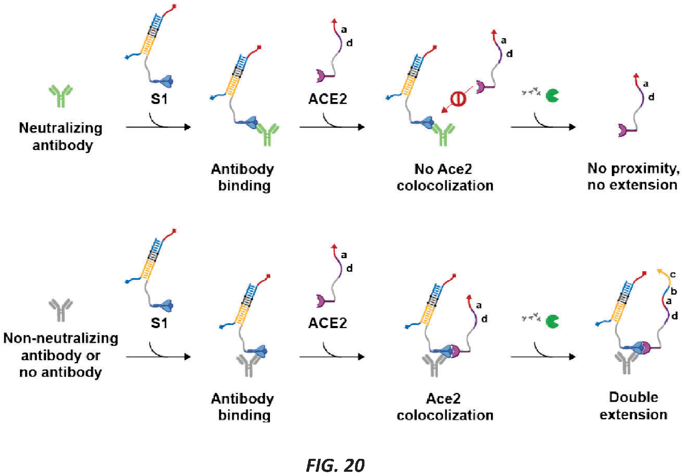

**FIG. 20** is a schematic of SPEAR-NAb, a neutralization assay (competitive binding assay) for anti-SARS-COV2 antibodies. SPEAR-NAb deploys similar affinity probes construct as SPEAR assay with S probe and P probe conjugating respectively to S1 and Ace2 proteins. Assay starts by incubating sample with the S1 conjugates, followed by Ace2 incubation. The mixture will then undergo polymerase reaction before qPCR amplification. Neutralizing antibody which exhibits higher affinity in binding to S1 on the same epitope than Ace2, inhibits Ace2 from binding and colocalizing with S1 for DNA extension. In contrast, in condition where no antibody or non-neutralizing antibody is present, binding of the antibody on a different epitope of S1 will not prevent Ace2 from binding with S1. Ace2 is able to colocalize with S1 and performs double extension to generate a complete reporter sequence.

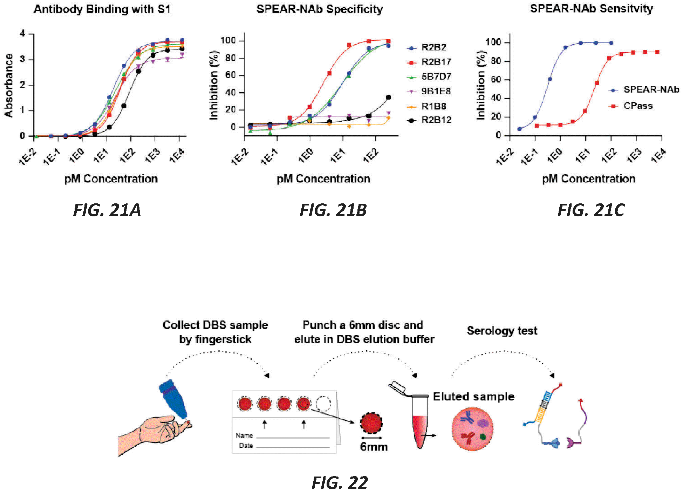

**FIGS. 21A-21C** are line graphs showing a SPEAR-NAb specificity and sensitivity on anti-SARS COV2 neutralizing antibody detection. (**FIG. 21A**) Six anti-SARS COV2 monoclonal antibodies of different neutralizing capabilities were tested on ELISA and showed good and similar binding affinity with S1. SPEAR-NAb test was performed on these antibodies at 4-fold dilutions from 250nM and was able to differentiate anti-SARS COV2 antibodies with different neutralizing capabilities. (**FIG. 21B**) Antibodies with high reported neutralization capabilities (R2B12, R2B17 and 5B7D7) showed significantly higher signal inhibition than non-neutralizing counterparts (9B1E8, R1B8 and R2B12). (**FIG. 21C**) SPEAR has two-order increase in sensitivity in comparison to commercially-available CPASS assay.
**FIG. 22** is a schematic of experimental workflow on DBS sample collection for SPEAR-NAb test. Blood drawn by fingerstick is collected on DBS card and dried overnight. A 6mm disc is punched and eluted in 500 µL DBS elution buffer at 37 °C for 1.5 hr.

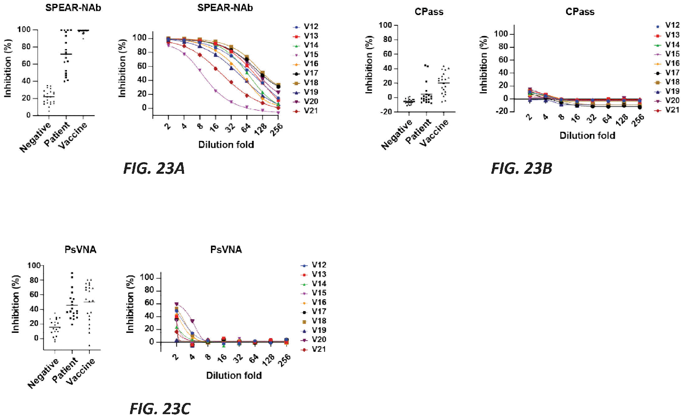

**FIGS. 23A-23C** are line graphs showing comparison of SPEAR-NAb vs CPASS vs PsVNA on neutralization measurement of negative, infected patient and vaccinated donor DBS samples.
**FIGS. 23A and 23B** demonstrates excellent sensitivity (100%) and specificity (100%) of SPEAR-NAb in clearly differentiating vaccinated and infected patient samples from negative samples (uninfected and unvaccinated), with all vaccinated samples (n=21) above the grand mean of patient samples (n=19) and patient samples above the grand mean of negative subjects (n=22); whereas CPASS and PsVNA poorly differentiate vaccine from patient samples, and vaccine/ patient samples from negative subjects with DBS samples.
**FIG. 23C** is the neutralizing titer measurement at 2-fold dilution on 10 vaccinated DBS samples. SPEAR-Nab is sensitive to measure changes across titers for all vaccine samples (up to 256x) and is capable of quantifying NT50 across vaccine samples. In contrast, CPass and PsVNA show much lower inhibition on 2x dilution and is unable to detect neutralizing antibodies at 8x dilution from all the DBS samples.

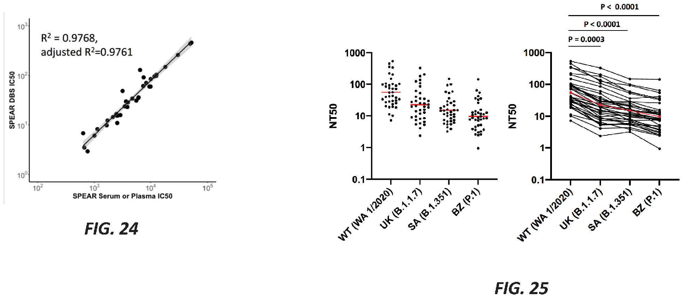

**FIG. 24** is a line graph showing concordance measurement of SPEAR-NAb on serum, plasma and DBS samples. Excellent concordance was observed between DBS and serum/plasma samples (R2=0.9788) by SPEAR-Nab. Shaded area shows 90% confidence interval of simple linear regression.
**FIG. 25** is a plot and line graph showing SPEAR-NAb on measuring neutralization titers of vaccinated donor (n=37) against SARS-COV-2 wildtype (WT) and different variants identified from United Kingdom (UK), south Africa (SA) and Brazil (BZ) from a single DBS sample. Neutralizing titer at 50% inhibition (NT50) was interpolated from 8x 2-fold dilution on each sample. Red bar/line shows the geometric mean titer (GMT). The left panel shows that lower NT50 for variants (UK (B.1.1.7) > SA (B.1.351) > BZ (P.1)) in comparison to WT. Variant measurement per individual is shown on the right panel and similar decrease of NT50 on S1 variants is observed. Statistical significance test is Friedman test with subsequent Dunn's multiple comparison.

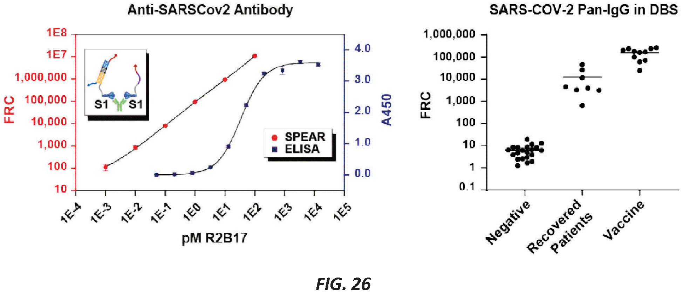

**FIG. 26** is a line and plot graph showing SARS-COV2 Pan-IgG assay by SPEAR. SPEAR demonstrates high sensitivity in quantifying anti-SARS COV2 monoclonal antibodies (R2B17) by S1 conjugated SPEAR probes at low fM over a wide dynamic range, with four orders improvement in sensitivity in comparison to ELISA (left panel). DBS samples from vaccine subjects and recovered patients are perfectly differentiated (100% sensitivity and 100% specificity) from negative DBS samples (uninfected and unvaccinated donors).

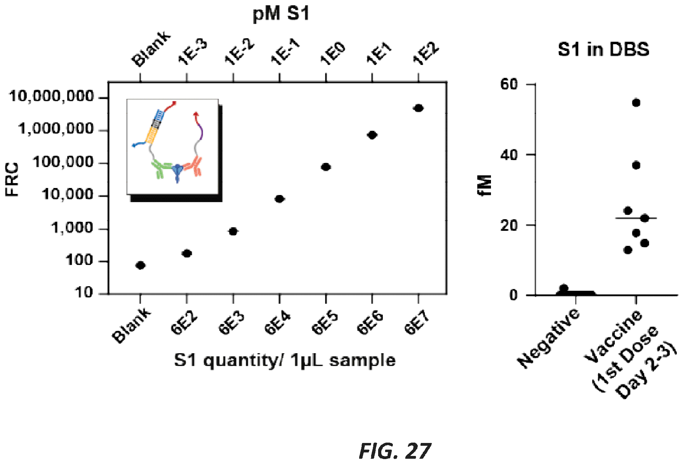

**FIG. 27** is plot graphs showing S protein expression assay by SPEAR. SPEAR works well in quantifying S proteins at low fM over a wide dynamic range using probes conjugated to two anti-SARS COV2 monoclonal antibodies of different S1 binding epitopes. SPEAR is able to detect fM copies of S1 from DBS samples of vaccinated donors (Day 2 or Day 3 post first dose) with excellent specificity.

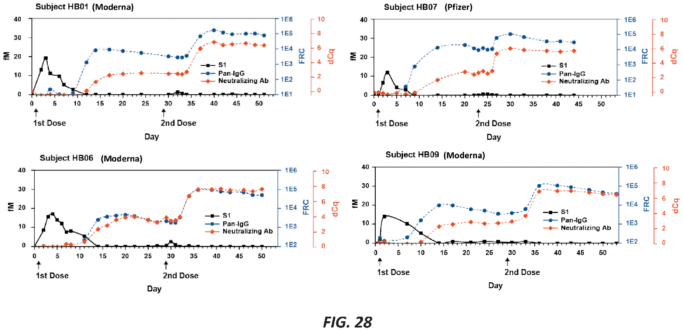

**FIG. 28** is a series of plot graphs showing multiple assays can be conducted on a single DBS sample. Longitudinal study of vaccinated subjects (Moderna/Pfizer) on S protein, Pan-IgG and neutralizing antibodies measurement by SPEAR/ SPEAR-Nab. DBS samples of four vaccinated subjects were measured over time-course before and after first and second inoculation. SPEAR shows distinct profiles of S1 over time where initial peak was observed roughly 3 days post the first dose for all four individuals and flatten when Pan-IgG and neutralizing antibody appeared to rise. Similar profile was observed for Pan-IgG and neutralizing antibody that first peak was observed on Day 14 after the first jab and gradually decrease over time and the second peak was observed roughly 9 days after the second dose.

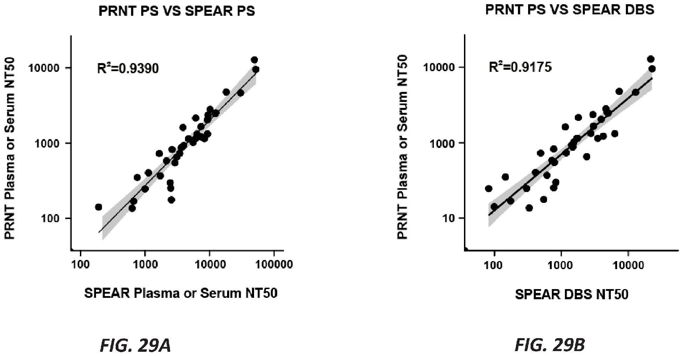

**FIGS. 29A and 29B** are line graphs showing measurement of SPEAR-NAb on serum, plasma/serum (**FIG. 29A**) and DBS (**FIG. 29B**) samples. Excellent concordance was observed between plaque reduction neutralization test (PRNT) and SPEAR-Nab for the serum/plasma samples (R²=0.9390). Excellent concordance was observed between PRNT serum/plasma samples and SPEAR-Nab DBS samples (R²=0.9175). Shaded area shows 90% confidence interval of simple linear regression.

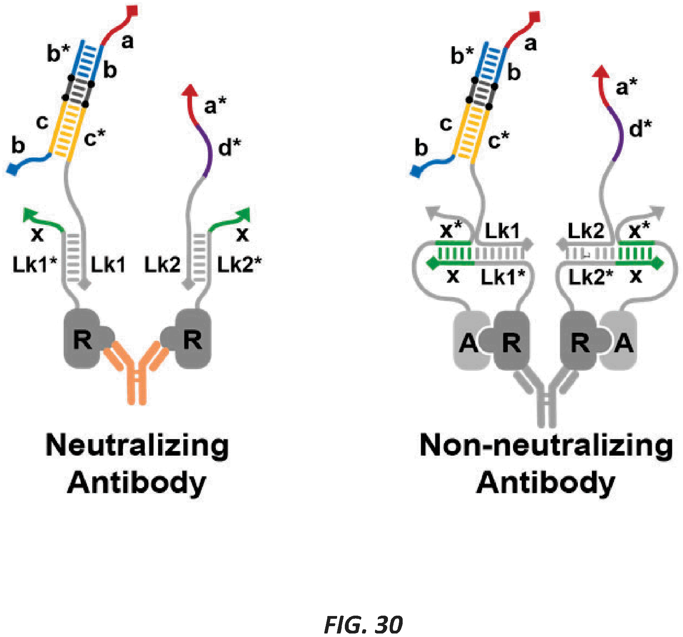

**FIG. 30** is a schematic representation of an exemplary probe design for neutralizing antibody assay based on DNA strand-displacement approach. The nucleic acid on the blocking probe (A, ACE2) is designed to be complementary to a portions of the linking strands on the reporter probes (R, RBD). Colocalization of the blocking probe (A, ACE2) and the reporter probes (R, RBD) initiates a toehold-mediated strand displacement reaction to dissociate nucleic acids from the reporter probes needed for the proximity reaction. The blocking probes ACE2 and the reporter probes RBD are labeled as A and R.
**FIGS. 31A and 31B** are schematic representation of the reaction workflow of non-enzymatic strand displacement approach for the detection of neutralizing target e.g., neutralizing antibodies (**FIG. 31A**) vs. a non-neutralizing target (**FIG. 31B**) according to an embodiment of the disclosure. (**FIG. 31A**) Reaction workflow for neutralizing antibody. Neutralizing antibody is allowed to incubate with a pair of DNA conjugated RBDs in equilibrium and their binding brought the probes into proximity. Another DNA conjugated ACE2 is sequentially added to bind with RBDs. However, ACE2 which shares the same binding motif with RBDs as the neutralizing antibody at a lower affinity is generally unable to colocalize with RBDs to which the binding site is already occupied by the neutralizing antibody. Proximity of nucleic acids of the reporter probes is maintained and allow double extension of the primer probe to generate a nucleic acid record by the addition of polymerase. (**FIG. 31B**) Reaction workflow for non-neutralizing antibody. While non-neutralizing antibody binds with RBDs at a different epitope, ACE2 is able to bind with RBDs. The colocalization allows domain x* to bind on the complementary toehold of the linker strand on RBDs and triggers a toehold mediated strand displacement reaction that dissociates the probes from proximity. The generation of reporter sequence from double extension is generally prevented.

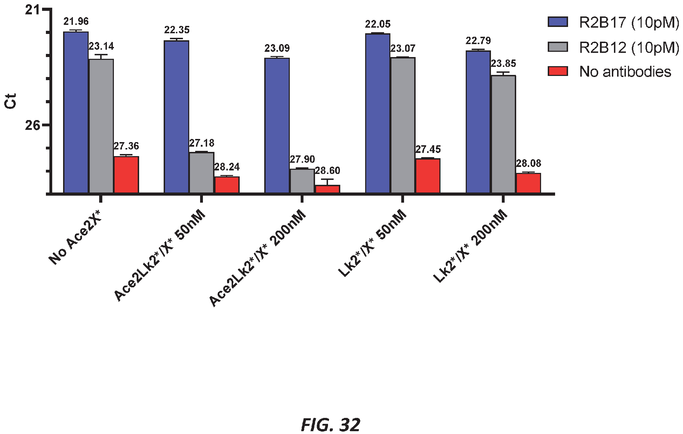

**FIG. 32** is bar graph showing detection of neutralizing and non-neutralizing antibodies according to an exemplary embodiment shown in **FIGS. 31A and 31B**.

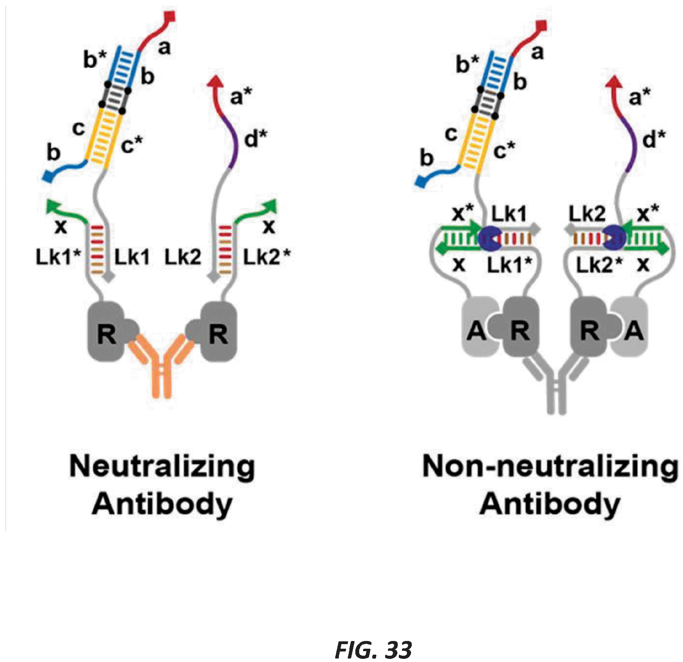

**FIG. 33** is a schematic representation of an exemplary probe design for neutralizing antibody assay based on enzymatic and DNA strand-displacement approaches. Reacting DNA strands are composed of 2-letter or 3-letter code to allow polymerase extension in a sequential fashion. An initial polymerase extension is programmed to extend the 2-letter coded linking strands (presented as brown and pink on Lk1/1* and Lk2/2*) on the blocking probe (A, ACE2) and the reporter probes (R, RBD) when they are colocalized. The extension disrupts proximity of the nucleic acids of the reporter probes and suppresses the generation of a nucleic acid record from double extension of the a* domain. The blocking probes ACE2 and the reporter probes RBD are labeled as A and R.
**FIGS. 34A and 34B** are schematic representation of the reaction workflow of non-enzymatic strand displacement approach for the detection of neutralizing target e.g., neutralizing antibodies (**FIG. 34A**) vs. a non-neutralizing target (**FIG. 34B**) according to an embodiment of the disclosure. (**FIG. 34A**) Reaction workflow for neutralizing antibody. Neutralizing antibody is allowed to incubate with a pair of DNA conjugated RBDs in equilibrium and their binding brings reporter probes into proximity. Another DNA conjugated ACE2 is sequentially added to bind with RBDs. However, ACE2 which shares the same binding motif with RBDs as the neutralizing antibody at a lower affinity is generally unable to colocalize with RBDs to which the binding site is already occupied by the neutralizing antibody. Proximity of nucleic acids of the two reporter probes is maintained and allow double extension of the primer probe in presence of three types of dNTPs to generate a nucleic acid record. (**FIG. 34B**) Reaction workflow for non-neutralizing antibody. While non-neutralizing antibody binds with RBDs at a different epitope, ACE2 is able to bind with RBDs. The colocalization allows x* (green domain) to bind on the complementary toehold (x) of the linker strand on RBDs. By adding two types of dNTPS (i.e., dTTPs and dCTPs), x* is allowed to extend by copying the two-letter coded sequence of the linker regions (Lk1 and Lk2*) on RBDs and displace the DNA probes from RBDs. It is noteworthy that in this enzymatic step, polymerase extension of the primer probe is generally prohibited which requires a third letter code to begin with. With proximity of reporter probes being disrupted, polymerase extension of the primer strand is unfavorable to happen after the addition of a third letter code and generation of the reporter sequence from double extension is generally prevented.

### DETAILED DESCRIPTION

An exemplary embodiment for detecting a target is depicted in **FIG. 4**. When the first target binding ligand of the first reporter probe and the second target binding ligand of the second reporter probe are co-bound to the target, the toehold domain (a) in the first nucleic acid first strand of the double-stranded nucleic acid of the first reporter probe can hybridize with the toehold domain (a*) of the second nucleic acid of the second reporter probe. A polymerase, e.g., a strand displacing polymerase extends the hybridized strands until it reaches a stopper molecule. This extension results in a newly formed subdomain (b*) appended to toehold domain (a*) of the second reporter probe. The target binding ligands of the reporter probes remain bound to the target, which allows the nucleic acid strands to rearrange. This rearrangement allows the newly formed subdomain (b*) to bind with the complementary subdomain (b) of the first nucleic acid second strand of the first reporter probe. A strand displacing polymerase extends the rearranged hybridized strands until it reaches a stopper molecule. This second extension appends a second primer binding domain (c*) to the newly formed subdomain (b*). The resulting nucleic acid comprises, e.g., in order, domains d*, a*, b* and c*, and provides a record of interaction between the first nucleic acid of the first reporter probe and the second nucleic acid of the second reporter probe.

### Probes

In some embodiments of any one of the aspects, the blocking ligand and target are competitive binding partners of the target binding ligand. For example, the target and the blocking ligand may bind to the same part of the target binding ligand. Alternatively, or in addition, binding of the target to the target binding ligand alter the binding site for the blocking ligand thereby inhibiting binding of target binding ligand and the blocking ligand to each other.

It is noted that the target binding ligand and the blocking ligand can form a complex directly or indirectly. For example, the target binding ligand and the blocking ligand can bind to each other to directly form a complex. Alternately, the target binding ligand and the blocking ligand can bind simultaneously with another molecule to indirectly form a complex. Regardless of how the complex is formed, binding of the target to the target binding ligand inhibits formation of a complex between the target binding ligand and the blocking ligand.

Any pair of molecules that can bind to each other and where one pair of the member is competitive binding partner with the target can be used for the reporter and blocking probes described herein. In some embodiments of any of the aspects, the pair of molecules are members of a binding pair. As used herein, the term "binding pair" refers to a pair of moieties that specifically bind each other with high affinity, generally in the low micromolar to picomolar range. When one member of a binding pair is conjugated to a first element and the other member of the pair is conjugated to a second element, the first and second elements will be brought together by the interaction of the members of the binding pair. Non-limiting examples of binding pairs include antigen:antibody (including antigen-binding fragments or derivatives thereof), biotin:avidin, biotin:streptavidin, biotin:neutravidin (or other variants of avidin that bind biotin such as ), receptor:ligand, and the like. Additional molecule for binding pair can include, neutravidin, strep-tag, strep-tactin and derivatives, and other peptide, hapten, dye-based tags-anti-Tag combinations such as SpyCatcher-SpyTag, His-Tag, Fc Tag, Digitonin, GFP, FAM, haptens, SNAP-TAG. HRP, FLAG, HA, myc, glutathione S-transferase (GST), maltose binding protein (MBP), small molecules, and the like. Some exemplary pair of molecules include, but are not limited to, a receptor and ligand, nucleic acid and nucleic acid binding protein, antibody and antigen, antigen binding fragment of an antibody and antigen, antibody and Fc receptor, antibody and protein A, aptamer and its binding target, a drug and its target, and the like.

In some embodiments, the target binding ligand is a receptor and the blocking ligand is a ligand for the receptor or vice-versa.

In some embodiments, the target binding ligand can be an antibody, e.g., a class specific antibody.

In some embodiments, the target binding ligand can be a DNA or RNA binding protein and the blocking ligand is a nucleic acid or vice versa.

### Nucleic acid strands

As described herein, the probes comprise nucleic acid strands linked to target binding ligands or the blocking ligand. It is noted that the nucleic acid strand can be linked covalently or non-covalently to the ligand (e.g., target binding ligand or the blocking ligand). The strand can be linked by its 5'-end or by its 3'-end. In some preferred embodiments, the strand is linked to the ligand (e.g., target binding ligand or the blocking ligand) by its 5'-end. In some embodiments of any one of the aspects, the linking strand comprises a single-stranded toehold domain or pairing domain at an end distal to the linked end.

As used herein, "toehold domain" and "pairing domain" refer to a portion of a strand having complementary for hybridizing with a second strand. The toehold and/or the pairing domain can be of any desired length or sequence. For example, the toehold and/or the pairing domain can be independently at least 4, at least 10, at least 15, at least 20, at least 25, at least 30, at least 35, at least 40, at least 45, at least 50, at least 55, at least 60, at least 65, at least 70, at least 75, at least 80, at least 85, at least 90 or more nucleotides in length. In some embodiments, the toehold and/or the pairing domain is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less or 30 nucleotides or less in length. In some embodiments, the toehold and/or the pairing domain can be from about 4 nucleotides to about 50 nucleotides in length. For example, a toehold and/or the pairing domain may have a length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. In some embodiments, the toehold and/or the pairing domain has a length of 4 to 10, 4 to 15, 4 to 20, 4 to 25, 4 to 30, 4 to 35, 4 to 40, 4 to 45 or 4 to 50 nucleotides. In some embodiments, the toehold and/or the pairing domain is longer than 40 nucleotides. For example, the toehold and/or the pairing domain may have a length of 4 to 100 nucleotides. In some embodiments, the toehold and/or the pairing domain has a length of 4 to 90, 4 to 80, 4 to 70, 4 to 60, or 4 to 50 nucleotides

In some embodiments, the first reporter probe comprises a double-stranded nucleic acid linked to the first target binding ligand where a first reporter probe first strand of the double-stranded nucleic acid is linked to the target binding ligand and comprises a single-stranded toehold domain (a) at an end distal to the linked end. A first reporter probe second strand of the double-stranded nucleic acid comprises, at the end distal to the toehold domain (a) of the first strand, a subdomain (b) that is linked to a pairing domain (x), where the pairing domain (x) is closer to the end of second strand. The two strands are complementary to each other and form a double-stranded region (duplex region) having single stranded regions at each end. For example, the double stranded molecule comprises a first single stranded region comprising the toehold domain (a) of the first strand at one end of the duplex region and a second single-stranded region comprising the subdomain (b) and the pairing domain (x) of the second strand at the opposing end of the duplex region.

In some embodiments, the first reporter probe comprises a double-stranded nucleic comprising a first reporter probe first strand and a first reporter probe second strand, wherein the first strand of the double-stranded nucleic acid is hybridized to a linking strand linked to the first target binding ligand. The linking strand linked to the first target binding ligand comprises a first nucleic acid first hybridizing domain linked to a paring domain (x), where the pairing domain (x) is at end distal to the end linked to the first target binding ligand. The first strand of the double-stranded nucleic acid comprises a first nucleic acid second hybridizing domain at one end and a single-stranded toehold domain (a) at an end distal to the first nucleic acid second hybridizing domain. The second strand of the double-stranded nucleic acid comprises, at the end distal to the toehold domain (a) of the first strand, a subdomain (b). The two strands are complementary to each other and form a double-stranded region (duplex region) having single stranded regions at each end. For example, the double stranded molecule comprises a first single stranded region comprising the toehold domain (a) of the first strand at one end of the duplex region and a second single-stranded region comprising the subdomain (b) of the second strand at the opposing end of the duplex region. The hybridizing domain of the linking strand and the hybridizing domain of the first strand are hybridized to each other.

In some embodiments of any one of the aspects described herein, the duplex region of the double-stranded nucleic acid of the first reporter probe comprises a stopper molecule or modification (•) in the first strand and a stopper molecule or modification (•) in the second strand. As used herein, a "stopper molecule or modification" refers to a molecule or modification that terminates template directed polymerization by a polymerase. In other words, the stopper molecule or modification is capable of preventing a polymerase, e.g., a strand displacing polymerase from extending through the duplex region.

In some embodiments, a stopper molecule or modification can be a natural nucleotide. For example, the stopper molecule can be a natural nucleotide and the extension with the polymerase can be carried out in absence of the nucleotide complementary to the stopper nucleotide. For example, a "C-stopper" comprises at least one C nucleotide that can terminate polymerization in the absence of dGTPs. In some embodiments, a stopper is at least one C nucleotide. In some embodiments, a stopper is at least one G nucleotide. In some embodiments, a stopper is at least one A nucleotide. In some embodiments, a stopper is at least one T nucleotide.

In some embodiments, the molecule that terminates polymerization is a single or paired non-natural nucleotide sequence, such as iso-dG and iso-dC (IDT), which are chemical variants of cytosine and guanine, respectively. Iso-dC will base pair (hydrogen bond) with iso-dG but not with dG. Similarly, iso-dG will base pair with iso-dC but not with dC. By incorporating these nucleotides in a pair on opposite sides of the hairpin, at the stopper position, the polymerase will be halted, as it does not have a complementary nucleotide in solution to add at that position.

In some embodiments, RNA bases and/or methylated RNA bases may be used as stop sequences within a hairpin primer. For example, 2'-O-methylated RNA may be used as a molecule that terminates polymerization.

In some embodiments, the molecule that terminates polymerization is an inverted nucleotide. For example, a nucleotide linked via a 5'->5' or a 3'->3' linkage.

In some embodiments, the molecule that terminates polymerization is a synthetic non-DNA linker, for example, a triethylene glycol spacer, such as the Int Spacer 9 (iSp9) or Spacer 18 (Integrated DNA Technologies (IDT)). It should be understood that any non-native linker that terminates polymerization by a polymerase may be used as provided herein. Other non-limiting examples of such molecules and modifications include a three-carbon linkage (/iSpC3/) (IDT), ACRYDITE^{™} (IDT), adenylation, azide, digoxigenin (NHS ester), cholesteryl-TEG (IDT), I-LINKER^{™} (IDT), and 3-cyanovinylcarbazole (CNVK) and variants thereof. Typically, but not always, short linkers (e.g., iSp9) lead to faster reaction times.

Inclusion of a molecule or modification that terminates polymerization often creates a "bulge" in the duplex region, because the molecule or modification is not paired. Thus, in some embodiments, the duplex region is designed to include, opposite the stopper molecule or modification, a single nucleotide (e.g., thymine), at least two of same nucleotide (e.g., a thymine dimer (TT) or trimer (TTT)), or a non-natural modification.

It is noted that the stopper molecules can be in a complementary position or in non-complementary positions. For example, the first and second stopper molecules can be positioned 1, 2, 3, 4, 5, 6, 7, 8, 9, 10, 11, 12, 13, 14, 15, 16, 17, 18, 19, 20, 21, 22, 23, 24, 25 or more nucleotides (or base-pairs) apart. When the stopper molecules are in non-complementary positions, one stopper molecule can be closer to the end of the duplex with the toehold domain (a) of the first strand and the second stopper molecule can be closer to the end of the duplex with the toehold domain (x) of second strand. In some embodiments, the stopper molecule in the first strand is closer to the duplex end with the toehold domain (a) of the first strand than the stopper molecule in the second strand.

In some embodiments of any one of the aspects, a nucleic acid described herein comprises a primer binding domain (d). The primer binding domain can be paired, i.e., part of the duplex region, or non-paired, i.e., single-stranded. A "primer-binding domain" of a nucleic acid is a sequence of nucleotides to which a complementary primer binds. The primer-binding domain can be of any desired length or sequence. For example, a primer-binding domain can be at least 4, at least 10, at least 15, at least 20, at least 25, at least 30, at least 35, at least 40, at least 45, at least 50, at least 55, at least 60, at least 65, at least 70, at least 75, at least 80, at least 85, at least 90 or more nucleotides in length. In some embodiments, a primer-binding domain is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less or 30 nucleotides or less in length. In some embodiments, a primer binding domain can be from about 4 nucleotides to about 50 nucleotides in length. For example, a primer-binding domain may have a length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. In some embodiments, a primer-binding domain has a length of 4 to 10, 4 to 15, 4 to 20, 4 to 25, 4 to 30, 4 to 35, 4 to 40, 4 to 45 or 4 to 50 nucleotides. In some embodiments, a primer-binding domain is longer than 40 nucleotides. For example, a primer-binding domain may have a length of 4 to 100 nucleotides. In some embodiments, a primer-binding domain has a length of 4 to 90, 4 to 80, 4 to 70, 4 to 60, or 4 to 50 nucleotides. A primer-binding domain can be designed to accommodate binding of more than one (e.g., 2 or 3 different) primer.

In some embodiments of any one of the aspects, the first strand of the double-stranded nucleic acid of the first reporter probe comprises a primer binding domain (c*) near or at an end distal to end with the toehold domain (a).

In some embodiments of any one of the aspects, the first strand of the double-stranded nucleic acid of the first reporter probe can comprise a domain divided into a first subdomain and a second subdomain (b) separated by stopper molecule or modification (•). For example, a primer binding domain (c*) can be linked to the first domain and the second domain (b) can be linked to the toehold domain (a). Thus, in some embodiments, the first strand comprises a primer binding domain (c*) linked to a first subdomain that is linked to a stopper molecule (•) that is linked to a second subdomain (b) that is linked to a toehold domain (a).

When present, the first subdomain and/or the second subdomain (b) of the first strand can be of any desired length or sequence. Further, the first subdomain and/or the second subdomain (b) of the first strand can be of same length or different lengths. For example, the first subdomain and/or the second subdomain (b) can be independently at least 4, at least 10, at least 15, at least 20, at least 25, at least 30, at least 35, at least 40, at least 45, at least 50, at least 55, at least 60, at least 65, at least 70, at least 75, at least 80, at least 85, at least 90 or more nucleotides in length. In some embodiments, the first subdomain and/or the second subdomain (b) is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less or 30 nucleotides or less in length. In some embodiments, the first subdomain and/or the second subdomain (b) can be from about 4 nucleotides to about 50 nucleotides in length. For example, the first subdomain and/or the second subdomain (b) may have a length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. In some embodiments, the first subdomain and/or the second subdomain (b) has a length of 4 to 10, 4 to 15, 4 to 20, 4 to 25, 4 to 30, 4 to 35, 4 to 40, 4 to 45 or 4 to 50 nucleotides. In some embodiments, the first subdomain and/or the second subdomain (b) is longer than 40 nucleotides. For example, the first subdomain and/or the second subdomain (b) may have a length of 4 to 100 nucleotides. In some embodiments, the first subdomain and/or the second subdomain (b) has a length of 4 to 90, 4 to 80, 4 to 70, 4 to 60, or 4 to 50 nucleotides.

In some embodiments, the first subdomain and/or the second subdomain (b) of the first strand can be a bar-code or unique molecule identifier (UMI) domain. For example, the second subdomain (b) of the first strand can be a bar-code or unique molecule identifier (UMI) domain.

A "barcode" domain or subdomain is a sequence of nucleotides that uniquely identifies a particular molecule. Barcodes may also be referred to in the art as "unique molecular identifiers" (UMIs). UMIs associate distinct sequences with a nucleic acid molecule and can be used to uniquely identify an amplified nucleic acid molecule. In some embodiments, the barcode domain may contain a nucleotide sequence that contains only three of the four nucleotides. For example, a barcode domain or subdomain may include (a) only As, Ts, and Cs, (b) only As, Ts, and Gs, (c) only Gs, Ts, and Cs, or (d) only As, Gs, and Cs, Thus, a barcode domain or subdomain may lack (may not include) one of A, T, C or G. The length of a barcode domain or subdomain may vary.

The second strand of the duplex comprises a nucleotide complementary to the first strand to form the duplex region. Thus, in some embodiments, the second strand comprises a first subdomain (b*) that is substantially complementary to the second subdomain (b) of the first strand.

In some embodiments, the second strand of the double-stranded nucleic acid of the first reporter probe comprises a second subdomain that is complementary to the first subdomain of the first strand. A stopper molecule (•) can be present between the first subdomain (b*) and the second subdomain of the second strand. In some embodiments, the second strand also comprises a "primer domain" (c) that is substantially complementary to the primer binding domain (c*) of the first strand.

In some embodiments, the second strand of the double-stranded nucleic acid of the first reporter probe comprises a first subdomain (b*) linked to a second subdomain that is linked to a stopper molecule (•) that is linked to a primer domain (c) that is linked to a third subdomain (b) that is linked to a toehold domain (x). For example, the second strand comprises the first subdomain (b*) at one end and the toehold domain (x) at the other end.

In some embodiments, the second strand of the double-stranded nucleic acid of the first reporter probe does not comprise a toehold domain. In other words, the second strand comprises a first subdomain (b*) linked to a second subdomain that is linked to a stopper molecule (•) that is linked to a primer domain (c) that is linked to a third subdomain (b). For example, the second strand comprises the first subdomain (b*) at one end and the primer domain (c) at the other end.

In some embodiments of any one of the aspects, the blocking probe comprises a blocking ligand and a blocking probe nucleic acid linked thereto. It is noted that the blocking probe nucleic acid can be linked covalently or non-covalently to the blocking ligand. The strand can be linked by its 5'-end or by its 3'-end. In some preferred embodiments, the strand is linked to the blocking ligand by its 5'-end.

Generally, the blocking probe nucleic acid linked to the blocking ligand comprises a pairing domain (x*) at an end distal to the linked end. The pairing domain (x*) is substantially complementary to the pairing domain (x) of the first reporter probe. In some embodiments, the pairing domain (x) of the first reporter probe and the pairing domain (x*) of the blocking probe are hybridized to each other.

In some embodiments of any one of the aspects, the blocking probe nucleic acid linked to the blocking ligand comprises a toehold domain (a) that is linked to a pairing domain (x*), wherein the pairing domain (x*) is at an end distal to the linked end. The toehold domain (a) of linking strand and the toehold domain (a) of first reporter probe can comprise a substantially identical nucleotide sequence. In other words, the toehold domain (a) of the blocking probe and the toehold domain (a) of the first reporter probe are substantially identical to each other.

In some embodiments, the methods, compositions, and kits described herein may include a second reporter probe. Generally, the second reporter probe comprises a second target binding ligand and a second nucleic acid linked thereto. It is noted that the second nucleic acid can be linked covalently or non-covalently to the second target binding ligand. The strand can be linked by its 5'-end or by its 3'-end. In some preferred embodiments, the second nucleic acid is linked to the target binding ligand by its 5'-end. Generally, the linking strandsecond nucleic acid comprises a primer binding domain (d*) linked to a toehold domain (a*). In some embodiments, the toehold domain (a*) is at the end distal to the end linked with the target binding ligand. The toehold domain (a*) of the second reporter probe is substantially complementary to the toehold domain (a) of the first reporter probe.

In some embodiments of any one of the aspects, the second reporter probe comprises a second target binding ligand and a second nucleic acid linked to the second target binding ligand. The second nucleic acid comprises a second nucleic acid first strand linked to the second target binding ligand and a second nucleic acid second strand hybridized with the first strand. It is noted that the first strand can be linked covalently or non-covalently to the second target binding ligand. The first strand can be linked by its 5'-end or by its 3'-end. In some preferred embodiments, the first strand is linked to the target binding ligand by its 5'-end. Generally, the first strand comprises a second nucleic acid first hybridizing domain linked to a pairing domain (x) at an end distal to the linked end. The pairing domain (x) is substantially identical to the pairing domain (x) of the first reporter probe. The second strand comprises a second nucleic acid second hybridizing domain linked to a primer binding domain (d*) linked to a toehold domain (a*). The hybridizing domains of the first strand (i.e., the linking strand) and the second strand are substantially complementary and hybridize to each other. In some embodiments, the toehold domain (a*) is at the end distal to the end hybridized with the linking strand. The toehold domain (a*) of the second reporter probe is substantially complementary to the toehold domain (a) of the first reporter probe.

In some embodiments, the second targeting binding ligand of the second reporter probe is a class specific antibody.

### Nucleic acid strand domains

Various nucleic acid described herein comprise one or more domains. As used herein, a "domain" refers to a discrete, contiguous sequence of nucleotides or nucleotide base pairs, depending on whether the domain is unpaired (contiguous stretch of nucleotides that are not bound to complementary nucleotides) or paired (contiguous stretch of nucleotide base pairs - nucleotides bound to complementary nucleotides), respectively. In some embodiments, a domain is described as having multiple subdomains for the purpose of defining intramolecular (within the same molecular species) and intermolecular (between two separate molecular species) complementarity. One domain (or one subdomain) is "complementary to" another domain (or another subdomain) if one domain contains nucleotides that base pair (hybridize/bind through Watson-Crick nucleotide base pairing) with nucleotides of the other domain such that the two domains form a paired (double-stranded) or partially-paired molecular species/structure. Complementary domains need not be perfectly (100%) complementary to form a paired structure, although perfect complementarity is provided, in some embodiments. It should be understood that "domain a," "domain b," "domain c," etc., refer to domains having nucleotide sequences that are different from each other. Thus, a "domain a" has a nucleotide sequence that is different from the nucleotide sequence of a "domain b." It should also be understood that "domain a" and "domain a*" refer to domains having nucleotide sequences that are complementary to each other (either partially or completely complementary) such that the two domains are capable of hybridizing (binding) to each other.

Domains or other discrete nucleotide sequences are considered "adjacent" to each other if they are contiguous with each other (there are no nucleotides separating the two domains), or if they are within 50 nucleotides (e.g., 1-50, 1-40, 1-30, 1-20, 1-10, 1-5) of each other. That is, in some embodiments, two domains may be considered adjacent if the two domains are separated from each other by 1, 2, 3, 4, 5, 6, 7, 8, 9, 10, 11, 12, 13, 14, 15, 16, 17, 18, 19, 20, 25, 30, 35, 40, 45 or 50 nucleotides.

Nucleotide domains and subdomains are described in terms of a 3' and/or 5' position relative to one another, or relative to the entire length of a nucleic acid strand.
It is noted that a domain described herein can be of any desired length or sequence. For example, a domain described herein can be at least 4, at least 10, at least 15, at least 20, at least 25, at least 30, at least 35, at least 40, at least 45, at least 50, at least 55, at least 60, at least 65, at least 70, at least 75, at least 80, at least 85, at least 90 or more nucleotides in length. In some embodiments, a domain described herein is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less or 30 nucleotides or less in length. In some embodiments, a domain described herein can be from about 4 nucleotides to about 50 nucleotides in length. For example, a domain described herein may have a length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. In some embodiments, a domain described herein has a length of 4 to 10, 4 to 15, 4 to 20, 4 to 25, 4 to 30, 4 to 35, 4 to 40, 4 to 45 or 4 to 50 nucleotides. In some embodiments, a domain described herein is longer than 40 nucleotides. For example, a domain described herein may have a length of 4 to 100 nucleotides. In some embodiments, a domain described herein has a length of 4 to 90, 4 to 80, 4 to 70, 4 to 60, or 4 to 50 nucleotides.

The methods, compositions, kits and systems described herein can be used to detect a single target or multiple targets. For example, at least 1, 2, 3, 4, 5, 6, 7, 8, 9, 10, 15, 20, 25, 30, 35, 40, 50, 60, 70, 80, 90 or 100 different targets can be detected, e.g., in a single reaction.

Advantageously, the methods, compositions, kits and systems described herein may be used to detect targets having low copy numbers present in a reaction. For example, a copy number of 10-100 (e.g., 10, 15, 20, 25, 30, 35, 40, 45, 50, 55, 60, 65, 70, 75, 80, 85, 90, 95 or 100) can be detected using the methods, compositions, kits and systems described herein.

### Nucleic acid modifications

At least one nucleic acid strand provided herein can independently comprise one or more nucleic acid modifications known in the art. For example, the nucleic acid strands can independently comprise non-naturally occurring nucleic acids and/or non-naturally occurring nucleotides and/or nucleotide analogs, and/or chemical modifications. Non-naturally occurring nucleic acids can include, for example, mixtures of naturally and non-naturally occurring nucleotides. Non-naturally occurring nucleotides and/or nucleotide analogs can be modified at the ribose, phosphate, and/or base moiety.

Exemplary nucleic acid modifications include, but are not limited to, nucleobase modifications, sugar modifications, inter-sugar linkage modifications, conjugates (*e.g.*, ligands), and combinations thereof. In one embodiment, a modification does not include replacement of a ribose sugar with a deoxyribose sugar as occurs in deoxyribonucleic acid. Nucleic acid modifications are known in the art, see, *e.g.*, US20160367702; US20190060458; U.S. Pat. No. 8,710,200; and U.S. Pat No. 7,423,142, which are incorporated herein by reference in their entireties.

Exemplary modified nucleobases include, but are not limited to, inosine, xanthine, hypoxanthine, nubularine, isoguanisine, tubercidine, and substituted or modified analogs of adenine, guanine, cytosine and uracil, such as 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, N6, N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N4-acetyl cytosine, 2-thiocytosine, N6-methyladenine, N6-isopentyladenine, 2-methylthio-N6-isopentenyladenine, N-methylguanines, or O-alkylated bases. Further purines and pyrimidines include those disclosed in U.S. Pat. No. 3,687,808, those disclosed in the Concise Encyclopedia of Polymer Science and Engineering, pages 858-859, Kroschwitz, J. I., ed. John Wiley & Sons, 1990, and those disclosed by Englisch et al., Angewandte Chemie, International Edition, 1991, 30, 613.

Exemplary sugar modifications include, but are not limited to, 2'-Fluoro, 3'-Fluoro, 2'-OMe, 3'-OMe, and acyclic nucleotides, *e.g.*, peptide nucleic acids (PNA), unlocked nucleic acids (UNA) or glycol nucleic acid (GNA).

In some embodiments, a nucleic acid modification can include replacement or modification of an inter-sugar linkage. Exemplary inter-sugar linkage modifications include, but are not limited to, phosphotriesters, methylphosphonates, phosphoramidate, phosphorothioates, methylenemethylimino, thiodiester, thionocarbamate, siloxane, N,N'-dimethylhydrazine (-CH2-N(CH3)-N(CH3)-), amide-3 (3'-CH2-C(=O)-N(H)-5') and amide-4 (3'-CH2-N(H)-C(=O)-5'), hydroxylamino, siloxane (dialkylsiloxxane), carboxamide, carbonate, carboxymethyl, carbamate, carboxylate ester, thioether, ethylene oxide linker, sulfide, sulfonate, sulfonamide, sulfonate ester, thioformacetal (3'-S-CH2-O-5'), formacetal (3 '-O-CH2-O-5'), oxime, methyleneimino, methykenecarbonylamino, methylenemethylimino (MMI, 3'-CH2-N(CH3)-O-5'), methylenehydrazo, methylenedimethylhydrazo, methyleneoxymethylimino, ethers (C3'-O-C5'), thioethers (C3'-S-C5'), thioacetamido (C3'-N(H)-C(=O)-CH2-S-C5', C3'-O-P(O)-O-SS-C5', C3'-CH2-NH-NH-C5', 3'-NHP(O)(OCH3)-O-5' and 3'-NHP(O)(OCH3)-O-5'.

In some embodiments of any of the aspects, 2'-modified nucleoside comprises a modification selected from the group consisting of 2'-halo (*e.g.*, 2'-fluoro), 2'-alkoxy (*e.g.*, 2'-Omethyl, 2'-Omethylmethoxy and 2'-Omethylethoxy), 2'-aryloxy, 2'-O-amine or 2'-O-alkylamine (amine NH₂; alkylamino, dialkylamino, heterocyclyl, arylamino, diaryl amino, heteroaryl amino, dihet.eroaryl amino, ethylene diamine or polyamino), O-CH₂CH₂(NCH₂CH₂NMe₂)₂, methyleneoxy (4'-CH₂-O-2') LNA, ethyleneoxy (4'-(CH₂)₂-O-2') ENA, 2'-amino (*e.g.* 2'-NH₂, 2'-alkylamino, 2'-dialkylamino, 2'-heterocyclylamino, 2'-arylamino, 2'-diaryl amino, 2'-heteroaryl amino, 2'-diheteroaryl amino, and 2'-amino acid); NH(CH₂CH₂NH)ₙCH₂CH₂-AMINE (AMINE = NH₂, alkylamino, dialkylamino, heterocyclyl, arylamino, diaryl amino, heteroaryl amino, or diheteroaryl amino), -NHC(O)R (R = alkyl, cycloalkyl, aryl, aralkyl, heteroaryl or sugar), 2'-cyano, 2'-mercapto, 2'-alkyl-thio-alkyl, 2'-thioalkoxy, 2'-thioalkyl, 2'-alkyl, 2'-cycloalkyl, 2'-aryl, 2'-alkenyl and 2'-alkynyl.

In some embodiments of any of the aspects, the inverted nucleoside is dT.

In some embodiments of any of the aspects, the 5'-modified nucleotide comprises a 5'-modification selected from the group consisting of 5'-monothiophosphate (phosphorothioate), 5'-monodithiophosphate (phosphorodithioate), 5'-phosphorothiolate, 5'-alpha-thiotriphosphate, 5'-beta-thiotriphosphate, 5'-gamma-thiotriphosphate, 5'-phosphoramidates, 5'-alkylphosphonate, 5'-alkyletherphosphonate, a detectable label, and a ligand; or the 3'-modified nucleotide comprises a 3'-modification selected from the group consisting of 3'-monothiophosphate (phosphorothioate), 3'-monodithiophosphate (phosphorodithioate), 3'-phosphorothiolate, 3'-alpha-thiotriphosphate, 3'-beta-thiotriphosphate, 3'-gamma-thiotriphosphate, 3'-phosphoramidates, 3'-alkylphosphonate, 3'-alkyletherphosphonate, and a detectable label.

In some embodiments, nucleic acid modifications can include peptide nucleic acids (PNA), bridged nucleic acids (BNA), morpholinos, locked nucleic acids (LNA), glycol nucleic acids (GNA), threose nucleic acids (TNA), or other xeno nucleic acids (XNA) described in the art.

It will be apparent to those skilled in the art that various modifications and variations can be made to improve the stability of the nucleic acid strands.

### Extension reaction

Embodiments of the methods described herein comprise extension of nucleic acid strands using a polymerase. Method of synthesizing nucleic acid from nucleic acid templates are well known in the art and available to one of ordinary skill in the art. See, for example, US20050277146A1, US20100035303A1, and WO2006030455A1, contents of all of which are incorporated herein by reference in their entirety

Generally, the extension step is carried out in presence of a polymerase and nucleotide triphosphates. All the components of a reaction can be provided in a reaction buffer. The concentration of components in a polymerase reaction composition, method or kit can be varied depending, for example, on the particular application and kinetics required for that particular application.

It is noted that kinetics of polymerization can be controlled by varying temperature, time, buffer/salt conditions, and deoxyribonucleotide triphosphate (dNTP) concentrations, for example. Polymerases, like most enzymes, are sensitive to many buffer conditions, including ionic strength, pH and types of metal ions present (*e.g.*, sodium ions vs. magnesium ions). Thus, the temperature at which extension step is performed can vary from, for example, 4°C to 65°C (*e.g.*, 4°C, 25°C, 37°C, 42°C or 65°C). In some embodiments of the various aspects, the temperature at which the extension step is performed is 4-25°C, 4-30°C, 4-35°C, 4-40°C, 4-45°C, 4-50°C, 4-55°C, 4-60°C, 10-25C, 10-30°C, 10-35°C, 10-40°C, 10-45°C, 10-50°C, 10-55°C, 10-60°C, 25-30°C, 25-35°C, 25-40°C, 25-45°C, 25-50°C, 25-55°C, 25-60°C, 25-65°C, 35-40°C, 35-45°C, 35-50°C, 35-55°C, 35-60°C, or 35-65°C. In some embodiments of the various aspects, the extension step is performed at room temperature. In some other embodiments, the extension step is performed at 37°C.

The extension step can be performed (incubated) for about 30 minutes to about 24 hours. In some embodiments of any of the aspects, the extension step can be carried out for about 1 minute, about 2 minutes, about 3 minutes, about 4 minutes, about 5 minutes, about 6 minutes, about 7 minutes, about 8 minutes, about 9 minutes, about 10 minutes, about 15 minutes, about 20 minutes, about 25 minutes, about 30 minutes, about 35 minutes, about 40 minutes, about 45 minutes, about 50 minutes, about 55 minutes, about 1 hour, about 2 hours, about 3 hours, about 4 hours, about 5 hours, about 6 hours, about 7 hours, about 8 hours, about 9 hours, about 10 hours, about 11 hours, about 12 hours, about 18 hours or about 24 hours.

In some embodiments of the various aspects described herein, concentration of dNTPs can be, for example, 2-1000 mM. For example, concentration of the dNTPs can be 2-10 pM, 2-15 pM, 2-20 pM, 2-25 pM, 2-30 pM, 2-35 pM, 2-40 pM, 2-45 pM, 2- 50 pM, 2-55 pM, 2-60 pM, 2-65 pM, 2-70 pM, 2-75 pM, 2-80 pM, 2-85 pM, 2-90 pM, 2-95 pM, 2-100 pM, 2-110 pM, 2-120 pM, 2-130 pM, 2-140 pM, 2-150 pM, 2-160 pM, 2-170 pM, 2-180 pM, 2-190 pM, 2-200 pM, 2-250 pM, 2-300 pM, 2-350 pM, 2-400 pM, 2-450 pM, 2-500 pM, 2-600 pM, 2-700 pM, 2-800 pM, 2-900 pM or 2-1000 pM. For example, concentration of the dNTPs can be 2 pM, 5 pM, 10 pM, 15 pM, 20 pM, 25 pM, 30 pM, 35 pM, 40 pM, 45 pM, 50 pM, 55 pM, 60 pM, 65 pM, 70 pM, 75 pM, 80 pM, 85 pM, 90 pM, 95 pM, 100 pM, 105 pM, 110 pM, 115 pM, 120 pM, 125 pM, 130 pM, 135 pM, 140 pM, 145 pM, 150 pM, 155 pM, 160 pM, 165 pM, 170 pM, 175 pM, 180 pM, 185 pM, 190 pM, 195 pM or 200 pM. In some embodiments of any of the aspects, concentration of the dNTPs can be 10-20 pM, 10-30 pM, 10-40 pM, 10-50 pM, 10-60 pM, 10-70 pM, 10-80 pM, 10-90 pM or 10-100 pM. It is noted that dNTP variants can also be used.

In some embodiments of the various aspects described herein, extension step comprises incubating the reaction under conditions that result in nucleic acid polymerization, strand displacement and annealing, for a time sufficient to produce a synthesized strand.

In some embodiments of any of the aspects, the polymerase is a DNA polymerase (DNAP), such as a DNA polymerase having DNA strand displacement activity (a strand displacing polymerase). "Strand displacement" describes the ability to displace downstream DNA encountered during synthesis. Examples of polymerases having DNA strand displacement activity that can be used as provided herein include, without limitation, phi29 DNA polymerase (*e.g.*, NEB #M0269), Bst DNA polymerase, large fragment (*e.g.*, NEB #M0275), or Bsu DNA polymerase, large fragment (*e.g.*, NEB #M0330). Other polymerases having strand displacement activity can be used.

In some embodiments of the various aspects described herein, the polymerase is phi29 DNA polymerase. In such embodiments, the reaction conditions can be as follows: reaction buffer (*e.g.*, 50 mM Tris-HCl, 10 mM MgCl₂, 10 mM (NH₄)₂S0₄, 4 mM DTT) supplement with purified bovine serum albumin (BSA), pH 7.5, incubated at 30°C.

In some embodiments of any of the aspects, the polymerase is Bst DNA polymerase, large fragment. In such embodiments, the reaction conditions can be as follows: IX reaction buffer (*e.g.*, 20 mM Tris-HCl, 10 mM (NH₄)₂S0₄, 10 mM KC1, 2 mM MgS0₄, 0.1% TRITON^{®} X-100), pH 8.8, incubated at 65°C.

In some embodiments of any of the aspects, the polymerase is Bsu DNA polymerase. In such embodiments, the reaction conditions can be as follows: reaction buffer (*e.g.*, 50 mM NaCl, 10 mM Tris-HCl, 10 mM MgCl₂, 1 mM DTT), pH 7.9, incubated at 37°C.

In some embodiments, the polymerase is Bst DNA polymerase Bsu, Bst 2.0, Bst 3.0, Bsu, phi29, SD polymerase, any combination or fragment thereof.

Other exemplary reaction conditions that can be used in the methods provided herein include but are not limited to: Thermopol^{®} buffer; NEB^{®} buffers 1,2,3,4; CutSmart^{®} buffer; Isothermal Amplification Buffer; and the like. Custom buffers can be made with 0.5 to 2X PBS; 5 to 200 mM Tris-HCl; 5-200 mM Potassium Acetate; 5-200 mM Magnesium Acetate; 5-200 mM Tris-Acetate; or 5-200 mM Bis-Tris-Propane-HCl can be used with the addition one or all of these additives to modulate the enzyme activity (*e.g.*, 1-50 mM KCl, 1-20 mM MgSO4, 1-20 mM MgCl2, 1-5 mM DTT, 0-500 ug/ml BSA, 1-500 NaCl, 0.01% to 0.5% Triton X-100 at pH values of 6-9.5). The buffer can also include dNTPs (*e.g.*, dATP, dCTP, dGTP and dTTP). When only 2-3 types of dNTPs are used, the omitted nucleotides serve as stoppers for the polymerase action, optionally with functional modifications.

### Detection of nucleic acid record

The nucleic acid record produced from the interaction between a nucleic acid strand in the first reporter probe and the nucleic acid strand of the second reporter probe can be detected using any method for detecting nucleic acid strands. Methods for detecting nucleic acids are well in the art and available to one of ordinary skill in the art. Exemplary methods for detecting nucleic acids include, but are not limited to, DNA sequencing, PCR-based techniques, and hybridization-based methods.

The nucleic acid record can be detected using a DNA binding dye. Exemplary DNA binding dyes include, but are not limited to, acridines (e.g., acridine orange, and acriflavine), actinomycin D (Jain, et al. J. Mol. Biol. 68:21 (1972), which is incorporated by reference herein in its entirety), anthramycin, B0B0^{™}-1, BOBO^{™}-3, B0-PR0^{™}-1, cbromomycin, DAPI (Kapuseinski, et al. Nucl. Acids Res. 6(112): 3519 (1979), which is incorporated by reference herein in its entirety), daunomycin, distamycin (e.g., distamycin D), dyes described in U.S. Patent No. 7,387,887, which is incorporated by reference herein in its entirety, ellipticine, ethidium salts (e.g., ethidium bromide), fluorcoumanin, fluorescent intercalators as described in U.S. Patent No. 4,257,774, which is incorporated by reference herein in its entirety, GelStar^{®} (Cambrex Bio Science Rockland Inc., Rockland, Me.), Hoechst 33258 (Searle and Embrey, Nucl. Acids Res. 18:3753-3762 (1990), which is incorporated by reference herein in its entirety), Hoechst 33342, homidium, JO-PRO^{™}- 1, LIZ dyes, LO-PRO^{™}-l, mepacrine, mithramycin, NED dyes, netropsin, 4',6-diamidino-a-phenylindole, proflavine, POPO^{™}-l, POPO^{™}-3, PO- PRO^{™}-l, propidium iodide, ruthenium polypyridyls, S5, SYBR^{®} Gold, SYBR^{®} Green I (U.S. Patent No. 5,436,134 and 5,658,751), SYBR^{®} Green II, SYTOX blue, SYTOX green, SYTO^{®} 43, SYTO^{®} 44, SYTO^{®} 45, SYTOX^{®} Blue, TO-PRO^{®}-l, SYTO^{®} 11, SYTO^{®} 13, SYTO^{®} 15, SYTO^{®} 16, SYTO^{®} 20, SYTO^{®} 23, thiazole orange (Aldrich Chemical Co., Milwaukee, Wis ), TOTO^{™}-3, YO-PRO^{®}-l, and YOYO^{®}-3 (Molecular Probes, Inc., Eugene, OR), among others. SYBR^{®} Green I (see, e.g., U.S. Patent Nos. 5,436, 134; 5,658,751; and or 6,569,927, which are incorporated by reference herein in their entireties), for example, has been used to monitor a PCR reactions. Other DNA binding dyes may also be suitable as would be understood by one of skill in the art. See, for example, U.S. Patent Nos. 4,883,867; 5,658,751; 8,865,904; 8,883,415; 9,040,561; and 9,115,397, which are incorporated by reference herein in their entireties. Optionally, the DNA binding dye is OliGreen or PicoGreen. See, e.g., U.S. Patent No. 5,436,134, which is incorporated by reference herein in its entirety.

In some embodiments, the nucleic acid record can be using sequencing methods. Sequencing methods are known and can be performed with a variety of platforms including, but not limited to, platforms provided by Ulumina, Inc., (La Jolla, CA) or Life Technologies (Carlsbad, CA). See, e.g., Wang, et al., Nat Rev Genet. 10(l):57-63 (2009); and Martin, Nat Rev Genet. 12(10):671-82 (2011), which are incorporated by reference herein in their entireties. Optionally, methods for detecting the nucleic acid record include microarray methods, which are known and can be performed with a variety of platforms including, but not limited to, platforms provided by Ambion, Inc., (Austin, TX) and Life Technologies (Carlsbad, CA).

### Amplification of nucleic acid record

In some embodiments, the method further comprises a step of amplifying the nucleic acid record, e.g., prior to detecting the nucleic acid record. As used herein, the term "amplifying" refers to a step of submitting a nucleic acid strand to conditions sufficient to allow for amplification of a polynucleotide if all of the components of the reaction are intact. Components of an amplification reaction include, *e.g.*, primers, a polynucleotide template, polymerase, nucleotides, and the like. The term "amplifying" typically refers to an "exponential" increase in target nucleic acid. However, "amplifying" as used herein can also refer to linear increases in the numbers of a select target sequence of nucleic acid, such as is obtained with cycle sequencing. Methods of amplifying and synthesizing nucleic acid sequences are known in the art. For example, see US Patent Nos. 7,906.282, 8,367,328, 5,518,900, 7,378,262, 5,476,774, and 6,638,722, contents of all of which are incorporated by reference herein in their entirety. Such methods include, but are not limited to, isothermal amplification, polymerase chain reaction (PCR) and variants of PCR such as Rapid amplification of cDNA ends (RACE), ligase chain reaction (LCR), multiplex RT-PCR, immuno-PCR, SSIPA, qPCR, Real Time RT-qPCR and nanofluidic digital PCR. Accordingly, the methods described herein comprise a step of contacting the sample with a DNA polymerase and a set of primers. In some embodiments of any of the aspects, a set of primers comprises at least 2 primers and comprises a forward primer and reverse primer that amplify a target of about 50 base pairs (bp) to about 50,000 bp, unless indicated otherwise.

In some embodiments of any of the aspects, the amplification step comprises isothermal amplification. As used herein, "isothermal amplification" refers to amplification that occurs at a single temperature. For example, the amplification process is performed at a single temperature or where the major aspect of the amplification process is performed at a single temperature. Generally, isothermal amplification relies on the ability of a polymerase to copy the template strand being amplified to form a bound duplex. Isothermal amplification permits rapid and specific amplification of a target nucleic acid at a constant temperature. In general, isothermal amplification is comprised of (i) sequence-specific hybridization of primers to sequences within a target nucleic acid, and (ii) subsequent amplification involving multiple rounds of primer annealing, elongation, and strand displacement (as a non-limiting example, using a combination of recombinase, single-stranded binding proteins, and DNA polymerase). The primers used in isothermal amplification are oligonucleotides of sufficient length and appropriate sequence to provide initiation of polymerization, i.e. each primer is specifically designed to be complementary to a strand of the target nucleic acid to be amplified.

Non-limiting examples of isothermal amplification include: Loop Mediated Isothermal Amplification (LAMP), Recombinase Polymerase Amplification (RPA), Helicase-dependent isothermal DNA amplification (HDA), Rolling Circle Amplification (RCA), Nucleic acid sequence-based amplification (NASBA), strand displacement amplification (SDA), nicking enzyme amplification reaction (NEAR), and polymerase Spiral Reaction (PSR). See *e.g.*, Yan et al., Isothermal amplified detection of DNA and RNA, March 2014, Molecular BioSystems 10(5), DOI: 10.1039/c3mb70304e, the content of which is incorporated herein by reference in its entirety.

In some embodiments of any of the aspects, the isothermal amplification reaction(s) is Loop Mediated Isothermal Amplification (LAMP), i.e., the step of amplifying the target nucleic acids comprises Loop Mediated Isothermal Amplification. LAMP is a single tube technique for the amplification of DNA; LAMP uses 4-6 primers, which form loop structures to facilitate subsequent rounds of amplification. Accordingly, in some embodiments of the aspects, the amplification step comprises contacting the sample with a DNA polymerase and a set of primers, wherein the set of primers comprises 4, 5, or 6 loop-forming primers.

In some embodiments of any of the aspects, the isothermal amplification reaction(s) is Recombinase Polymerase Amplification (RPA), i.e., the step of amplifying the target nucleic acids comprises Recombinase Polymerase Amplification. RPA is a low temperature DNA and RNA amplification technique. The RPA process employs three core enzymes - a recombinase, a single-stranded DNA-binding protein (SSB) and strand-displacing polymerase. Recombinases are capable of pairing oligonucleotide primers with homologous sequence in duplex DNA. SSB bind to displaced strands of DNA and prevent the primers from being displaced. Finally, the strand displacing polymerase begins DNA synthesis where the primer has bound to the target DNA. By using two opposing primers, much like PCR, if the target sequence is indeed present, an exponential DNA amplification reaction is initiated. No other sample manipulation such as thermal or chemical melting is required to initiate amplification. At optimal temperatures (*e.g.*, 37-42 °C), the RPA reaction progresses rapidly and results in specific DNA amplification from just a few target copies to detectable levels, typically within 10 minutes, for rapid detection of the target nucleic acid. In some embodiments of any of the aspects, the single-stranded DNA-binding protein is a gp32 SSB protein. In some embodiments of any of the aspects, the recombinase is a uvsX recombinase. See *e.g.*, US Patent 7,666,598, the content of which is incorporated herein by reference in its entirety. In some embodiments of any of the aspects, RPA can also be referred to as Recombinase Aided Amplification (RAA). Accordingly, in some embodiments of any of the aspects, the amplification step comprises contacting the sample with a recombinase and single-stranded DNA binding protein. In some embodiments of any of the aspects, the amplification step comprises contacting the sample with a DNA polymerase, a set of primers, a recombinase, and single-stranded DNA binding protein.

In some embodiments of any of the aspects, the isothermal amplification reaction(s) is Helicase-dependent isothermal DNA amplification (HDA). HDA uses the double-stranded DNA unwinding activity of a helicase to separate strands for *in vitro* DNA amplification at constant temperature. In some embodiments of any of the aspects, the helicase is a thermostable helicase, which can improve the specificity and performance of HDA; as such, the isothermal amplification reaction(s) can be thermophilic helicase-dependent amplification (tHDA). As a non-limiting example, the helicase is the thermostable UvrD helicase (Tte-UvrD), which is stable and active from 45 to 65 °C. Accordingly, in some embodiments of the aspects, the amplification step comprises contacting the sample with a DNA polymerase, a set of primers, and a helicase, wherein the helicase is optionally a thermostable helicase.

In some embodiments of any of the aspects, the isothermal amplification reaction(s) is Rolling Circle Amplification (RCA). RCA starts from a circular DNA template and a short DNA or RNA primer to form a long single stranded molecule. Accordingly, in some embodiments of the aspects, the amplification step comprises contacting the sample (*e.g.*, a circular DNA) with a DNA polymerase and a set of primers, wherein the second set of primers comprises a single primer.

In some embodiments of any of the aspects, the isothermal amplification reaction(s) is Nucleic acid sequence-based amplification (NASBA), which is also known as transcription mediated amplification (TMA). NASBA is an isothermal technique predominantly used for the amplification of RNA through the cyclic formation of complimentary DNA and destruction of original RNA sequence (*e.g.*, using RNase H). The NASBA reaction mixture contains three enzymes-reverse transcriptase (RT), RNase H, and T7 RNA polymerase-and two primers. T7 RNA Polymerase is an RNA polymerase from the T7 bacteriophage that catalyzes the formation of RNA from DNA in the 5'→ 3' direction. Primer 1 (P1) contains a 3' terminal sequence that is complementary to a sequence on the target nucleic acid and a 5' terminal (+)sense sequence of a promoter that is recognized by the T7 RNA polymerase. Primer 2 (P2) contains a sequence complementary to the P1-primed DNA strand. The NASBA enzymes and primers operate in concert to amplify a specific nucleic acid sequence exponentially. NASBA results in the amplification of the target RNA to cDNA to RNA to cDNA, etc., with alternating reverse transcription (*e.g.*, RNA to DNA) and transcription steps (*e.g.*, DNA to RNA), and the RNA being degraded after each transcription. Accordingly, in some embodiments of the aspects, the amplification step comprises contacting the sample (*e.g.*, a cDNA) with an RNA polymerase, a reverse transcriptase, RNaseH, and a set of primers, wherein the set of primers comprise a 5' sequence that is recognized by the RNA polymerase.

In some embodiments of any of the aspects, the isothermal amplification reaction(s) is Strand Displacement Amplification (SDA). SDA is an isothermal, *in vitro* nucleic acid amplification technique based upon the ability of the restriction endonuclease HincII to nick the unmodified strand of a hemiphosphorothioate form of its recognition site, and the ability of exonuclease deficient klenow (exo-klenow) DNA polymerase to extend the 3'-end at the nick and displace the downstream DNA strand. Exponential amplification results from coupling sense and antisense reactions in which strands displaced from a sense reaction serve as target for an antisense reaction and vice versa. Accordingly, in some embodiments of the aspects, the amplification step comprises contacting the sample with a DNA polymerase (*e.g.*, exo-klenow), a set of primers, and a restriction endonuclease (*e.g.*, HincII).

In some embodiments of any of the aspects, the isothermal amplification reaction(s) is nicking enzyme amplification reaction (NEAR), which is a similar approach to SDA. In NEAR, DNA is amplified at a constant temperature (*e.g.*, 55 °C to 59 °C) using a polymerase and nicking enzyme. The nicking site is regenerated with each polymerase displacement step, resulting in exponential amplification. Accordingly, in some embodiments of the aspects, the amplification step comprises contacting the sample with a DNA polymerase (*e.g.*, exo-klenow), a set of primers, and a nicking enzyme (*e.g.*, N.BstNBI).

In some embodiments of any of the aspects, the isothermal amplification reaction(s) is Polymerase Spiral Reaction (PSR). The PSR method employs a DNA polymerase (*e.g.*, Bst) and a pair of primers. The forward and reverse primer sequences are reverse to each other at their 5' end, whereas their 3' end sequences are complementary to their respective target nucleic acid sequences. The PSR method is performed at a constant temperature 61 °C-65 °C, yielding a complicated spiral structure. Accordingly, in some embodiments of the aspects, the amplification step comprises contacting the sample with a DNA polymerase (*e.g.*, exo-klenow) and a set of primers that are reverse to each other at their 5' end.

In some embodiments of any of the aspects, the isothermal amplification reaction(s) is polymerase cross-linking spiral reaction (PCLSR). PCLSR uses three primers (*e.g.*, two outer-spiral primers and a cross-linking primer) to produce three independent prerequisite spiral products, which can be cross-linked into a final spiral amplification product. Accordingly, in some embodiments of the aspects, the amplification step comprises contacting the sample with a DNA polymerase and a set of primers (*e.g.*, two outer-spiral primers and a cross-linking primer).

Thus, the methods, compositions and kits provided herein can comprise a primer or a primer set that amplify the nucleic acid record, creating many copies of the record.

### Targets

The methods, compositions, kits and/or systems described herein can be used for detecting any desired molecule. For example, any target of interest can be detected using the methods, compositions, kits and/or systems described herein provided that the target has at least two binding sites for binding to a member of the first reporter probe and a member of the second reporter probe. The examples included in the disclosure depicting detection of a neutralizing antibody are for the purpose of illustration and are not intended to limit the scope of the invention.

Examples of targets include, but are not limited to, proteins, saccharides (e.g., polysaccharides), lipids, nucleic acids (e.g., DNA, RNA, microRNAs), small molecules, and antigens. In some embodiments, the target is a biomolecule. As used herein, a "biomolecule" is any molecule that is produced by a living organism, including large macromolecules such as proteins, e.g., antibodies, polysaccharides, lipids and nucleic acids (e.g., DNA and RNA such as mRNA), as well as small molecules such as primary metabolites, secondary metabolites, and natural products. In some embodiments, the target is an antibody. In some embodiments, the target is a small molecule.

In one embodiment, the target is an antigen. Antigens are molecules or molecular structures that are present on the outer surface of a pathogen; binding of an antigen by an antigen-specific antibody or receptor can trigger an immune response. Antigens can be proteins, peptides, and/or polysaccharides. Exemplary antigens include exogenous antigens, endogenous antigens, autoantigens, alloantigens, heterophile antigens, superantigens, immunogens (complete antigens), and haptens (incomplete antigens). In one embodiment, the antigen is comprised in a vaccine and/or is encoded in a vaccine. In one embodiment, the antigen is encoded by an RNA vaccine. In one embodiment, the antigen is encoded by a DNA vaccine.

In one embodiment, the antigen is a spike protein, i.e., an outward protruding protein structure of a viral envelope. Spike proteins are also known as S-glycoproteins.

The target can be a molecule produced by a subject in response to an infection. For example, the target can be a neutralizing antibody. The term "neutralizing antibody" refers to an antibody that is capable of keeping an infectious agent, usually a virus from infecting a cell by neutralizing or inhibiting its biological effect, for example by blocking the receptors on the cell or the virus. Neutralization can happen when antibodies bind to specific viral antigens, blocking the pathogen from entering their host cells.

Additional examples of targets include, without limitation, DNA, RNA, cDNA, or the DNA product of RNA subjected to reverse transcription, A23187 (Calcimycin, Calcium Ionophore), Abamectine, Abietic acid, Acetic acid, Acetylcholine, Actin, Actinomycin D, Adenosine, Adenosine diphosphate (ADP), Adenosine monophosphate (AMP), Adenosine triphosphate (ATP), Adenylate cyclase, Adonitol, Adrenaline, epinephrine, Adrenocorticotropic hormone (ACTH), Aequorin, Aflatoxin, Agar, Alamethicin, Alanine, Albumins, Aldosterone, Aleurone, Alphaamanitin, Allantoin, Allethrin, a-Amanatin, Amino acid, Amylase, Anabolic steroid, Anethole, Angiotensinogen, Anisomycin, Antidiuretic hormone (ADH), Arabinose, Arginine, Ascomycin, Ascorbic acid (vitamin C), Asparagine, Aspartic acid, Asymmetric dimethylarginine, Atrial-natriuretic peptide (ANP), Auxin, Avidin, Azadirachtin A-C35H44O16, Bacteriocin, Beauvericin, Bicuculline, Bilirubin, Biopolymer, Biotin (Vitamin H), Brefeldin A, Brassinolide, Brucine, Cadaverine, Caffeine, Calciferol (Vitamin D), Calcitonin, Calmodulin, Calmodulin, Calreticulin, Camphor-(C10H16O), Cannabinol, Capsaicin, Carbohydrase, Carbohydrate, Carnitine, Carrageenan, Casein, Caspase, Cellulase, Cellulose-(C6H10O5), Cerulenin, Cetrimonium bromide (Cetrimide)-C19H42BrN, Chelerythrine, Chromomycin A3, Chaparonin, Chitin, -Chloralose, Chlorophyll, Cholecystokinin (CCK), Cholesterol, Choline, Chondroitin sulfate, Cinnamaldehyde, Citral, Citric acid, Citrinin, Citronellal, Citronellol, Citrulline, Cobalamin (vitamin B12), Coenzyme, Coenzyme Q, Colchicine, Collagen, Coniine, Corticosteroid, Corticosterone, Corticotropin releasing hormone (CRH), Cortisol, Creatine, Creatine kinase, Crystallin, a-Cyclodextrin, Cyclodextrin glycosyltransferase, Cyclopamine, Cyclopiazonic acid, Cysteine, Cystine, Cytidine, Cytochalasin, Cytochalasin E, Cytochrome, Cytochrome C, Cytochrome c oxidase, Cytochrome c peroxidase, Cytokine, Cytosine-C4H5N3O, Deoxycholic acid, DON (DeoxyNivalenol), Deoxyribofuranose, Deoxyribose, Deoxyribose nucleic acid (DNA), Dextran, Dextrin, DNA, Dopamine, Enzyme, Ephedrine, Epinephrine-C9H13NO3, Erucic acid-CH3(CH2)7CH=CH(CH2)11COOH, Erythritol, Erythropoietin (EPO), Estradiol, Eugenol, Fatty acid, Fibrin, Fibronectin, Folic acid (Vitamin M), Follicle stimulating hormone (FSH), Formaldehyde, Formic acid, Formnoci, Fructose, Fumonisin B1, Gamma globulin, Galactose, Gamma globulin, Gamma-aminobutyric acid, Gamma-butyrolactone, Gamma-hydroxybutyrate (GHB), Gastrin, Gelatin, Geraniol, Globulin, Glucagon, Glucosamine, Glucose-C6H12O6, Glucose oxidase, Gluten, Glutamic acid, Glutamine, Glutathione, Gluten, Glycerin (glycerol), Glycine, Glycogen, Glycolic acid, Glycoprotein (e.g., glycoprotein enzymes such as prostate-specific antigen (PSA)), Gonadotropin-releasing hormone (GnRH), Granzyme, Green fluorescent protein, Growth hormone, Growth hormone releasing hormone (GHRH), GTPase, Guanine, Guanosine, Guanosine triphosphate (+GTP), Haptoglobin, Hematoxylin, Heme, Hemerythrin, Hemocyanin, Hemoglobin, Hemoprotein, Heparan sulfate, High density lipoprotein, HDL, Histamine, Histidine, Histone, Histone methyltransferase, HLA antigen, Homocysteine, Hormone, human chorionic gonadotropin (hCG), Human growth hormone, Hyaluronate, Hyaluronidase, Hydrogen peroxide, 5-Hydroxymethylcytosine, Hydroxyproline, 5-Hydroxytryptamine, Indigo dye, Indole, Inosine, Inositol, Insulin, Insulin-like growth factor, Integral membrane protein, Integrase, Integrin, Intein, Interferon, Inulin, Ionomycin, Ionone, Isoleucine, Iron-sulfur cluster, K252a, K252b, KT5720, KT5823, Keratin, Kinase, Lactase, Lactic acid, Lactose, Lanolin, Lauric acid, Leptin, Leptomycin B, Leucine, Lignin, Limonene, Linalool, Linoleic acid, Linolenic acid, Lipase, Lipid, Lipid anchored protein, Lipoamide, Lipoprotein, Low density lipoprotein, LDL, Luteinizing hormone (LH), Lycopene, Lysine, Lysozyme, Malic acid, Maltose, Melatonin, Membrane protein, Metalloprotein, Metallothionein, Methionine, Mimosine, Mithramycin A, Mitomycin C, Monomer, Mycophenolic acid, Myoglobin, Myosin, Natural phenols, Nucleic Acid, Ochratoxin A, Oestrogens, Oligopeptide, Oligomycin, Orcin, Orexin, Ornithine, Oxalic acid, Oxidase, Oxytocin, p53, PABA, Paclitaxel, Palmitic acid, Pantothenic acid (vitamin B5), parathyroid hormone (PTH), Paraprotein, Pardaxin, Parthenolide, Patulin, Paxilline, Penicillic acid, Penicillin, Penitrem A, Peptidase, Pepsin, Peptide, Perimycin, Peripheral membrane protein, Perosamine, Phenethylamine, Phenylalanine, Phosphagen, phosphatase, Phospholipid, Phenylalanine, Phytic acid, Plant hormones, Polypeptide, Polyphenols, Polysaccharides, Porphyrin, Prion, Progesterone, Prolactin (PRL), Proline, Propionic acid, Protamine, Protease, Protein, Proteinoid, Putrescine, Pyrethrin, Pyridoxine or pyridoxamine (Vitamin B6), Pyrrolysine, Pyruvic acid, Quinone, Radicicol, Raffinose, Renin, Retinene, Retinol (Vitamin A), Rhodopsin (visual purple), Riboflavin (vitamin B2), Ribofuranose, Ribose, Ribozyme, Ricin, RNA-Ribonucleic acid, RuBisCO, Safrole, Salicylaldehyde, Salicylic acid, Salvinorin A-C23H28O8, Saponin, Secretin, Selenocysteine, Selenomethionine, Selenoprotein, Serine, Serine kinase, Serotonin, Skatole, Signal recognition particle, Somatostatin, Sorbic acid, Squalene, Staurosporin, Stearic acid, Sterigmatocystin, Sterol, Strychnine, Sucrose (sugar), Sugars (in general), superoxide, tau protein, T2 Toxin, Tannic acid, Tannin, Tartaric acid, Taurine, Tetrodotoxin, Thaumatin, Topoisomerase, Tyrosine kinase, Taurine, Testosterone, Tetrahydrocannabinol (THC), Tetrodotoxin, Thapsigargin, Thaumatin, Thiamine (vitamin B1)-C12H17ClN4OS.HCl, Threonine, Thrombopoietin, Thymidine, Thymine, Triacsin C, Thyroid-stimulating hormone (TSH), Thyrotropin-releasing hormone (TRH), Thyroxine (T4), Tocopherol (Vitamin E), Topoisomerase, Triiodothyronine (T3), Transmembrane receptor, Trichostatin A, Trophic hormone, Trypsin, Tryptophan, Tubulin, Tunicamycin, Tyrosine, Ubiquitin, Uracil, Urea, Urease, Uric acid-C5H4N4O3, Uridine, Valine, Valinomycin, Vanabins, Vasopressin, Verruculogen, Vitamins (in general), Vitamin A (retinol), Vitamin B, Vitamin B1 (thiamine), Vitamin B2 (riboflavin), Vitamin B3 (niacin or nicotinic acid), Vitamin B4 (adenine), Vitamin B5 (pantothenic acid), Vitamin B6 (pyridoxine or pyridoxamine), Vitamin B12 (cobalamin), Vitamin C (ascorbic acid), Vitamin D (calciferol), Vitamin E (tocopherol), Vitamin F, Vitamin H (biotin), Vitamin K (naphthoquinone), Vitamin M (folic acid), Wortmannin and Xylose.

In some embodiments, the target is a protein such as, for example, proteins of a cellular environment (e.g., intracellular or membrane proteins). Examples of proteins include, without limitation, fibrous proteins such as cytoskeletal proteins (e.g., actin, arp2/3, coronin, dystrophin, FtsZ, keratin, myosin, nebulin, spectrin, tau, titin, tropomyosin, tubulin and collagen) and extracellular matrix proteins (e.g., collagen, elastin, f-spondin, pikachurin, and fibronectin); globular proteins such as plasma proteins (e.g., serum amyloid P component and serum albumin), coagulation factors (e.g., complement proteins,C1-inhibitor and C3-convertase, Factor VIII, Factor XIII, fibrin, Protein C, Protein S, Protein Z, Protein Z-related protease inhibitor, thrombin, Von Willebrand Factor) and acute phase proteins such as C-reactive protein; hemoproteins; cell adhesion proteins (e.g., cadherin, ependymin, integrin, Ncam and selectin); transmembrane transport proteins (e.g., CFTR, glycophorin D and scramblase) such as ion channels (e.g., ligand-gated ion channels such nicotinic acetylcholine receptors and GABAa receptors, and voltage-gated ion channels such as potassium, calcium and sodium channels), synport/antiport proteins (e.g., glucose transporter); hormones and growth factors (e.g., epidermal growth factor (EGF), fibroblast growth factor (FGF), vascular endothelial growth factor (VEGF), peptide hormones such as insulin, insulin-like growth factor and oxytocin, and steroid hormones such as androgens, estrogens and progesterones); receptors such as transmembrane receptors (e.g., G-protein-coupled receptor, rhodopsin) and intracellular receptors (e.g., estrogen receptor); DNA-binding proteins (e.g., histones, protamines, CI protein); transcription regulators (e.g., c-myc, FOXP2, FOXP3, MyoD and P53); immune system proteins (e.g., immunoglobulins, major histocompatibility antigens and T cell receptors); nutrient storage/transport proteins (e.g., ferritin); chaperone proteins; and enzymes.

### Sample/specimen

Described herein are methods, compositions, kits, and systems for detection of a target in a sample. The term "sample" or "test sample" as used herein can denote a sample taken or isolated from a biological organism, *e.g.*, a subject in need of testing.

Exemplary biological samples include tissue samples, such as liver, spleen, kidney, lung, intestine, thymus, colon, tonsil, testis, skin, brain, heart, muscle, and pancreas tissue. Other exemplary biological samples include, but are not limited to, biopsies, bone marrow samples, organ samples, skin fragments and organisms. Materials obtained from clinical or forensic settings are also within the intended meaning of the term biological sample. In one embodiment, the sample is derived from a human, animal or plant. In one embodiment, the biological sample is a tissue sample, preferably an organ tissue sample. In one embodiment, samples are human. The sample can be obtained, for example, from autopsy, biopsy, muscle punch, or from surgery. It can be a solid tissue or solid tumor such as parenchyme, connective or fatty tissue, heart or skeletal muscle, smooth muscle, skin, brain, nerve, kidney, liver, spleen, breast, carcinoma (e.g., bowel, nasopharynx, breast, lung, stomach etc.), cartilage, lymphoma, meningioma, placenta, prostate, thymus, tonsil, umbilical cord or uterus. The tissue can be a tumor (benign or malignant), cancerous or precancerous tissue. The sample can be obtained from an animal or human subject affected by disease or other pathology or suspected of same (normal or diseased), or considered normal or healthy.

In some embodiments of any of the aspects, the biological sample is a sputum sample, a pharyngeal sample, or a nasal sample. In some embodiments of any of the aspects, the biological sample is cells, or tissue, or peripheral blood, or bodily fluid. In some embodiments, the biological sample is a biopsy, a tumor sample, biofluid sample; blood; serum; plasma; urine; semen; mucus; tissue biopsy; organ biopsy; synovial fluid; bile fluid; cerebrospinal fluid; mucosal secretion; effusion; sweat; saliva; interstitial fluid; or tissue sample. The term also includes a mixture of the above-mentioned samples. The term "test sample" also includes untreated or pretreated (or pre-processed) biological samples.

In one embodiment, the blood sample is a dried blood spot sample. Methods for obtaining a dried blood spot are known in the art, and generally involves spotting at least 5, 10, 15 or more microliters of blood onto Whatman 31ETF card paper, and allowing it to air dry for at least 0.25, 0.5, 1, 2 3, or more hours. Methods for obtaining a dried blood spot sample are further described in, e.g., US Patent Nos 8,093,062; 9,110,053; 9,207,151; 10,531,821; 11,000,850; which are incorporated herein by reference in their entireties.

In various embodiments, when a dried blood spot is used, the sample droplet is prepared by reconstituting a dried blood spot disc in a solution, for example, an elution buffer. In one embodiment, the elution buffer consists of the following:

| **Component** | **Volume** |
|---|---|
| Nuclease free water | 58% |
| 10xPBS, pH7.4 | 10% |
| 10% NP40 | 2% |
| 10% Protease free, IgG free BSA | 10% |
| 5x EDTA-free Protease inhibitor | 20% |

In some cases, the disc has a diameter of less than about 10 mm and is reconstituted in less than about 1000 µL of solution; or less than about 750 µL of solution; or less than about 500 µL of solution; or less than about 250 µL of solution; or ranging from about 25 µL to about 750 µL; or ranging from about 25 µL to about 500 µL; or ranging from about 25 µL to about 250 µL; or ranging from about 25 µL to about 150 µL.

In some cases, the sample comprises at least 1, 2, 3, 4, 5, 6, 7, 8, 9, 10 or more dried blood spots samples.

In some cases, the dried blood spot disc has a diameter ranging from about 1 mm to about 15 mm; or from about 1 mm to about 8 mm; or from about 1 mm to about 6 mm; or from about 1 mm to about 4 mm. The disc is prepared by depositing blood on a substrate. In one embodiment, the dried blood spot is made using less than about 10 mL of blood or less than about 1 mL of blood or less than about 100 µL.

In one embodiment, the assays described herein are performed on a sample comprised in a single vessel substrate. In one embodiment, the assays described herein are performed on a sample comprised in a multi-vessel substrate, e.g., a 24-well, 96-well, 384-well, or more plate.

In some embodiments of any of the aspects, the test sample can be an untreated test sample. As used herein, the phrase "untreated test sample" refers to a test sample that has not had any prior sample pre-treatment except for dilution and/or suspension in a solution. Exemplary methods for treating a test sample include, but are not limited to, centrifugation, filtration, sonication, homogenization, heating, freezing and thawing, and combinations thereof. In some embodiments of any of the aspects, the test sample can be a frozen test sample. The frozen sample can be thawed before employing methods, assays and systems described herein. After thawing, a frozen sample can be centrifuged before being subjected to methods, assays and systems described herein. In some embodiments of any of the aspects, the test sample is a clarified test sample, for example, by centrifugation and collection of a supernatant comprising the clarified test sample. In some embodiments of any of the aspects, a test sample can be a pre-processed test sample, for example, supernatant or filtrate resulting from a treatment selected from the group consisting of centrifugation, homogenization, sonication, filtration, thawing, purification, and any combinations thereof. In some embodiments of any of the aspects, the test sample can be treated with a chemical and/or biological reagent. Chemical and/or biological reagents can be employed, for example, to protect and/or maintain the stability of the sample, including biomolecules (*e.g.*, nucleic acid and protein) therein, during processing. The skilled artisan is well aware of methods and processes appropriate for pre-processing of biological samples required for detection of targets, such as neutralizing antibodies as described herein.

### Compositions and kits

Also provided herein are compositions, kits and systems for detecting a target using a method described herein.

In some embodiments, the composition comprises at least one of a first reporter probe, a second reporter probe and a blocking probe described herein.

A composition described herein can further comprise one or more reagents/components for nucleic acid polymerization and/or linking. Accordingly, in some embodiments of the various aspects described herein, the composition further comprises a polymerase. For example, a DNA polymerase. In some embodiments of any of the aspects, the polymerase is a polymerase having strand displacing activity.

In some embodiments of any of the aspects, the composition further comprises one or more reagents for nucleic acid polymerization and/or amplification, such as deoxyribonucleotide triphosphates (dNTPs), salt and/or buffers.

In some embodiments, the composition is a dry composition.

In some embodiments, the composition further comprises a target, e.g., a reference target.

In one aspect, provided herein is a kit for detecting a target in a sample. The kit can comprise any of one of the compositions provided herein and packaging and materials therefore. Accordingly, in some embodiments, the kit comprises at least one of a first reporter probe, a second reporter probe and a blocking probe described herein.

In some embodiments, the kit can further comprise one or more reagents/components for nucleic acid polymerization and/or amplification. For example, the kit can further comprise a polymerase, such as a DNA polymerase, e.g., a polymerase having strand displacing activity.

In some embodiments of any of the aspects, the kit further comprises one or more reagents for nucleic acid polymerization and/or amplification, such as deoxyribonucleotide triphosphates (dNTPs), salt and/or buffers.

In some embodiments, the kit can further comprise a target, e.g., a reference target.

Also provided herein is a reaction mixture comprising at least one of a first reporter probe and a second reporter probe described herein. In some embodiments, the reaction mixture further comprises one or more reagents for nucleic acid polymerization and/or amplification, such as a polymerase (e.g., a polymerase having strand displacing activity), deoxyribonucleotide triphosphates (dNTPs), salt and/or buffers.

In another aspect provided herein is a system for detecting a target. The system comprises at least one of a first reporter probe and a second reporter probe described herein. In some embodiments, the system further comprises one or more reagents for nucleic acid polymerization and/or amplification, such as a polymerase (e.g., a polymerase having strand displacing activity), deoxyribonucleotide triphosphates (dNTPs), salt and/or buffers.

Some embodiments of the various aspects described herein are represented by the following numbered embodiments:
Embodiment 1) A method of detecting a target molecule in a sample, the method comprising:
   a. contacting the sample with a first reporter probe and a blocking probe, wherein:
      i. the first reporter probe comprises a target binding ligand linked to a first nucleic acid;
      ii. the blocking probe comprises a ligand capable of directly or indirectly forming a complex with the target binding ligand of the first probe and linked to a second nucleic acid,
         wherein a portion of the first nucleic acid and a portion of the second nucleic acid capable of hybridizing in absence of the target molecule,
         wherein binding of the target molecule to the first reporter probe inhibits the formation of the complex, and
         wherein a portion of the first nucleic acid capable of being extended at least two times to produce a nucleic acid record in the presence of the target molecule and no more than one time in absence of the target molecule;
   b. producing a nucleic acid record of an interaction between the first reporter probe and the target molecule; and
   c. detecting the nucleic acid record.
Embodiment 2) The method of Embodiment 1, comprising:
   a. contacting the sample with a first reporter probe, a second reporter probe and a blocking probe, wherein:
      i. the first reporter probe comprises a first target biding ligand and linked to a double stranded nucleic acid molecule, wherein the double-stranded nucleic acid comprises:
         1. a first nucleic acid strand linked to the first target binding molecule and comprising a single-stranded toe hold domain (a) at a first end of the double stranded nucleic acid; and
         2. a second nucleic acid strand substantially complementary to the first strand, forming a double-stranded region (duplex region) with the first strand and comprising toe hold domains (x and b), at a second end of the double-stranded nucleic acid, and
            wherein the double stranded nucleic acid comprises a first stopper molecule in the first strand and a second stopper molecule in the second strand in the duplex region;
      ii. the second reporter probe comprises a second target binding ligand capable of binding the target molecule and linked to a nucleic acid comprising a toe-hold domain (a*) that is substantially complementary to the toe-hold domain (a) of the double-stranded nucleic acid of the first reporter probe, and wherein the target molecule can simultaneously bind with the first reporter probe and the second reporter probe;
      iii. the blocking probe comprises a blocking ligand capable of directly or indirectly forming a complex with the first and/or second targeting ligand and linked to a nucleic acid strand, wherein the nucleic acid strand comprises, a first toe-hold domain (a) that is substantially identical to the toe hold domain (a) of the nucleic acid of the first reporter probe and a second toe-hold domain (x*) that is substantially complementary to the toe-hold domain (x) of the nucleic acid of the first reporter probe, and wherein binding of the target molecule to the first reporter probe inhibits the first reporter probe and the blocking probe from forming a complex and/or binding of the target molecule to the second reporter probe inhibits the second reporter probe and the blocking probe from forming a complex;
   b. producing a nucleic acid record of an interaction between the nucleic acid of the first probe and the nucleic acid of the second probe; and
   c. detecting the nucleic acid record.
Embodiment 3) The method of Embodiment 1, comprising:
   a. contacting the sample with a first reporter probe, a second reporter probe and a blocking probe, wherein:
      i. the first reporter probe comprises a first target binding ligand capable of binding the target molecule and linked to a double stranded nucleic acid molecule, wherein the double-stranded nucleic acid comprises:
         1. a first nucleic acid strand linked to the first target binding ligand and comprising single-stranded toe hold domain (a) at a first end of the double stranded nucleic acid; and
         2. a second nucleic acid strand substantially complementary to the first strand, forming a double-stranded region (duplex region) with the first strand and comprising toe hold domains (x and b), at a second end of the double-stranded nucleic acid, and
            wherein the double stranded nucleic acid comprises a first stopper molecule in the first strand and a second stopper molecule in the second strand in the duplex region;
      ii. the second reporter probe comprises a second target binding ligand of binding the target molecule and linked to a first nucleic acid strand comprising a hybridization domain linked to a toe hold domain (x) that is substantially identical to the pairing domain (x) of the nucleic acid of the first reporter probe; a second strand hybridized to the first strand and comprising a hybridization domain, substantially complementary to the hybridization domain of the first strand, linked to a primer binding domain (d*) linked to a toehold domain (a*) that is substantially complementary to the toehold domain (a) of the nucleic acid of the first reporter probe;
      iii. the blocking probe comprises a blocking ligand capable of directly or indirectly forming a complex with the first and/or second target binding ligand and linked to a nucleic acid strand, wherein the nucleic acid strand comprises, a toe-hold domain (x*) that is substantially complementary to the toe-hold domain (x) of the nucleic acid of the first reporter probe, and wherein binding of the target molecule to the first reporter probe inhibits the first reporter probe and the blocking probe from forming a complex and/or binding of the target molecule to the second reporter probe inhibits the second reporter probe and the blocking probe from forming a complex
   b. producing a nucleic acid record of an interaction between the nucleic acid of the first probe and the nucleic acid of the second probe; and
   c. detecting the nucleic acid record.
Embodiment 4) The method of Embodiment 1, comprising:
   a. contacting the sample with a first reporter probe, a second reporter probe and a blocking probe, wherein:
      i. the first reporter probe comprises a first target binding ligand capable of binding the target molecule and linked to a nucleic acid strand comprising a hybridization domain linked to a toe hold domain (x), a double stranded nucleic acid hybridized to the first strand, wherein the double-stranded nucleic acid comprises:
         1. a first nucleic acid strand hybridized to the nucleic acid strand linked to the target binding ligand and comprising a single-stranded toe hold domain (a) at an end distal from the toe hold domain (x); and
         2. a second nucleic acid strand substantially complementary to the first strand, forming a double-stranded region (duplex region) with the first strand and comprising a toe hold domain (b), at a second end of the double-stranded nucleic acid, and wherein the double stranded nucleic acid comprises a first stopper molecule in the first strand and a second stopper molecule in the second strand in the duplex region;
      ii. the second reporter probe comprises a second target binding ligand capable of binding the target molecule and linked to a first nucleic acid strand comprising a hybridization domain linked to a toe hold domain (x) that is substantially identical to the pairing domain (x) of the nucleic acid of the first reporter probe; a second strand hybridized to the first strand and comprising a hybridization domain, substantially complementary to the hybridization domain of the first strand, linked to a primer binding domain (d*) linked to a toehold domain (a*) that is substantially complementary to the toehold domain (a) of the nucleic acid of the first reporter probe;
      iii. the blocking probe comprises a blocking ligand capable of directly or indirectly forming a complex with first and/or second targeting ligand and linked to a nucleic acid strand, wherein the nucleic acid strand comprises, a toe-hold domain (x*) that is substantially complementary to the toe-hold domain (x) of the nucleic acid of the first reporter probe, and wherein binding of the target molecule to the first reporter probe inhibits the first reporter probe and the blocking probe from forming a complex and/or binding of the target molecule to the second reporter probe inhibits the second reporter probe and the blocking probe from forming a complex;
   b. producing a nucleic acid record of an interaction between the nucleic acid of the first probe and the nucleic acid of the second probe; and
   c. detecting the nucleic acid record.
Embodiment 5) A method of detecting a target molecule in a sample, the method comprising:
   a. contacting the sample with a first reporter probe and a blocking probe, wherein:
      i. the first reporter probe comprises a target binding ligand linked to a first nucleic acid;
      ii. the blocking probe comprises a ligand capable of directly or indirectly forming a complex with the target binding ligand of the first probe and linked to a second nucleic acid,
         wherein a portion of the first nucleic acid and a portion of the second nucleic acid capable of hybridizing in absence of the target molecule,
         wherein binding of the target molecule to the first reporter probe inhibits the formation of the complex, and
         wherein a portion of the first nucleic acid capable of being extended at least two times to produce a nucleic acid record in the absence of the target molecule and no more than one time in the presence of the target molecule;
   b. producing a nucleic acid record of an interaction between the first nucleic acid and the second nucleic acid; and
   c. detecting the nucleic acid record, and wherein absence of the nucleic acid record indicates the target molecule is present in the sample.
Embodiment 6) The method of Embodiment 1, comprising:
   a. contacting the sample with a first reporter probe and a blocking probe, wherein:
      i. the first reporter probe comprises a first target biding ligand and linked to a double stranded nucleic acid molecule, wherein the double-stranded nucleic acid comprises:
         1. a first nucleic acid strand linked to the first target binding molecule and comprising a single-stranded toe hold domain (a) at a first end of the double stranded nucleic acid; and
         2. a second nucleic acid strand substantially complementary to the first strand, forming a double-stranded region (duplex region) with the first strand and comprising a toe hold domain (b), at a second end of the double-stranded nucleic acid, and
            wherein the double stranded nucleic acid comprises a first stopper molecule in the first strand and a second stopper molecule in the second strand in the duplex region;
      ii. the blocking probe comprises a blocking ligand capable of directly or indirectly forming a complex with the first and/or second targeting ligand and linked to a nucleic acid strand, wherein the nucleic acid strand comprises, a first toe-hold domain (a) that is substantially identical to the toe hold domain (a) of the nucleic acid of the first reporter probe and a primer binding domain (d*), and wherein binding of the target molecule to the first reporter probe inhibits the first reporter probe and the blocking probe from forming a complex and/or binding of the target molecule to the second reporter probe inhibits the second reporter probe and the blocking probe from forming a complex;
   b. a nucleic acid record of an interaction between the first nucleic acid and the second nucleic acid; and
   c. detecting the nucleic acid record, and wherein absence of the nucleic acid record indicates the target molecule is present in the sample.
Embodiment 7) The method of any one of the preceding Embodiments, wherein the first reporter probe and the blocking probe form a complex prior to contacting with the sample and/or the second reporter probe and the blocking probe form a complex prior to contacting with the sample.
Embodiment 8) The method of any one of the preceding Embodiments, wherein the first reporter probe and the blocking probe are not in a complex prior to contacting with the sample and/or the second reporter probe and the blocking probe are not in a complex prior to contacting with the sample.
Embodiment 9) The method of any one of the preceding Embodiments, wherein the step of producing the record nucleic acid comprises extension by a polymerase.
Embodiment 10) The method of Embodiment 7, wherein said polymerase is a stand displacing polymerase.
Embodiment 11) The method of any one of the preceding Embodiments, wherein the method further comprises amplifying the nucleic acid record prior to detecting the nucleic acid record.
Embodiment 12) The method of any one of the preceding Embodiments, wherein the first targeting ligand and the blocking ligand are a binding pair.
Embodiment 13) The method of any one of the preceding Embodiments, wherein the second targeting ligand and the blocking ligand are a binding pair.
Embodiment 14) The method of any one of the preceding Embodiments, wherein the target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 15) The method of any one of the preceding Embodiments, wherein the first target binding ligand and the second target binding are the same.
Embodiment 16) The method of any one of Embodiments 1-14, wherein the first target binding ligand and the second target binding are different.
Embodiment 17) The method of any one of the preceding Embodiments, wherein the second target binding ligand is a class specific antibody.
Embodiment 18) The method of any one of the preceding Embodiments, wherein the target molecule is a biomolecule.
Embodiment 19) The method of any one of the preceding Embodiments, wherein the target molecule is an antibody, a nucleic acid, a small molecule, or an antigen.
Embodiment 20) The method of any one of the preceding Embodiments, wherein the target molecule is a neutralizing antibody.
Embodiment 21) The method of any one of Embodiments 1-20, wherein the target molecule is a nucleic acid.
Embodiment 22) The method of any one of Embodiments 1-20, wherein the target molecule is a small molecule.
Embodiment 23) The method of any one of Embodiments 1-20, wherein the target molecule is an antigen.
Embodiment 24) The method of Embodiments 19 or 23, wherein the antigen is a spike protein.
Embodiment 25) The method of Embodiments 19 or 23, wherein the antigen is encoded by an RNA vaccine.
Embodiment 26) The method of any one of the preceding Embodiments, wherein the sample is a biological sample.
   *Composition Embodiments*
Embodiment 27) A composition comprising:
   a. a first reporter probe comprising a target binding ligand linked to a first nucleic acid; and
   b. a blocking probe comprising a blocking ligand capable of directly or indirectly forming a complex with the target binding ligand of the first probe and linked to a second nucleic acid, and
      wherein a portion of the nucleic acid of the first reporter probe and a portion of the nucleic acid of the blocking probe capable of hybridizing in absence of the target molecule,
      wherein binding of the target molecule to the first reporter probe inhibits the formation of the complex, and
      wherein a portion of the nucleic acid of the first reporter probe capable of being extended at least two times to produce a nucleic acid record in the presence of the target molecule and no more than one time in absence of the target molecule.
Embodiment 28) A composition comprising:
   a. a first reporter probe comprising a target binding ligand linked to a first nucleic acid;
   b. a second reporter probe comprising a target binding ligand linked to a second nucleic acid, wherein a portion of the nucleic acid of the first reporter probe and a portion of the nucleic acid of the blocking probe capable of hybridizing;
   c. a blocking probe comprising a blocking ligand capable of directly or indirectly forming a complex with the target binding ligand of the first probe, and linked to a third nucleic acid, wherein a portion of the nucleic acid of the first reporter probe and a portion of the nucleic acid of the blocking probe capable of hybridizing in absence of the target molecule,
      wherein a portion of the nucleic acid of the second reporter probe and a portion of the nucleic acid of the blocking probe capable of hybridizing in absence of the target molecule, wherein binding of the target molecule to the first reporter probe inhibits the formation of the complex, and
      wherein a portion of the nucleic acid of the first reporter probe capable of being extended at least two times to produce a nucleic acid record in the presence of a target molecule and no more than one time in absence of the target molecule.
Embodiment 29) The composition of Embodiment 24 or 25, wherein the first reporter comprises double-stranded nucleic acid linked to the target binding ligand, wherein the double-stranded nucleic acid comprises:
   a. a first nucleic acid strand linked to the target binding ligand and comprising a single-stranded toe hold domain (a) at a first end of the double stranded nucleic acid; and
   b. a second nucleic acid strand substantially complementary to the first strand, forming a double-stranded region (duplex region) with the first strand and comprising toe hold domains (x and b), at a second end of the double-stranded nucleic acid, and
      wherein the double stranded nucleic acid comprises a first stopper molecule in the first strand and a second stopper molecule in the second strand in the duplex region.
Embodiment 30) The composition of any one of the preceding Embodiments, wherein the first reporter comprises a nucleic acid strand linked to the target binding ligand and comprising a hybridization domain linked to a toe hold domain (x), a double stranded nucleic acid hybridized to the linked strand, wherein the double-stranded nucleic acid comprises:
   a. a first nucleic acid strand hybridized to the nucleic acid strand linked to the target binding ligand and comprising a hybridization domain linked a single-stranded toe hold domain (a) at an end distal from the hybridization domain; and
   b. a second nucleic acid strand substantially complementary to the first strand, forming a double-stranded region (duplex region) with the first strand and comprising a toe hold domain (b), at a second end of the double-stranded nucleic acid, and wherein the double stranded nucleic acid comprises a first stopper molecule in the first strand and a second stopper molecule in the second strand in the duplex region.
Embodiment 31) The composition of any one of the preceding Embodiments, wherein the first strand comprises a primer binding domain (c*) linked to a first subdomain that is linked to a stopper molecule (•) that is linked to a second subdomain (b) that is linked to the toehold domain (a).
Embodiment 32) The composition of any one of the preceding Embodiments, wherein the first strand comprises a hybridization domain linked to a primer binding domain (c*) that is linked to a first subdomain that is linked to a stopper molecule (•) that is linked to a second subdomain (b) that is linked to the toehold domain (a), wherein the hybridization domain is substantially complementary to the hybridization domain of the strand linked to the target binding ligand.
Embodiment 33) The composition of any one of the preceding Embodiments, wherein the second strand comprises a first subdomain (b*) linked to a second subdomain that is linked to a stopper molecule (•) that is linked to a primer domain (c) that is linked to the toe-hold domain (b).
Embodiment 34) The composition of any one of the preceding Embodiments, wherein the second strand comprises a first subdomain (b*) linked to a second subdomain that is linked to a stopper molecule (•) that is linked to a primer domain (c) that is linked to the toe-hold domain (b) that is linked to the toehold domain (x).
Embodiment 35) The composition of any one of the preceding Embodiments, wherein the second reporter probe comprises a nucleic acid strand linked to the target binding ligand and comprises a toe-hold domain (a*) that is substantially complementary to the toe-hold domain (a) of the first reporter probe.
Embodiment 36) The composition of any one of the preceding Embodiments, wherein the second reporter probe comprises a nucleic acid strand linked to the target binding ligand and comprises a primer binding domain (d*) linked to a toe-hold domain (a*), wherein the toe-hold domain (a*) is substantially complementary to the toe-hold domain (a) of the first reporter probe.
Embodiment 37) The composition of any one of the preceding Embodiments, wherein the second reporter probe comprises a first nucleic acid strand linked to the target binding ligand and comprising hybridizing domain linked to a toehold domain (x), wherein the toehold domain (x) is at an end distal to the end linked to the target binding ligand; and a second nucleic acid strand comprising a hybridizing domain linked to a toehold domain (a*), wherein the hybridization domain of the first and second strand are substantially complementary to each other.
Embodiment 38) The composition of any one of the preceding Embodiments, wherein the second reporter probe comprises a first nucleic acid strand linked to the target binding ligand and comprising hybridizing domain linked to a toehold domain (x), wherein the toehold domain (x) is at an end distal to the end linked to the target binding ligand; and a second nucleic acid strand comprising a hybridizing domain linked to primer binding domain (d*) that is linked to a toehold domain (a*).
Embodiment 39) The composition of any one of the preceding Embodiments, wherein the blocking probe comprises a nucleic acid strand comprising a toe-hold domain (x*) at an end distal to the end linked to the blocking ligand, wherein the toe-hold domain (x*) is substantially complimentary to the toe-hold domain (x) of the first reporter probe.
Embodiment 40) The composition of any one of the preceding Embodiments, wherein the blocking probe comprises a nucleic acid strand comprising a first toe-hold domain (a*) that is linked to a second toe-hold domain (x*), wherein the toe-hold domain (x*) is at an end distal to the end linked to the blocking ligand, wherein the toe-hold domain (x*) is substantially complimentary to the toe-hold domain (x) of the first and/or second reporter probe and the toe-hold domain (a*) is substantially identical to the toe-hold domain (a) of the first probe.
Embodiment 41) A composition comprising:
   a. a first reporter probe comprising a target binding ligand linked to a first nucleic acid; and
   b. a blocking probe comprising a blocking ligand capable of directly or indirectly forming a complex with the target binding ligand of the first probe and linked to a second nucleic acid, and
      wherein a portion of the nucleic acid of the first reporter probe and a portion of the nucleic acid of the blocking probe capable of hybridizing in absence of the target molecule,
      wherein binding of the target molecule to the first reporter probe inhibits the formation of the complex, and
      wherein a portion of the nucleic acid of the first reporter probe capable of being extended at least two times to produce a nucleic acid record in the absence of the target molecule and no more than one time in presence of the target molecule.
Embodiment 42) The composition of Embodiment 38, wherein the first reporter comprises a double-stranded nucleic acid linked to the target binding ligand, wherein the double-stranded nucleic acid comprises:
   a. a first nucleic acid strand linked to the target binding ligand and comprising a single-stranded toe hold domain (a) at a first end of the double stranded nucleic acid; and
   b. a second nucleic acid strand substantially complementary to the first strand, forming a double-stranded region (duplex region) with the first strand and comprising a toe hold domain (b), at a second end of the double-stranded nucleic acid, and
      wherein the double stranded nucleic acid comprises a first stopper molecule in the first strand and a second stopper molecule in the second strand in the duplex region.
Embodiment 43) The composition of Embodiment 41 or 42, wherein the first strand comprises a primer binding domain (c*) linked to a first subdomain that is linked to a stopper molecule (•) that is linked to a second subdomain (b) that is linked to the toehold domain (a).
Embodiment 44) The composition of any one of Embodiments 41-43, wherein the first strand comprises a hybridization domain linked to a primer binding domain (c*) that is linked to a first subdomain that is linked to a stopper molecule (•) that is linked to the second subdomain (b) that is linked to the toehold domain (a).
Embodiment 45) The composition of any one of Embodiments 41-44, wherein the second strand comprises a first subdomain (b*) linked to a second subdomain that is linked to a stopper molecule (•) that is linked to a primer domain (c) that is linked to the toehold domain (b), wherein the toe-hold domain is substantially complementary to the first subdomain (b*).
Embodiment 46) The composition of any one of Embodiments 41-45, wherein the blocking probe comprises a nucleic acid strand comprising a toe-hold domain (a*) at an end distal to the end linked to the blocking ligand, wherein the toe-hold domain (a*) is substantially complimentary to the toe-hold domain (a) of the first reporter probe.
Embodiment 47) The composition of any one of Embodiments 41-46, wherein the blocking probe comprises a nucleic acid strand comprising a primer binding domain (x*) that is linked to a toe-hold domain (a*), wherein the toe-hold domain (a*) is at an end distal to the end linked to the blocking ligand, wherein the toe-hold domain (a*) is substantially complimentary to the toe-hold domain (a) of the first reporter probe.
Embodiment 48) The composition of any one of the preceding Embodiments, wherein the first targeting ligand and the blocking ligand are a binding pair.
Embodiment 49) The composition of any one of the preceding Embodiments, wherein the second targeting ligand and the blocking ligand are a binding pair.
Embodiment 50) The composition of any one of the preceding Embodiments, wherein the target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 51) The composition of any one of the preceding Embodiments, wherein the first target binding ligand and the second target binding are the same.
Embodiment 52) The composition of any one of the preceding Embodiments, wherein the first target binding ligand and the second target binding are different.
Embodiment 53) The composition of any one of the preceding Embodiments, wherein the first target binding ligand is a receptor.
Embodiment 54) The composition of any one of the preceding Embodiments, wherein the first target binding ligand is a receptor and the blocking ligand is ligand of the receptor.
Embodiment 55) The composition of any one of the preceding Embodiments, wherein the second target binding ligand is a class specific antibody.
Embodiment 56) The composition of any one of the preceding Embodiments, wherein the toehold domain (x) of the first reporter probe and the toe hold domain (x*) of the blocking probe are hybridized to each other.
Embodiment 57) The composition of any one of the preceding Embodiments, wherein the toe-hold domain (a*) of the second reporter probe and the toe-hold domain (a) of the blocking probe are hybridized to each other.
Embodiment 58) The composition of any one of the preceding Embodiments, wherein the toehold domain (x) of the second reporter probe and the toe hold domain (x*) of the blocking probe are hybridized to each other.
Embodiment 59) The composition of any one of the preceding Embodiments, wherein the toehold domain (x) of the first reporter probe and the toe hold domain (x*) of a first blocking probe are hybridized to each other and the toehold domain (x) of the second reporter probe and the toe hold domain (x*) of a second blocking probe are hybridized to each other.
Embodiment 60) The composition of any one of the preceding Embodiments, wherein the composition further comprises one or more components for nucleic acid amplification.
Embodiment 61) The composition of any one of the preceding Embodiments, wherein the composition further comprises a polymerase.
Embodiment 62) The composition of Embodiment 61, wherein said polymerase is a stand displacing polymerase.
Embodiment 63) The composition of any one of the preceding Embodiments, wherein the composition further comprises dNTPs.
Embodiment 64) The composition of any one of the preceding Embodiments, wherein the composition further comprises a salt.
Embodiment 65) The composition of any one of the preceding Embodiments, wherein the composition further comprises a buffer.
Embodiment 66) The composition of any one of the preceding Embodiments, wherein the composition further comprises the target molecule.
   *Kit Embodiments*
Embodiment 67) A kit comprising:
   a. a first reporter probe comprising a target binding ligand linked to a first nucleic acid; and
   b. a blocking probe comprising a blocking ligand capable of directly or indirectly forming a complex with the target binding ligand of the first probe and linked to a second nucleic acid, and
      wherein a portion of the nucleic acid of the first reporter probe and a portion of the nucleic acid of the blocking probe capable of hybridizing in absence of the target molecule,
      wherein binding of the target molecule to the first reporter probe inhibits the formation of the complex, and
      wherein a portion of the nucleic acid of the first reporter probe capable of being extended at least two times to produce a nucleic acid record in the presence of the target molecule and no more than one time in absence of the target molecule.
Embodiment 68) A kit comprising:
   a. a first reporter probe comprising a target binding ligand linked to a first nucleic acid;
   b. a second reporter probe comprising a target binding ligand linked to a second nucleic acid, wherein a portion of the nucleic acid of the first reporter probe and a portion of the nucleic acid of the blocking probe capable of hybridizing;
   c. a blocking probe comprising a blocking ligand capable of directly or indirectly forming a complex with the target binding ligand of the first probe, and linked to a third nucleic acid, wherein a portion of the nucleic acid of the first reporter probe and a portion of the nucleic acid of the blocking probe capable of hybridizing in absence of the target molecule,
      wherein a portion of the nucleic acid of the second reporter probe and a portion of the nucleic acid of the blocking probe capable of hybridizing in absence of the target molecule, wherein binding of the target molecule to the first reporter probe inhibits the formation of the complex, and
      wherein a portion of the nucleic acid of the first reporter probe capable of being extended at least two times to produce a nucleic acid record in the presence of a target molecule and no more than one time in absence of the target molecule.
Embodiment 69) The kit of Embodiment 63 or 64, wherein the first reporter comprises double-stranded nucleic acid linked to the target binding ligand, wherein the double-stranded nucleic acid comprises:
   a. a first nucleic acid strand linked to the target binding ligand and comprising a single-stranded toe hold domain (a) at a first end of the double stranded nucleic acid; and
   b. a second nucleic acid strand substantially complementary to the first strand, forming a double-stranded region (duplex region) with the first strand and comprising toe hold domains (x and b), at a second end of the double-stranded nucleic acid, and
      wherein the double stranded nucleic acid comprises a first stopper molecule in the first strand and a second stopper molecule in the second strand in the duplex region.
Embodiment 70) The kit of any one of the preceding Embodiments, wherein the first reporter comprises a nucleic acid strand linked to the target binding ligand and comprising a hybridization domain linked to a toe hold domain (x), a double stranded nucleic acid hybridized to the linked strand, wherein the double-stranded nucleic acid comprises:
   a. a first nucleic acid strand hybridized to the nucleic acid strand linked to the target binding ligand and comprising a hybridization domain linked a single-stranded toe hold domain (a) at an end distal from the hybridization domain; and
   b. a second nucleic acid strand substantially complementary to the first strand, forming a double-stranded region (duplex region) with the first strand and comprising a toe hold domain (b), at a second end of the double-stranded nucleic acid, and wherein the double stranded nucleic acid comprises a first stopper molecule in the first strand and a second stopper molecule in the second strand in the duplex region.
Embodiment 71) The kit of any one of the preceding Embodiments, wherein the first strand comprises a primer binding domain (c*) linked to a first subdomain that is linked to a stopper molecule (•) that is linked to a second subdomain (b) that is linked to the toehold domain (a).
Embodiment 72) The kit of any one of the preceding Embodiments, wherein the first strand comprises a hybridization domain linked to a primer binding domain (c*) that is linked to a first subdomain that is linked to a stopper molecule (•) that is linked to a second subdomain (b) that is linked to the toehold domain (a), wherein the hybridization domain is substantially complementary to the hybridization domain of the strand linked to the target binding ligand.
Embodiment 73) The kit of any one of the preceding Embodiments, wherein the second strand comprises a first subdomain (b*) linked to a second subdomain that is linked to a stopper molecule (•) that is linked to a primer domain (c) that is linked to the toe-hold domain (b).
Embodiment 74) The kit of any one of the preceding Embodiments, wherein the second strand comprises a first subdomain (b*) linked to a second subdomain that is linked to a stopper molecule (•) that is linked to a primer domain (c) that is linked to the toe-hold domain (b) that is linked to the toehold domain (x).
Embodiment 75) The kit of any one of the preceding Embodiments, wherein the second reporter probe comprises a nucleic acid strand linked to the target binding ligand and comprises a toe-hold domain (a*) that is substantially complementary to the toe-hold domain (a) of the first reporter probe.
Embodiment 76) The kit of any one of the preceding Embodiments, wherein the second reporter probe comprises a nucleic acid strand linked to the target binding ligand and comprises a primer binding domain (d*) linked to a toe-hold domain (a*), wherein the toe-hold domain (a*) is substantially complementary to the toe-hold domain (a) of the first reporter probe.
Embodiment 77) The kit of any one of the preceding Embodiments, wherein the second reporter probe comprises a first nucleic acid strand linked to the target binding ligand and comprising hybridizing domain linked to a toehold domain (x), wherein the toehold domain (x) is at an end distal to the end linked to the target binding ligand; and a second nucleic acid strand comprising a hybridizing domain linked to a toehold domain (a*), wherein the hybridization domain of the first and second strand are substantially complementary to each other.
Embodiment 78) The kit of any one of the preceding Embodiments, wherein the second reporter probe comprises a first nucleic acid strand linked to the target binding ligand and comprising hybridizing domain linked to a toehold domain (x), wherein the toehold domain (x) is at an end distal to the end linked to the target binding ligand; and a second nucleic acid strand comprising a hybridizing domain linked to primer binding domain (d*) that is linked to a toehold domain (a*).
Embodiment 79) The kit of any one of the preceding Embodiments, wherein the blocking probe comprises a nucleic acid strand comprising a toe-hold domain (x*) at an end distal to the end linked to the blocking ligand, wherein the toe-hold domain (x*) is substantially complimentary to the toe-hold domain (x) of the first reporter probe.
Embodiment 80) The kit of any one of the preceding Embodiments, wherein the blocking probe comprises a nucleic acid strand comprising a first toe-hold domain (a*) that is linked to a second toe-hold domain (x*), wherein the toe-hold domain (x*) is at an end distal to the end linked to the blocking ligand, wherein the toe-hold domain (x*) is substantially complimentary to the toe-hold domain (x) of the first and/or second reporter probe and the toe-hold domain (a*) is substantially identical to the toe-hold domain (a) of the first probe.
Embodiment 81) A kit comprising:
   a. a first reporter probe comprising a target binding ligand linked to a first nucleic acid; and
   b. a blocking probe comprising a blocking ligand capable of directly or indirectly forming a complex with the target binding ligand of the first probe and linked to a second nucleic acid, and
      wherein a portion of the nucleic acid of the first reporter probe and a portion of the nucleic acid of the blocking probe capable of hybridizing in absence of the target molecule,
      wherein binding of the target molecule to the first reporter probe inhibits the formation of the complex, and
      wherein a portion of the nucleic acid of the first reporter probe capable of being extended at least two times to produce a nucleic acid record in the absence of the target molecule and no more than one time in presence of the target molecule.
Embodiment 82) The kit of Embodiment 81, wherein the first reporter comprises a double-stranded nucleic acid linked to the target binding ligand, wherein the double-stranded nucleic acid comprises:
   a. a first nucleic acid strand linked to the target binding ligand and comprising a single-stranded toe hold domain (a) at a first end of the double stranded nucleic acid; and
   b. a second nucleic acid strand substantially complementary to the first strand, forming a double-stranded region (duplex region) with the first strand and comprising a toe hold domain (b), at a second end of the double-stranded nucleic acid, and
      wherein the double stranded nucleic acid comprises a first stopper molecule in the first strand and a second stopper molecule in the second strand in the duplex region.
Embodiment 83) The kit of Embodiment 81 or 82, wherein the first strand comprises a primer binding domain (c*) linked to a first subdomain that is linked to a stopper molecule (•) that is linked to a second subdomain (b) that is linked to the toehold domain (a).
Embodiment 84) The kit of any one of Embodiments 81-83, wherein the first strand comprises a hybridization domain linked to a primer binding domain (c*) that is linked to a first subdomain that is linked to a stopper molecule (•) that is linked to the second subdomain (b) that is linked to the toehold domain (a).
Embodiment 85) The kit of any one of Embodiments 81-84, wherein the second strand comprises a first subdomain (b*) linked to a second subdomain that is linked to a stopper molecule (•) that is linked to a primer domain (c) that is linked to the toehold domain (b), wherein the toe-hold domain is substantially complementary to the first subdomain (b*).
Embodiment 86) The kit of any one of Embodiments 81-85, wherein the blocking probe comprises a nucleic acid strand comprising a toe-hold domain (a*) at an end distal to the end linked to the blocking ligand, wherein the toe-hold domain (a*) is substantially complimentary to the toe-hold domain (a) of the first reporter probe.
Embodiment 87) The kit of any one of Embodiments 81-86, wherein the blocking probe comprises a nucleic acid strand comprising a primer binding domain (x*) that is linked to a toe-hold domain (a*), wherein the toe-hold domain (a*) is at an end distal to the end linked to the blocking ligand, wherein the toe-hold domain (a*) is substantially complimentary to the toe-hold domain (a) of the first reporter probe.
Embodiment 88) The kit of any one of the preceding Embodiments, wherein the first targeting ligand and the blocking ligand are a binding pair.
Embodiment 89) The kit of any one of the preceding Embodiments, wherein the second targeting ligand and the blocking ligand are a binding pair.
Embodiment 90) The kit of any one of the preceding Embodiments, wherein the target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 91) The kit of any one of the preceding Embodiments, wherein the first target binding ligand and the second target binding are the same.
Embodiment 92) The kit of any one the preceding Embodiment, wherein the first target binding ligand and the second target binding are different.
Embodiment 93) The kit of any one of the preceding Embodiments, wherein the first target binding ligand is a receptor.
Embodiment 94) The kit of any one of the preceding Embodiments, wherein the first target binding ligand is a receptor and the blocking ligand is ligand of the receptor.
Embodiment 95) The kit of any one of the preceding Embodiments, wherein the second target binding ligand is a class specific antibody.
Embodiment 96) The kit of any one of the preceding Embodiments, wherein the toehold domain (x) of the first reporter probe and the toe hold domain (x*) of the blocking probe are hybridized to each other.
Embodiment 97) The kit of any one of the preceding Embodiments, wherein the toe-hold domain (a*) of the second reporter probe and the toe-hold domain (a) of the blocking probe are hybridized to each other.
Embodiment 98) The kit of any one of the preceding Embodiments, wherein the toehold domain (x) of the second reporter probe and the toe hold domain (x*) of the blocking probe are hybridized to each other.
Embodiment 99) The kit of any one of the preceding Embodiments, wherein the toehold domain (x) of the first reporter probe and the toe hold domain (x*) of a first blocking probe are hybridized to each other and the toehold domain (x) of the second reporter probe and the toe hold domain (x*) of a second blocking probe are hybridized to each other.
Embodiment 100) The kit of any one of the preceding Embodiments, wherein the kit further comprises one or more components for nucleic acid amplification.
Embodiment 101) The kit of any one of the preceding Embodiments, wherein the kit further comprises a polymerase.
Embodiment 102) The kit of Embodiment 101, wherein said polymerase is a stand displacing polymerase.
Embodiment 103) The kit of any one of the preceding Embodiments, wherein the kit further comprises dNTPs.
Embodiment 104) The kit of any one of the preceding Embodiments, wherein the kit further comprises a salt.
Embodiment 105) The kit of any one of the preceding Embodiments, wherein the kit further comprises a buffer.
Embodiment 106) The kit of any one of the preceding Embodiments, wherein the kit further comprises the target molecule.

Some embodiments of the various aspects described herein are represented by the following numbered embodiments:
Embodiment 1: A method of detecting a target in a sample, the method comprising:
   a. contacting the sample with a first reporter probe and a blocking probe, wherein:
      i. the first reporter probe comprises a first target binding ligand linked to a first nucleic acid;
      ii. the blocking probe comprises a blocking ligand capable of directly or indirectly forming a complex with the first target binding ligand and linked to a blocking probe nucleic acid,

      wherein a portion of the first nucleic acid and a portion of the blocking probe nucleic acid are capable of hybridizing in the absence of the target,
      wherein binding of the target to the first reporter probe inhibits the formation of the complex, and
      wherein a portion of the first nucleic acid is capable of being extended at least two times to produce a nucleic acid record in the presence of the target and no more than one time in the absence of the target;
   b. providing a polymerase, thereby producing the nucleic acid record of an interaction between the first reporter probe and the target; and
   c. detecting the nucleic acid record.
Embodiment 2: The method of Embodiment 1, wherein
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising a single-stranded toehold domain (a) at a first end of the double stranded nucleic acid; and
         2. a first nucleic acid second strand substantially complementary to the first strand, forming a double-stranded region (duplex region) with the first strand and comprising toehold domains (x and b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid comprising a toehold domain (a*) that is substantially complementary to the toehold domain (a) of the double-stranded nucleic acid of the first reporter probe, and wherein the target can simultaneously bind with the first reporter probe and the second reporter probe;
      iii. the blocking ligand is capable of directly or indirectly forming a complex with the first target binding ligand and/or the second target binding ligand and is linked to the blocking probe nucleic acid, wherein the blocking probe nucleic acid comprises a first toehold domain (a) that is substantially identical to the toehold domain (a) of the first reporter probe, and a second toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 3: The method of Embodiment 1, wherein:
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising single-stranded toehold domain (a) at a first end of the double stranded nucleic acid; and
         2. a first nucleic acid second strand substantially complementary to the first nucleic acid first strand, forming a double-stranded region (duplex region) with the first nucleic acid first strand and comprising toe hold domains (x and b) at a second end of the double-stranded nucleic acid, and
         wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the toehold domain (x) of the first reporter probe; a second nucleic acid second strand hybridized to the second nucleic acid first strand and comprising a second nucleic acid second hybridizing domain, substantially complementary to the second nucleic acid first hybridizing domain and linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or the second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 4: The method of Embodiment 1, wherein:
   a. the contacting the sample with the first reporter probe and the blocking probe comprises contacting the sample with the first reporter probe, a second reporter probe, and the blocking probe, wherein:
      i. the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a single-stranded toehold domain (a) at an end distal from the toehold domain (x); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the pairing domain (x) of the first reporter probe; a second nucleic acid second strand hybridized to the second nucleic acid first strand and comprising a second nucleic acid second hybridizing domain, substantially complementary to the second nucleic acid first hybridizing domain, linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 5: A method of detecting a target in a sample, the method comprising:
   a. contacting the sample with a first reporter probe, wherein:
      i. the first reporter probe comprises a first target binding ligand linked to a first nucleic acid;
   b. contacting the sample with a blocking probe, wherein:
      i. the blocking probe comprises a blocking ligand capable of directly or indirectly forming a complex with the first target binding ligand and linked to a blocking probe nucleic acid,

      wherein a portion of the first nucleic acid and a portion of the blocking probe nucleic acid are capable of hybridizing in the absence of the target,
      wherein binding of the target to the first reporter probe inhibits the formation of the complex, and
      wherein a portion of the first nucleic acid is capable of being extended at least two times to produce a nucleic acid record in the absence of the target and no more than one time in the presence of the target;
   c. providing a polymerase, thereby producing the nucleic acid record of an interaction between the first nucleic acid and the blocking probe nucleic acid if said interaction is present; and
   d. detecting the presence or absence of the nucleic acid record, and wherein the absence of the nucleic acid record indicates the target is present in the sample.
Embodiment 6: The method of Embodiment 5, wherein
   i. the first nucleic acid comprises a double stranded nucleic acid, wherein the double-stranded nucleic acid comprises:
      1. a first nucleic acid first strand linked to the first target binding ligand and comprising a single-stranded toehold domain (a) at a first end of the double stranded nucleic acid; and
      2. a first nucleic acid second strand substantially complementary to the first strand, forming a double-stranded region (duplex region) with the first nucleic acid first strand and comprising a toehold domain (b) at a second end of the double-stranded nucleic acid, and
      wherein the double-stranded nucleic acid comprises a first stopper molecule in the first nucleic acid first strand and a second stopper molecule in the first nucleic acid second strand in the duplex region;
   ii. the blocking probe nucleic acid comprises: a toeholdfirst toehold domain (a) that is substantially identical to the toehold domain (a) of the first reporter probe; and a primer binding domain (d*).
Embodiment 7: The method of any one of the preceding Embodiments, wherein the first reporter probe and the blocking probe form a complex prior to contacting with the sample and/or the second reporter probe and the blocking probe form a complex prior to contacting with the sample.
Embodiment 8: The method of any one of the preceding Embodiments, wherein the first reporter probe and the blocking probe are not in a complex prior to contacting with the sample and/or the second reporter probe and the blocking probe are not in a complex prior to contacting with the sample.
Embodiment 9: A method of detecting a target in a sample, the method comprising:
   a. contacting the sample with a first reporter probe and a second reporter probe, wherein:
      i. the first reporter probe comprises a first target binding ligand capable of binding the target and linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
      wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
   c. providing a polymerase, thereby producing a nucleic acid record of an interaction between the first nucleic acid and the second nucleic acid if said interaction is present; and
   d. detecting the presence or absence of the nucleic acid record, and wherein the presence of the nucleic acid record indicates the target is present in the sample.
Embodiment 10: A method of detecting a target in a sample, the method comprising:
   a. contacting the sample with a first reporter probe and a second reporter probe, wherein:
      i. the first reporter probe comprises first target binding ligand capable of binding the target and linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
         wherein the toehold domain (b) of the first reporter probe comprises a nucleotide that is not present in the hybridizing domain (Lk1) of the first reporter probe and the hybridizing domain (Lk2) of the second probe, and
         wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and wherein binding of the target to the first reporter probe inhibits the first reporter probe and the first blocking probe from forming a complex; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex;
   c. providing a polymerase and a first dNTP mix, where the first dNTP mix is substantially free of a dNTP complementary to the nucleotide present in the toehold domain (b) of the first reporter probe but not in the hybridizing domain (Lk1) of the first reporter probe and the hybridizing domain (Lk2) of the second reporter probe;
   d. providing a second dNTP mix and optionally a polymerase, wherein the second dNTP mix comprises a dTNP complementary to the nucleotide present in the toehold domain (a) or (b) of the first reporter probe but not in the hybridizing domain (Lk1) of the first reporter probe and the hybridizing domain (Lk2) of the second reporter probe, thereby producing a nucleic acid record of an interaction between the first nucleic acid and the second nucleic acid if said interaction is present; and
   e. detecting the presence or absence of the nucleic acid record, and wherein the presence of the nucleic acid record indicates the target is present in the sample.
Embodiment 11: The method of any one of the preceding Embodiments, wherein the providing the polymerase extends one or more of the nucleic acids.
Embodiment 12: The method of Embodiment 11, wherein said polymerase is a stand displacing polymerase.
Embodiment 13: The method of any one of the preceding Embodiments, wherein the method further comprises amplifying the nucleic acid record, if present, prior to detecting the presence or the absence of nucleic acid record.
Embodiment 14: The method of any one of the preceding Embodiments, wherein the first target binding ligand and the blocking ligand are a binding pair.
Embodiment 15: The method of any one of the preceding Embodiments, wherein the second target binding ligand and the blocking ligand are a binding pair.
Embodiment 16: The method of any one of the preceding Embodiments, wherein the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 17: The method of any one of the preceding Embodiments, wherein the first target binding ligand and the second target binding ligand are the same.
Embodiment 18: The method of any one of the preceding Embodiments, wherein the first target binding ligand and the second target binding ligand are different.
Embodiment 19: The method of any one of the preceding Embodiments, wherein the second target binding ligand is a class specific antibody.
Embodiment 20: The method of any one of the preceding Embodiments, wherein the target is a biomolecule.
Embodiment 21: The method of any one of the preceding Embodiments, wherein the target is an antibody, a nucleic acid, a small molecule, or an antigen.
Embodiment 22: The method of any one of the preceding Embodiments, wherein the target is a neutralizing antibody.
Embodiment 23: The method of any one of the preceding Embodiments, wherein the target is a nucleic acid.
Embodiment 24: The method of any one of the preceding Embodiment, wherein the target is a small molecule.
Embodiment 25: The method of any one of the preceding Embodiments, wherein the target is an antigen.
Embodiment 26: The method of Embodiment 21 or 25, wherein the antigen is a spike protein.
Embodiment 27: The method of Embodiment 21 or 25, wherein the antigen is comprised in a vaccine, or wherein the antigen is encoded by a vaccine.
Embodiment 28: The method of Embodiment 27, wherein the antigen is encoded by a DNA vaccine.
Embodiment 29: The method of Embodiment 27, wherein the antigen is encoded by an RNA vaccine.
Embodiment 30: The method of any one of the preceding Embodiments, wherein the sample is a biological sample.
Embodiment 31: A composition for detecting a target in a sample, the composition comprising:
   a. a first reporter probe comprising a target binding ligand linked to a first nucleic acid; and
   b. a blocking probe comprising a blocking ligand capable of directly or indirectly forming a complex with the target binding ligand of the first reporter probe and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 32: The composition of Embodiment 31, further comprising:
   a second reporter probe comprising a second target binding ligand linked to a second nucleic acid,
   wherein a portion of the first nucleic acid and a portion of the second nucleic acid are capable of hybridizing, and
   wherein a portion of the second nucleic acid and a portion of the blocking probe nucleic acid are capable of hybridizing in the absence of the target.
Embodiment 33: The composition of Embodiment 31 or 32, wherein the first reporter probe comprises a double-stranded nucleic acid linked to the first target binding ligand, wherein the double-stranded nucleic acid comprises:
   a. a first nucleic acid first strand linked to the first target binding ligand and comprising a single-stranded toehold domain (a) at a first end of the double stranded nucleic acid; and
   b. a first nucleic acid second strand substantially complementary to the first nucleic acid first strand, forming a double-stranded region (duplex region) with the first nucleic acid first strand and comprising toehold domains (x and b) at a second end of the double-stranded nucleic acid, and
   wherein the double stranded nucleic acid comprises a first stopper molecule in the first nucleic acid first strand and a second stopper molecule in the first nucleic acid second strand in the duplex region.
Embodiment 34: The composition of any one of the preceding Embodiments, wherein the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
   a. a first nucleic acid first strand hybridized to the linking strand linked to the first target binding ligand and comprising a first nucleic acid second hybridizing domain linked to a single-stranded toehold domain (a) at an end distal from the first nucleic acid second hybridizing domain; and
   b. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region.
Embodiment 35: The composition of any one of the preceding Embodiments, wherein the first nucleic acid first strand comprises a primer binding domain (c*) linked to a first subdomain that is linked to a stopper molecule (•) that is linked to a second subdomain (b) that is linked to the toehold domain (a).
Embodiment 36: The composition of any one of the preceding Embodiments, wherein the first nucleic acid first strand comprises the first nucleic acid second hybridizing domain linked to a primer binding domain (c*) that is linked to a first subdomain that is linked to a stopper molecule (•) that is linked to a second subdomain (b) that is linked to the toehold domain (a), wherein the first nucleic acid second hybridizing domain is substantially complementary to the first nucleic acid first hybridizing domain of the linking strand linked to the first target binding ligand.
Embodiment 37: The composition of any one of the preceding Embodiments, wherein the second strand comprises a first subdomain (b*) linked to a second subdomain that is linked to a stopper molecule (•) that is linked to a primer domain (c) that is linked to the toehold domain (b). Embodiment 38: The composition of any one of the preceding Embodiments, wherein the first nucleic acid second strand comprises a first subdomain (b*) linked to a second subdomain that is linked to a stopper molecule (•) that is linked to a primer domain (c) that is linked to the toehold domain (b) that is linked to the toehold domain (x).
Embodiment 39: The composition of any one of the preceding Embodiments, wherein the second reporter probe comprises a second nucleic acid linked to the second target binding ligand and comprises a toehold domain (a*) that is substantially complementary to the toehold domain (a) of the first reporter probe.
Embodiment 40: The composition of any one of the preceding Embodiments, wherein the second reporter probe comprises a second nucleic acid linked to the second target binding ligand and comprises a toeholdprimer binding domain (d*) linked to a toehold domain (a*), wherein the toehold domain (a*) is substantially complementary to the toehold domain (a) of the first reporter probe.
Embodiment 41: The composition of any one of the preceding Embodiments, wherein the second reporter probe comprises a second nucleic acid first strand linked to the toeholdsecond target binding ligand and comprising a second nucleic acid first hybridizing domain linked to a toehold domain (x), wherein the toehold domain (x) is at an end distal to the end linked to the second target binding ligand; and a second nucleic acid second strand comprising a second nucleic acid second hybridizing domain linked to a toehold domain (a*), wherein the second nucleic acid first hybridizing domain and the second nucleic acid second hybridizing domain are substantially complementary to each other.
Embodiment 42: The composition of any one of the preceding Embodiments, wherein the second reporter probe comprises a second nucleic acid first strand linked to the second target binding ligand and comprising second nucleic acid first hybridizing domain linked to a toehold domain (x), wherein the toehold domain (x) is at an end distal to the end linked to the second target binding ligand; and a second nucleic acid second strand comprising a second nucleic acid second hybridizing domain linked to a primer binding domain (d*) that is linked to a toehold domain (a*).
Embodiment 43: The composition of any one of the preceding Embodiments, wherein the blocking probe nucleic acid toeholdcomprises a toehold domain (x*) at an end distal to the end linked to the blocking ligand, wherein the toehold domain (x*) is substantially complimentary to the toehold domain (x) of the first reporter probe.
Embodiment 44: The composition of any one of the preceding Embodiments, wherein the blocking probe nucleic acid comprises a first toehold domain (a) that is linked to a second toehold domain (x*), wherein the toeholdsecond toehold domain (x*) is at an end distal to the end linked to the blocking ligand, wherein the toehold domain (x*) is substantially complimentary to the toehold domain (x) of the first reporter probe and/or the toeholdsecond reporter probe, and wherein the first toehold domain (a) is substantially identical to the toehold domain (a) of the first reporter probe.
Embodiment 45: A composition for detecting a target in a sample, the composition comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the absence of the target and no more than one time in presence of the target.
Embodiment 46: The composition of Embodiment 45, wherein the first nucleic acid comprises a double-stranded nucleic acid linked to the first target binding ligand, wherein the double-stranded nucleic acid comprises:
   a. a first nucleic acid first strand linked to the first target binding ligand and comprising a single-stranded toehold domain (a) at a first end of the double stranded nucleic acid; and
   b. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and
   wherein the double stranded nucleic acid comprises a first stopper molecule in the first nucleic acid first strand and a second stopper molecule in the first nucleic acid second strand in the duplex region.
Embodiment 47: The composition of Embodiment 41 or 42, wherein the first nucleic acid first strand comprises a primer binding domain (c*) linked to a first subdomain that is linked to a stopper molecule (•) that is linked to a second subdomain (b) that is linked to the toehold domain (a).
Embodiment 48: The composition of any one of Embodiments 41-43, wherein the first nucleic acid first strand comprises a first nucleic acid second hybridizing domain linked to a primer binding domain (c*) that is linked to a first subdomain that is linked to a stopper molecule (•) that is linked to the second subdomain (b) that is linked to the toehold domain (a).
Embodiment 49: The composition of any one of Embodiments 41-44, wherein the first nucleic acid second strand comprises a first subdomain (b*) linked to a second subdomain that is linked to a stopper molecule (•) that is linked to a primer domain (c) that is linked to the toehold domain (b), wherein the toehold domain is substantially complementary to the first subdomain (b*).
Embodiment 50: The composition of any one of Embodiments 41-45, wherein the blocking probe nucleic acid comprises a toehold domain (a*) at an end distal to the end linked to the blocking ligand, wherein the toehold domain (a*) is substantially complimentary to the toehold domain (a) of the first reporter probe.
Embodiment 51: The composition of any one of Embodiments 41-46, wherein the blocking probe nucleic acid comprises a primer binding domain (d*) that is linked to a toehold domain (a*), wherein the toehold domain (a*) is at an end distal to the end linked to the blocking ligand, wherein the toehold domain (a*) is substantially complimentary to the toehold domain (a) of the first reporter probe.
Embodiment 52: The composition of any one of the preceding Embodiments, wherein the first target binding ligand and the blocking ligand are a binding pair.
Embodiment 53: The composition of any one of the preceding Embodiments, wherein the second target binding ligand and the blocking ligand are a binding pair.
Embodiment 54: The composition of any one of the preceding Embodiments, wherein the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 55: The composition of any one of the preceding Embodiments, wherein the first target binding ligand and the second target binding ligand are the same.
Embodiment 56: The composition of any one of the preceding Embodiments, wherein the first target binding ligand and the second target binding ligand are different.
Embodiment 57: The composition of any one of the preceding Embodiments, wherein the first target binding ligand is a receptor.
Embodiment 58: The composition of any one of the preceding Embodiments, wherein the first target binding ligand is a receptor, and the blocking ligand is a ligand of the receptor.
Embodiment 59: The composition of any one of the preceding Embodiments, wherein the second target binding ligand is a class specific antibody.
Embodiment 60: The composition of any one of the preceding Embodiments, wherein the toehold domain (x) of the first reporter probe and the toehold domain (x*) of the blocking probe are hybridized to each other.
Embodiment 61: The composition of any one of the preceding Embodiments, wherein the toehold domain (a*) of the second reporter probe and the toehold domain (a) of the blocking probe are hybridized to each other.
Embodiment 62: The composition of any one of the preceding Embodiments, wherein the toehold domain (x) of the second reporter probe and the toehold domain (x*) of the blocking probe are hybridized to each other.
Embodiment 63: The composition of any one of the preceding Embodiments, wherein the toehold domain (x) of the first reporter probe and the toehold domain (x*) of the blocking probe are hybridized to each other, and/or wherein the toehold domain (x) of the second reporter probe and the toehold domain (x*) of the blocking probe are hybridized to each other.
Embodiment 64: A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 65: The composition of Embodiment 64, wherein the composition further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 66: The composition of Embodiment 65, wherein the composition further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking ligand hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
Embodiment 67: A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 68: The composition of Embodiment 67, wherein the composition further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 69: The composition of Embodiment 68, wherein the composition further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.
Embodiment 70: The composition of any one of Embodiments 64-69, wherein the first nucleic acid first strand comprises a primer binding domain (c*) linked to a first subdomain that is linked to a stopper molecule (•) that is linked to a second subdomain (b) that is linked to the toehold domain (a).
Embodiment 71: The composition of any one of Embodiments 64-70, wherein the first nucleic acid first strand comprises the hybridizing domain (Lk1) linked to a primer binding domain (c*) that is linked to a first subdomain that is linked to a stopper molecule (•) that is linked to a second subdomain (b) that is linked to the toehold domain (a), wherein the hybridizing domain (Lk1) is substantially complementary to the hybridizing domain (Lk1*) of the linking strand linked to the target binding ligand.
Embodiment 72: The composition of any one Embodiments 64-71, wherein the first nucleic acid second strand comprises a first subdomain (b*) linked to a second subdomain that is linked to a stopper molecule (•) that is linked to a primer domain (c) that is linked to the toehold domain (b).
Embodiment 73: The composition of any one of Embodiments 64-72, wherein the first nucleic acid second strand comprises a first subdomain (b*) linked to a second subdomain that is linked to a stopper molecule (•) that is linked to a primer domain (c) that is linked to the toehold domain (b) that is linked to the toehold domain (x).
Embodiment 74: The composition of any one of Embodiments 64-73, wherein the second nucleic acid second strand comprises the second nucleic acid second hybridizing domain (Lk2) linked to a primer binding domain (d*) linked to the toehold domain (a*).
Embodiment 75: The composition of any one of Embodiments 64-74, wherein the first target binding ligand and the first blocking ligand are a binding pair.
Embodiment 76: The composition of any one of Embodiments 64-75, wherein the second target binding ligand and the second blocking ligand are a binding pair.
Embodiment 77: The composition of any one of Embodiments 64-76, wherein the first target binding ligand and the first blocking ligand and/or the second target binding ligand and the second blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 78: The composition of any one of Embodiments 64-77, wherein the first target binding ligand and the second target binding ligand are the same.
Embodiment 79: The composition of any one of Embodiments 64-77, wherein the first target binding ligand and the second target binding ligand are different.
Embodiment 80: The composition of any one of Embodiments 64-79, wherein the first target binding ligand is a receptor.
Embodiment 81: The composition of any one of Embodiments 64-80, wherein the first target binding ligand is a receptor, and the first blocking ligand is a ligand of the receptor.
Embodiment 82: The composition of any one of Embodiments 64-81, wherein the second target binding ligand is a receptor, and the second blocking ligand is a ligand of the receptor.
Embodiment 83: The composition of any one of the preceding Embodiments, wherein the composition further comprises one or more components for nucleic acid amplification.
Embodiment 84: The composition of any one of the preceding Embodiments, wherein the composition further comprises a polymerase.
Embodiment 85: The composition of Embodiment 84, wherein said polymerase is a strand displacing polymerase.
Embodiment 86: The composition of any one of the preceding Embodiments, wherein the composition further comprises dNTPs.
Embodiment 87: The composition of any one of the preceding Embodiments, wherein the composition further comprises a salt.
Embodiment 88: The composition of any one of the preceding Embodiments, wherein the composition further comprises a buffer.
Embodiment 89: The composition of any one of the preceding Embodiments, wherein the composition further comprises the target.
Embodiment 90: A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 91: The kit of Embodiment 82, further comprising:
   a second reporter probe comprising a second target binding ligand linked to a second nucleic acid, wherein a portion of the first nucleic acid and a portion of the second nucleic acid are capable of hybridizing,
   wherein a portion of the second nucleic acid and a portion of the blocking probe nucleic acid are capable of hybridizing in the absence of the target.
Embodiment 92: The kit of Embodiment 82 or 83, wherein the first nucleic acid comprises a double-stranded nucleic acid linked to the target binding ligand, wherein the double-stranded nucleic acid comprises:
   a. a first nucleic acid first strand linked to the first target binding ligand and comprising a single-stranded toehold domain (a) at a first end of the double stranded nucleic acid; and
   b. a first nucleic acid second strand substantially complementary to the first nucleic acid first strand, forming a double-stranded region (duplex region) with the first nucleic acid first strand and comprising toehold domains (x and b) at a second end of the double-stranded nucleic acid, and
   wherein the double stranded nucleic acid comprises a first stopper molecule in the first nucleic acid first strand and a second stopper molecule in the first nucleic acid second strand in the duplex region.
Embodiment 93: The kit of any one of the preceding Embodiments, wherein the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
   a. a first nucleic acid first strand hybridized to the linking strand linked to the first target binding ligand and comprising a first nucleic acid second hybridizing domain linked to a single-stranded toehold domain (a) at an end distal from the first nucleic acid second hybridizing domain; and
   b. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region.
Embodiment 94: The kit of any one of the preceding Embodiments, wherein the first nucleic acid first strand comprises a primer binding domain (c*) linked to a first subdomain that is linked to a stopper molecule (•) that is linked to a second subdomain (b) that is linked to the toehold domain (a).
Embodiment 95: The kit of any one of the preceding Embodiments, wherein the first nucleic acid first strand comprises a first nucleic acid second hybridizing domain linked to a primer binding domain (c*) that is linked to a first subdomain that is linked to a stopper molecule (•) that is linked to a second subdomain (b) that is linked to the toehold domain (a), wherein the first nucleic acid second hybridizing domain is substantially complementary to the first nucleic acid first hybridizing domain of the linking strand linked to the first target binding ligand.
Embodiment 96: The kit of any one of the preceding Embodiments, wherein the first nucleic acid second strand comprises a first subdomain (b*) linked to a second subdomain that is linked to a stopper molecule (•) that is linked to a primer domain (c) that is linked to the toehold domain (b).
Embodiment 97: The kit of any one of the preceding Embodiments, wherein the first nucleic acid second strand comprises a first subdomain (b*) linked to a second subdomain that is linked to a stopper molecule (•) that is linked to a primer domain (c) that is linked to the toehold domain (b) that is linked to the toehold domain (x).
Embodiment 98: The kit of any one of the preceding Embodiments, wherein the second reporter probe comprises a second nucleic acid linked to the second target binding ligand and comprises a toehold domain (a*) that is substantially complementary to the toehold domain (a) of the first reporter probe.
Embodiment 99: The kit of any one of the preceding Embodiments, wherein the second reporter probe comprises a second nucleic acid first strand linked to the second target binding ligand and comprises a primer binding domain (d*) linked to a toehold domain (a*), wherein the toehold domain (a*) is substantially complementary to the toehold domain (a) of the first reporter probe.
Embodiment 100: The kit of any one of the preceding Embodiments, wherein the second reporter probe comprises a second nucleic acid first strand linked to the toeholdsecond target binding ligand and comprising a second nucleic acid first hybridizing domain linked to a toehold domain (x), wherein the toehold domain (x) is at an end distal to the end linked to the second target binding ligand; and a second nucleic acid second strand comprising a second nucleic acid second hybridizing domain linked to a toehold domain (a*), wherein the second nucleic acid first hybridizing domain and the second nucleic acid second hybridizing domain are substantially complementary to each other.
Embodiment 101: The kit of any one of the preceding Embodiments, wherein the second reporter probe comprises a second nucleic acid first strand linked to the second target binding ligand and comprising a second nucleic acid first hybridizing domain linked to a toehold domain (x), wherein the toehold domain (x) is at an end distal to the end linked to the second target binding ligand; and a second nucleic acid second strand comprising a second nucleic acid second hybridizing domain linked to a primer binding domain (d*) that is linked to a toehold domain (a*).
Embodiment 102: The kit of any one of the preceding Embodiments, wherein the blocking probe comprises a blocking probe nucleic acid comprising a toehold domain (x*) at an end distal to the end linked to the blocking ligand, wherein the toehold domain (x*) is substantially complimentary to the toehold domain (x) of the first reporter probe.
Embodiment 103: The kit of any one of the preceding Embodiments, wherein the blocking probe comprises a blocking probe nucleic acid comprising a first toehold domain (a) that is linked to a second toehold domain (x*), wherein the toeholdsecond toehold domain (x*) is at an end distal to the end linked to the blocking ligand, wherein the toeholdsecond toehold domain (x*) is substantially complimentary to the toehold domain (x) of the first reporter probe and/or the toeholdsecond reporter probe, and wherein the first toehold domain (a) is substantially identical to the toehold domain (a) of the first reporter probe.
Embodiment 104: A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the absence of the target and no more than one time in the presence of the target.
Embodiment 105: The kit of Embodiment 96, wherein the first nucleic acid comprises a double-stranded nucleic acid linked to the first target binding ligand, wherein the double-stranded nucleic acid comprises:
   a. a first nucleic acid first strand linked to the first target binding ligand and comprising a single-stranded toehold domain (a) at a first end of the double stranded nucleic acid; and
   b. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and
   wherein the double stranded nucleic acid comprises a first stopper molecule in the first nucleic acid first strand and a second stopper molecule in the first nucleic acid second strand in the duplex region.
Embodiment 106: The kit of Embodiment 96 or 97, wherein the first nucleic acid first strand comprises a primer binding domain (c*) linked to a first subdomain that is linked to a stopper molecule (•) that is linked to a second subdomain (b) that is linked to the toehold domain (a).
Embodiment 107: The kit of any one of Embodiments 96-98, wherein the first nucleic acid first strand comprises a first nucleic acid second hybridizing domain linked to a primer binding domain (c*) that is linked to a first subdomain that is linked to a stopper molecule (•) that is linked to the second subdomain (b) that is linked to the toehold domain (a).
Embodiment 108: The kit of any one of Embodiments 96-99, wherein the first nucleic acid second strand comprises a first subdomain (b*) linked to a second subdomain that is linked to a stopper molecule (•) that is linked to a primer domain (c) that is linked to the toehold domain (b), wherein the toehold domain is substantially complementary to the first subdomain (b*).
Embodiment 109: The kit of any one of Embodiments 96-100, wherein the blocking probe nucleic acid comprises a toehold domain (a*) at an end distal to the end linked to the blocking ligand, wherein the toehold domain (a*) is substantially complimentary to the toehold domain (a) of the first reporter probe.
Embodiment 110: The kit of any one of Embodiments 96-101, wherein the blocking probe nucleic acid comprises a primer binding domain (d*) that is linked to a toehold domain (a*), wherein the toehold domain (a*) is at an end distal to the end linked to the blocking ligand, wherein the toehold domain (a*) is substantially complimentary to the toehold domain (a) of the first reporter probe.
Embodiment 111: The kit of any one of the preceding Embodiments, wherein the first target binding ligand and the blocking ligand are a binding pair.
Embodiment 112: The kit of any one of the preceding Embodiments, wherein the second target binding ligand and the blocking ligand are a binding pair.
Embodiment 113: The kit of any one of the preceding Embodiments, wherein the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 114: The kit of any one of the preceding Embodiments, wherein the first target binding ligand and the second target binding ligand are the same.
Embodiment 115: The kit of any one the preceding Embodiment, wherein the first target binding ligand and the second target binding ligand are different.
Embodiment 116: The kit of any one of the preceding Embodiments, wherein the first target binding ligand is a receptor.
Embodiment 117: The kit of any one of the preceding Embodiments, wherein the first target binding ligand is a receptor, and the blocking ligand is a ligand of the receptor.
Embodiment 118: The kit of any one of the preceding Embodiments, wherein the second target binding ligand is a class specific antibody.
Embodiment 119: The kit of any one of the preceding Embodiments, wherein the toehold domain (x) of the first reporter probe and the toehold domain (x*) of the blocking probe are hybridized to each other.
Embodiment 120: The kit of any one of the preceding Embodiments, wherein the toehold domain (a*) of the second reporter probe and the toehold domain (a) of the blocking probe are hybridized to each other.
Embodiment 121: The kit of any one of the preceding Embodiments, wherein the toehold domain (x) of the second reporter probe and the toehold domain (x*) of the blocking probe are hybridized to each other.
Embodiment 122: The kit of any one of the preceding Embodiments, wherein the toehold domain (x) of the first reporter probe and the toehold domain (x*) of the blocking probe are hybridized to each other, and/or wherein the toehold domain (x) of the second reporter probe and the toehold domain (x*) of the blocking probe are hybridized to each other.
Embodiment 123: A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 124: The kit of Embodiment 123, wherein the kit further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 125: The kit of Embodiment 124, wherein the kit further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
Embodiment 126: A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
Embodiment 127: The kit of Embodiment 126, wherein the kit further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 128: The kit of Embodiment 127, wherein the kit further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.
Embodiment 129: The kit of any one of Embodiments 123-129, wherein the first nucleic acid first strand comprises a primer binding domain (c*) linked to a first subdomain that is linked to a stopper molecule (•) that is linked to a second subdomain (b) that is linked to the toehold domain (a).
Embodiment 130: The kit of any one of Embodiments 123-129, wherein the first nucleic acid first strand comprises the hybridizing domain (Lk1) linked to a primer binding domain (c*) that is linked to a first subdomain that is linked to a stopper molecule (•) that is linked to a second subdomain (b) that is linked to the toehold domain (a), wherein the hybridizing domain (Lk1) is substantially complementary to the hybridizing domain (Lk1*) of the linking strand linked to the first target binding ligand.
Embodiment 131: The kit of any one Embodiments 123-130, wherein the first nucleic acid second strand comprises a first subdomain (b*) linked to a second subdomain that is linked to a stopper molecule (•) that is linked to a primer domain (c) that is linked to the toehold domain (b).
Embodiment 132: The kit of any one of Embodiments 123-131, wherein the first nucleic acid second strand comprises a first subdomain (b*) linked to a second subdomain that is linked to a stopper molecule (•) that is linked to a primer domain (c) that is linked to the toehold domain (b) that is linked to the toehold domain (x).
Embodiment 133: The kit of any one of Embodiments 123-132, wherein the second nucleic acid second strand comprises the second nucleic acid second hybridizing domain (Lk2) linked to a primer binding domain (d*) linked to the toehold domain (a*).
Embodiment 134: The kit of any one of Embodiments 123-133, wherein the first target binding ligand and the first blocking ligand are a binding pair.
Embodiment 135: The kit of any one of Embodiments 123-134, wherein the second target binding ligand and the second blocking ligand are a binding pair.
Embodiment 136: The kit of any one of Embodiments 123-135, wherein the first target binding ligand and the first blocking ligand and/or the second target binding ligand and the second blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 137: The kit of any one of Embodiments 123-136, wherein the first target binding ligand and the second target binding ligand are the same.
Embodiment 138: The kit of any one of Embodiments 123-137, wherein the first target binding ligand and the second target binding ligand are different.
Embodiment 139: The kit of any one of Embodiments 123-138, wherein the first target binding ligand is a receptor.
Embodiment 140: The kit of any one of Embodiments 123-139, wherein the first target binding ligand is a receptor, and the first blocking ligand is a ligand of the receptor.
Embodiment 141: The kit of any one of Embodiments 123-140, wherein the second target binding ligand is a receptor, and the second blocking ligand is a ligand of the receptor.
Embodiment 142: The kit of any one of the preceding Embodiments, wherein the kit further comprises one or more components for nucleic acid amplification.
Embodiment 143: The kit of any one of the preceding Embodiments, wherein the kit further comprises a polymerase.
Embodiment 144: The kit of Embodiment 143, wherein said polymerase is a stand displacing polymerase.
Embodiment 145: The kit of any one of the preceding Embodiments, wherein the kit further comprises dNTPs.
Embodiment 146: The kit of any one of the preceding Embodiments, wherein the kit further comprises a salt.
Embodiment 147: The kit of any one of the preceding Embodiments, wherein the kit further comprises a buffer
Embodiment 148: The kit of any one of the preceding Embodiments, wherein the kit further comprises the target.

Some embodiments of the various aspects described herein are represented by the following numbered embodiments:
Embodiment 1) A method of detecting a target in a sample, the method comprising:
   a. contacting the sample with a first reporter probe and a blocking probe, wherein:
      i. the first reporter probe comprises a first target binding ligand linked to a first nucleic acid;
      ii. the blocking probe comprises a blocking ligand capable of directly or indirectly forming a complex with the first target binding ligand and linked to a blocking probe nucleic acid,
      iii. wherein a portion of the first nucleic acid and a portion of the blocking probe nucleic acid are capable of hybridizing in the absence of the target,
      iv. wherein binding of the target to the first reporter probe inhibits the formation of the complex, and
      v. wherein a portion of the first nucleic acid is capable of being extended at least two times to produce a nucleic acid record in the presence of the target and no more than one time in the absence of the target;
   b. providing a polymerase, thereby producing the nucleic acid record of an interaction between the first reporter probe and the target; and
   c. detecting the nucleic acid record.
Embodiment 2) The method of any one of the preceding Embodiments, wherein
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising a single-stranded toehold domain (a) at a first end of the double stranded nucleic acid; and
         2. a first nucleic acid second strand substantially complementary to the first strand, forming a double-stranded region (duplex region) with the first strand and comprising toehold domains (x and b) at a second end of the double-stranded nucleic acid, and
            a. wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid comprising a toehold domain (a*) that is substantially complementary to the toehold domain (a) of the double-stranded nucleic acid of the first reporter probe, and wherein the target can simultaneously bind with the first reporter probe and the second reporter probe;
      iii. the blocking ligand is capable of directly or indirectly forming a complex with the first target binding ligand and/or the second target binding ligand and is linked to the blocking probe nucleic acid, wherein the blocking probe nucleic acid comprises a first toehold domain (a) that is substantially identical to the toehold domain (a) of the first reporter probe, and a second toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 3) The method of any one of the preceding Embodiments, wherein:
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising single-stranded toehold domain (a) at a first end of the double stranded nucleic acid; and
         2. a first nucleic acid second strand substantially complementary to the first nucleic acid first strand, forming a double-stranded region (duplex region) with the first nucleic acid first strand and comprising toe hold domains (x and b) at a second end of the double-stranded nucleic acid, and
            a. wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the toehold domain (x) of the first reporter probe; a second nucleic acid second strand hybridized to the second nucleic acid first strand and comprising a second nucleic acid second hybridizing domain, substantially complementary to the second nucleic acid first hybridizing domain and linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or the second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 4) The method of any one of the preceding Embodiments, wherein:
   a. the contacting the sample with the first reporter probe and the blocking probe comprises contacting the sample with the first reporter probe, a second reporter probe, and the blocking probe, wherein:
      i. the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a single-stranded toehold domain (a) at an end distal from the toehold domain (x); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the pairing domain (x) of the first reporter probe; a second nucleic acid second strand hybridized to the second nucleic acid first strand and comprising a second nucleic acid second hybridizing domain, substantially complementary to the second nucleic acid first hybridizing domain, linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
Embodiment 5) A method of detecting a target in a sample, the method comprising:
   a. contacting the sample with a first reporter probe, wherein:
      i. the first reporter probe comprises a first target binding ligand linked to a first nucleic acid;
   b. contacting the sample with a blocking probe, wherein:
      i. the blocking probe comprises a blocking ligand capable of directly or indirectly forming a complex with the first target binding ligand and linked to a blocking probe nucleic acid,
      ii. wherein a portion of the first nucleic acid and a portion of the blocking probe nucleic acid are capable of hybridizing in the absence of the target,
      iii. wherein binding of the target to the first reporter probe inhibits the formation of the complex, and
      iv. wherein a portion of the first nucleic acid is capable of being extended at least two times to produce a nucleic acid record in the absence of the target and no more than one time in the presence of the target;
   c. providing a polymerase, thereby producing the nucleic acid record of an interaction between the first nucleic acid and the blocking probe nucleic acid if said interaction is present; and
   d. detecting the presence or absence of the nucleic acid record, and wherein the absence of the nucleic acid record indicates the target is present in the sample.
Embodiment 6) The method of any one of the preceding Embodiments, wherein
   i. the first nucleic acid comprises a double stranded nucleic acid, wherein the double-stranded nucleic acid comprises:
      1. a first nucleic acid first strand linked to the first target binding ligand and comprising a single-stranded toehold domain (a) at a first end of the double stranded nucleic acid; and
      2. a first nucleic acid second strand substantially complementary to the first strand, forming a double-stranded region (duplex region) with the first nucleic acid first strand and comprising a toehold domain (b) at a second end of the double-stranded nucleic acid, and
         a. wherein the double-stranded nucleic acid comprises a first stopper molecule in the first nucleic acid first strand and a second stopper molecule in the first nucleic acid second strand in the duplex region;
   ii. the blocking probe nucleic acid comprises: a first toehold domain (a) that is substantially identical to the toehold domain (a) of the first reporter probe; and a primer binding domain (d*).
Embodiment 7) A method of detecting a target in a sample, the method comprising:
   a. contacting the sample with a first reporter probe and a second reporter probe, wherein:
      i. the first reporter probe comprises a first target binding ligand capable of binding the target and linked to first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
         1. wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a blocking ligand capable of directly or indirectly forming a complex with the first target binding ligand and linked to a blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
   c. providing a polymerase, thereby producing a nucleic acid record of an interaction between the first nucleic acid and the second nucleic acid if said interaction is present; and
   d. detecting the presence or absence of the nucleic acid record, and wherein the presence of the nucleic acid record indicates the target is present in the sample.
Embodiment 8) A method of detecting a target in a sample, the method comprising:
   a. contacting the sample with a first reporter probe and a second reporter probe, wherein:
      i. the first reporter probe comprises first target binding ligand capable of binding the target and linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
         1. wherein the toehold domain (b) of the first reporter probe comprises a nucleotide that is not present in the hybridizing domain (Lk1) of the first reporter probe and the hybridizing domain (Lk2) of the second probe, and
         2. wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a blocking ligand capable of directly or indirectly forming a complex with the first target binding ligand and linked to a blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the first blocking probe from forming a complex, 1. ; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex;
   c. providing a polymerase and a first dNTP mix, where the first dNTP mix is substantially free of a dNTP complementary to the nucleotide present in the toehold domain (b) of the first reporter probe but not in the hybridizing domain (Lk1) of the first reporter probe and the hybridizing domain (Lk2) of the second reporter probe;
   d. providing a second dNTP mix. and optionally a polymerase, wherein the second dNTP mix comprises a dTNP complementary to the nucleotide present in the toehold domain (a) or (b) of the first reporter probe but not in the hybridizing domain (Lk1) of the first reporter probe and the hybridizing domain (Lk2) of the second reporter probe, thereby producing a nucleic acid record of an interaction between the first nucleic acid and the second nucleic acid if said interaction is present; and
   e. detecting the presence or absence of the nucleic acid record, and wherein the presence of the nucleic acid record indicates the target is present in the sample
Embodiment 9) The method of any one of the preceding Embodiments, wherein the providing the polymerase extends one or more of the nucleic acids.
Embodiment 10) The method of any one of the preceding Embodiments, wherein the method further comprises amplifying the nucleic acid record, if present, prior to detecting the presence or the absence of nucleic acid record.
Embodiment 11) The method of any one of the preceding Embodiments, wherein the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
Embodiment 12) The method of any one of the preceding Embodiments, wherein the target is a biomolecule.
Embodiment 13) The method of any one of the preceding Embodiments, wherein the target is an antibody, a nucleic acid, a small molecule, or an antigen.
Embodiment 14) The method of any one of the preceding Embodiments, wherein the target is a neutralizing antibody, an antigen, a spike protein, a nucleic acid, or a small molecule.
Embodiment 15) The method of any one of the preceding Embodiments, wherein the antigen is comprised in a vaccine, or wherein the antigen is encoded by a vaccine.
Embodiment 16) The method of any one of the preceding Embodiments, wherein the sample is a biological sample.
Embodiment 17) A composition for detecting a target in a sample, the composition comprising:
   a. a first reporter probe comprising a target binding ligand linked to a first nucleic acid; and
   b. a blocking probe comprising a blocking ligand capable of directly or indirectly forming a complex with the target binding ligand of the first reporter probe and linked to a blocking probe nucleic acid, and
   c. wherein a portion of the first nucleic acid and a portion of the blocking probe nucleic acid are capable of hybridizing in the absence of the target,
   d. wherein binding of the target to the first reporter probe inhibits the formation of the complex, and
   e. wherein a portion of the first nucleic acid is capable of being extended at least two times to produce a nucleic acid record in the presence of the target and no more than one time in the absence of the target.
Embodiment 18) The composition of any one of the preceding Embodiments, further comprising:
   a. a second reporter probe comprising a second target binding ligand linked to a second nucleic acid,
   b. wherein a portion of the first nucleic acid and a portion of the second nucleic acid are capable of hybridizing, and
   c. wherein a portion of the second nucleic acid and a portion of the blocking probe nucleic acid are capable of hybridizing in the absence of the target.
Embodiment 19) A composition for detecting a target in a sample, the composition comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and
   c. wherein a portion of the first nucleic acid and a portion of the blocking probe nucleic acid are capable of hybridizing in the absence of the target,
   d. wherein binding of the target to the first reporter probe inhibits the formation of the complex, and
   e. wherein a portion of the first nucleic acid is capable of being extended at least two times to produce a nucleic acid record in the absence of the target and no more than one time in presence of the target.
Embodiment 20) A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
   iii. wherein binding of the target to the first reporter probe inhibits the formation of the complex, and
   iv. wherein a portion of the first nucleic acid is capable of being extended at least two times to produce a nucleic acid record in the presence of the target and no more than one time in the absence of the target.
Embodiment 21) The composition any one of the preceding Embodiments, wherein the composition further comprises a second reporter probe, and wherein:
   a. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 22) The composition any one of the preceding Embodiments, wherein the composition further comprises a second blocking probe, and wherein:
   a. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
Embodiment 23) A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and
   iii. wherein binding of the target to the first reporter probe inhibits the formation of the complex, and
   iv. wherein a portion of the first nucleic acid is capable of being extended at least two times to produce a nucleic acid record in the presence of the target and no more than one time in the absence of the target.
Embodiment 24) The composition any one of the preceding Embodiments, wherein the composition further comprises a second reporter probe, and wherein:
   a. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 25) The composition any one of the preceding Embodiments, wherein the composition further comprises a second blocking probe, and wherein:
   a. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.
Embodiment 26) A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and
   c. wherein a portion of the first nucleic acid and a portion of the blocking probe nucleic acid are capable of hybridizing in the absence of the target,
   d. wherein binding of the target to the first reporter probe inhibits the formation of the complex, and
   e. wherein a portion of the first nucleic acid is capable of being extended at least two times to produce a nucleic acid record in the presence of the target and no more than one time in the absence of the target.
Embodiment 27) The kit any one of the preceding Embodiments, further comprising:
   a. a second reporter probe comprising a second target binding ligand linked to a second nucleic acid, wherein a portion of the first nucleic acid and a portion of the second nucleic acid are capable of hybridizing,
   b. wherein a portion of the second nucleic acid and a portion of the blocking probe nucleic acid are capable of hybridizing in the absence of the target.
Embodiment 28) A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and
   c. wherein a portion of the first nucleic acid and a portion of the blocking probe nucleic acid are capable of hybridizing in the absence of the target,
   d. wherein binding of the target to the first reporter probe inhibits the formation of the complex, and
   e. wherein a portion of the first nucleic acid is capable of being extended at least two times to produce a nucleic acid record in the absence of the target and no more than one time in the presence of the target.
Embodiment 29) A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
   iii. wherein binding of the target to the first reporter probe inhibits the formation of the complex, and
   iv. wherein a portion of the first nucleic acid is capable of being extended at least two times to produce a nucleic acid record in the presence of the target and no more than one time in the absence of the target.
Embodiment 30) The kit any one of the preceding Embodiments, wherein the kit further comprises a second reporter probe, and wherein:
   a. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 31) The kit any one of the preceding Embodiments, wherein the kit further comprises a second blocking probe, and wherein:
   a. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
Embodiment 32) A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and
   iii. wherein binding of the target to the first reporter probe inhibits the formation of the complex, and
   iv. wherein a portion of the first nucleic acid is capable of being extended at least two times to produce a nucleic acid record in the presence of the target and no more than one time in the absence of the target.
Embodiment 33) The kit any one of the preceding Embodiments, wherein the kit further comprises a second reporter probe, and wherein:
   a. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
Embodiment 34) The kit any one of the preceding Embodiments, wherein the kit further comprises a second blocking probe, and wherein:
   a. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.

### Some selected definitions

For convenience, the meaning of some terms and phrases used in the specification, examples, and appended claims, are provided below. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments of the aspects provided herein, and are not intended to limit the claimed invention, because the scope of the invention is limited only by the claims. Further, unless otherwise required by context, singular terms shall include pluralities and plural terms shall include the singular.

Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737), the contents of which are all incorporated by reference herein in their entireties.

As used herein, "nucleic acid" means DNA, RNA, single-stranded, double-stranded, or more highly aggregated hybridization motifs, and any chemical modifications thereof.

As used herein, the term "hybridize" refers to the phenomenon of a single-stranded nucleic acid or region thereof forming hydrogen-bonded base pair interactions with either another single stranded nucleic acid or region thereof (intermolecular hybridization) or with another single-stranded region of the same nucleic acid (intramolecular hybridization). Hybridization is governed by the base sequences involved, with complementary nucleobases forming hydrogen bonds, and the stability of any hybrid being determined by the identity of the base pairs (*e.g.*, G:C base pairs being stronger than A:T base pairs) and the number of contiguous base pairs, with longer stretches of complementary bases forming more stable hybrids.

The term "substantially identical" means two or more nucleotide sequences have at least 65%, 70%, 80%, 85%, 90%, 95%, or 97% identical nucleotides. In some embodiments, "substantially identical" means two or more nucleotide sequences have the same identical nucleotides, i.e., same nucleotide sequence.

As used herein the term "complementary" generally refers to the potential for a hybridized pairing or binding interaction between two sets of nucleic acids. Complementary nucleic acids are capable of binding to one another through hydrogen bond pairing according to canonical Watson-Crick base pairing and non-Watson-Crick base pairing (*e.g.*, Wobble base pairing and Hoogsteen base pairing). In some embodiments, two sets of nucleic acids may be 100% complementary to one another. In other embodiments, two sets of nucleic acids may comprise 1, 2, 3, 4, 5, 6, 7, 8, 9, 10, or more nucleotides that are not complementary. In other embodiments, two sets of nucleic acids may be at least 50%, at least 60%, at least 70%, at least 80%, or at least 90% complementary. In some embodiments, two sets of nucleic acids are complementary so long as they are capable of forming a stable or transient complex. "Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs includes, but not limited to, G:U Wobble or Hoogsteen base pairing.

The term "substantially complementary" means two or more nucleotide sequences are at least 65%, 70%, 80%, 85%, 90%, 95%, or 97% complementary. In some embodiments, "substantially complementary" means two or more nucleotide sequences are 100% complementary to each other.

When a nucleic acid is referred to as "double-stranded," it is understood by those of skill in the art that a pair of nucleic acid strands exists in a hydrogen-bonded, helical array typically associated with, for example, DNA. In addition to the 100% complementary form of double-stranded oligonucleotides, the term "double-stranded" as used herein is also meant to include those form which include such structural features as bulges and loops (see Stryer, Biochemistry, Third Ed. (1988), incorporated herein by reference in its entirety for all purposes).

A "polymerase" refers to an enzyme that performs template-directed synthesis of polynucleotides, *e.g.*, DNA and/or RNA. The term encompasses both the full length polypeptide and a domain that has polymerase activity. DNA polymerases are well-known to those skilled in the art, including but not limited to DNA polymerases isolated or derived from *Pyrococcus furiosus, Thermococcus litoralis*, and *Thermotoga maritime*, or modified versions thereof. Additional examples of commercially available polymerase enzymes include, but are not limited to: Klenow fragment (New England Biolabs^{®} Inc.), Taq DNA polymerase (QIAGEN^{®}), 9° N^{™} DNA polymerase (New England Biolabs^{®} Inc.), Deep Vent^{™} DNA polymerase (New England Biolabs^{®} Inc.), Manta DNA polymerase (Enzymatics^{®}), Bst DNA polymerase (New England Biolabs^{®} Inc.), and phi29 DNA polymerase (New England Biolabs^{®} Inc.). Polymerases include both DNA-dependent polymerases and RNA-dependent polymerases such as reverse transcriptase. At least five families of DNA-dependent DNA polymerases are known, although most fall into families A, B and C. There is little or no sequence similarity among the various families. Most family A polymerases are single chain proteins that can contain multiple enzymatic functions including polymerase, 3' to 5' exonuclease activity and 5' to 3' exonuclease activity. Family B polymerases typically have a single catalytic domain with polymerase and 3' to 5' exonuclease activity, as well as accessory factors. Family C polymerases are typically multi-subunit proteins with polymerizing and 3' to 5' exonuclease activity. In *E. coli*, three types of DNA polymerases have been found, DNA polymerases I (family A), II (family B), and III (family C). In eukaryotic cells, three different family B polymerases, DNA polymerases α, δ, and ε, are implicated in nuclear replication, and a family A polymerase, polymerase γ, is used for mitochondrial DNA replication. Other types of DNA polymerases include phage polymerases. Similarly, RNA polymerases typically include eukaryotic RNA polymerases I, II, and III, and bacterial RNA polymerases as well as phage and viral polymerases.

As used herein, the terms "protein" and "polypeptide" are used interchangeably to designate a series of amino acid residues, connected to each other by peptide bonds between the alpha-amino and carboxy groups of adjacent residues. The terms "protein", and "polypeptide" refer to a polymer of amino acids, including modified amino acids (*e.g.*, phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when referring to a gene product and fragments thereof. Thus, exemplary polypeptides or proteins include gene products, naturally occurring proteins, homologs, orthologs, paralogs, fragments and other equivalents, variants, fragments, and analogs of the foregoing.

The term "antibody" broadly refers to any immunoglobulin (Ig) molecule and immunologically active portions of immunoglobulin molecules (*i.e*., molecules that contain an antigen binding site that immunospecifically bind an antigen) comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art. Non-limiting embodiments of which are discussed below, and include but are not limited to a variety of forms, including full length antibodies and antigen-binding portions thereof; including, for example, an immunoglobulin molecule, a monoclonal antibody, a chimeric antibody, a CDR-grafted antibody, a human antibody, a humanized antibody, a single chain antibody, a Fab, a F(ab'), a F(ab')2, a Fv antibody, fragments produced by a Fab expression library, a disulfide linked Fv, a scFv, a single domain antibody (dAb), a diabody, a multispecific antibody, a dual specific antibody, an anti-idiotypic antibody, a bispecific antibody, a functionally active epitope-binding fragment thereof, bifunctional hybrid antibodies (*e.g.*, Lanzavecchia et al., Eur. J. Immunol. 17, 105 (1987)) and single chains (*e.g.*, Huston et al., Proc. Natl. Acad. Sci. U.S.A., 85, 5879-5883 (1988) and Bird et al., Science 242, 423-426 (1988), which are incorporated herein by reference) and/or antigen-binding fragments of any of the above (See, generally, Hood et al., Immunology, Benjamin, N.Y., 2ND ed. (1984), Harlow and Lane, Antibodies. A Laboratory Manual, Cold Spring Harbor Laboratory (1988) and Hunkapiller and Hood, Nature, 323, 15-16 (1986), which are incorporated herein by reference). It is to be understood that the antibody can be a polyclonal antibody or monoclonal antibody. In addition, the antibody can be a human antibody and/or a humanized antibody.

As used herein, "contacting" refers to any suitable means for delivering, or exposing, at least one component as provided herein (*e.g.*, sample, a target, etc.). In some embodiments, contacting comprises physical human activity, *e.g.*, an injection; an act of dispensing, mixing, and/or decanting; and/or manipulation of a delivery device or machine.

As used herein the term "comprising" or "comprises" is used in reference to compositions, methods, and respective component(s) thereof, that are essential to the method or composition, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

As used herein the term "extended . . . at least two times," "extended . . . no more than one time," or "extended" a given number of times refers to a plurality of nucleic acids, the great majority of which being "extended . . . at least two times," "extended . . . no more than one time," or "extended" said given number of times. The great majority may be at least 90%, at least 91%, at least 92%, at least 93%, at least 94%, at least 95%, at least 96%, at least 97%, at least 98%, at least 99%, at least 99.5%, or at least 99.9% of the plurality. The term does not require, but may encompass, that each and every nucleic acid within the plurality (that is, 100% of the plurality) be extended as such.

The singular terms "a," "an," and "the" include plural referents unless context clearly indicates otherwise. Similarly, the word "or" is intended to include "and" unless the context clearly indicates otherwise. Although methods and materials similar or equivalent to those provided herein can be used in the practice or testing of this disclosure, suitable methods and materials are described below. The abbreviation, "*e.g.*" is derived from the Latin *exempli gratia*, and is used herein to indicate a non-limiting example. Thus, the abbreviation "*e.g.*" is synonymous with the term "for example."

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Further, unless otherwise required by context, singular terms shall include pluralities and plural terms shall include the singular.

Other than in the operating examples, or where otherwise indicated, all numbers expressing quantities of ingredients or reaction conditions used herein should be understood as modified in all instances by the term "about." The term "about" when used in connection with percentages can mean ±5% (*e.g.*, ±4%, ±3%, ±2%, ±1%) of the value being referred to.

Where a range of values is provided, each numerical value between the upper and lower limits of the range is contemplated and disclosed herein.

Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow. Further, to the extent not already indicated, it will be understood by those of ordinary skill in the art that any one of the various embodiments herein described and illustrated can be further modified to incorporate features shown in any of the other embodiments disclosed herein.

The description of embodiments of the disclosure is not intended to be exhaustive or to limit the disclosure to the precise form disclosed. While specific embodiments of, and examples for, the disclosure are disclosed herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments disclosed herein can be combined to provide further embodiments. Aspects of the disclosure can be modified, if necessary, to employ the compositions, functions and concepts of the above references and application to provide yet further embodiments of the disclosure.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

It will be apparent to those skilled in the relevant art that various modifications, additions, substitutions, and the like can be performed without altering the spirit or scope of the invention, and such modifications and variations are encompassed within the scope of the invention as defined in the claims which follow.

### Examples

The examples are provided for illustrative purposes only and not to limit the scope of the claims provided herein.

General methods and materials for the following Examples are as follows:
**Dried blood spot (DBS) sample preparation:** 6 mm DBS punches were obtained using a manual DBS puncher or a semi-automated DBS puncher and placed in 1.5 mL tubes. The puncher was cleaned by punching blank cards five times between samples. 500 µL DBS elution buffer was added in each tube and the samples were eluted for 1 hour at 37 °C with shaking at 500 rpm. The DBS elution buffer contained 1% BSA, 0.2% NP40, 0.2 × Protease inhibitor in 1 × PBS. The DBS eluates were spun down at 10,000 × g for 10 minutes, and 400 µL supernatant was collected for each sample.
**SARS-CoV-2 S1 protein detection SPEAR assay:** In Examples 12 and 13, two commercially available monoclonal antibodies targeting S1 proteins were conjugated with SPEAR DNA sequence S and P. DBS eluates from SARS-CoV-2 vaccine recipients were incubated with the SPEAR probes at 37 °C for 1 hour, followed by the enzymatic reaction and qPCR amplification. No heating step was added before the enzymatic reaction.
**SARS-CoV-2 Pan-IgG binding SPEAR assay:** In Examples 1, 2, 3, 11 and 13, Fc-tagged SARS-CoV-2 S1 proteins were conjugated with SPEAR DNA sequence S or P. DBS eluates from SARS-CoV-2 vaccine recipients were incubated with the SPEAR probes at 25 °C for 1 hour, followed by the enzymatic reaction and qPCR as described above. No heating step was added before the enzymatic reaction. For longitudinal experiments, DBS samples from vaccinated subjects were further diluted in 50 folds using DBS elution buffer.
**SARS-CoV-2 neutralizing antibody detection assay:** In Examples 2, 5, 6, 7, 9, 10, 13 and 14, SPEAR DNA sequence S-conjugated S1 proteins were incubated with the samples at 4 °C overnight or at room temperature for 2 hours, to allow antibodies in the samples to bind S1 proteins. SPEAR DNA sequence P-conjugated ACE2 proteins were then incubated with the samples at room temperature for 30 minutes, to allow ACE2 proteins to compete with the antibodies for S1 protein binding. It was followed by the enzymatic reaction to generate SPEAR DNA signal molecules. The more neutralizing antibodies the sample contained, the fewer ACE2 proteins could bind to S1 proteins, thus giving lower SPEAR signals. The positive control sample contained 1 nM R2B17 antibodies (a monoclonal SARS-CoV-2 neutralizing antibody, Genscript), and the negative control sample contained only the sample dilution buffer but no antibodies, which was used to represent 0% inhibition. The inhibition of the sample was calculated from the SPEAR signal difference between the sample and the negative control sample, using the equation below $Inhibition = \left(1-\frac{1}{{2}^{Cycle number difference}}\right) ∗ 100 %$

For quantitative neutralization titer measurement, the samples were serially diluted (DBS samples were diluted at 1:2 from 2 to 256 folds, and serum/plasma samples were diluted at 1:4 from 10 to 163840 folds), and the inhibition curve was plotted using 4PL Sigmoidal fitting and the dilution fold for 50% inhibition (i.e., NT50) was calculated from the curve using Prism 9. The final NT50 in whole blood was derived by multiplying the interpolated NT50 with 50. The multiplication number 50 was derived by estimating that the 6 mm DBS punch contained ~ 10 µL blood and eluted in 500 µL DBS elution buffer.

**Antibody binding detection using ELISA:** In Examples 1, 7 and 11, monoclonal antibodies targeting SARS-CoV-2 RBD proteins were purchased from Genscript. Fc-tagged RBD proteins were coated on a MaxiSorp^{™} 96 well ELISA plate at 1 µg/ mL overnight at 4 °C. After washing, the plate was blocked with assay diluent at room temperature for 1 hour. RBD-targeting antibodies were serially diluted at 1:4 in SARS-CoV-2 antibody-negative DBS eluates, starting from 8 µg/mL. The samples were incubated with RBD-coated plates at room temperature for 1 hour, followed by washing and HRP-conjugated secondary antibody incubation at room temperature for 1 hour (anti-Human 1:10000, anti-Mouse 1:25000, anti-Rabbit 1:10000). Substrate reaction was conducted for 15 minutes in dark and the sample absorbance was measured using a microplate reader at 450 nm.

**CPass^{™} SARS-CoV-2 neutralization antibody detection assay:** In Example 8, the CPass^{™} kits were purchased from Genscript, and the vendor's protocol was followed. In brief, the ACE2-coated plates were blocked with the blocking buffer (ELISA assay diluent) at room temperature for 1 hour. The samples and controls were diluted with sample dilution buffer with a volume ratio of 1:9. The samples were then incubated with HRP-conjugated RBD proteins at 37 °C for 30 minutes, followed by incubation with ACE2 plates at 37 °C for 15 minutes. After washing, TMB solution was added for 15 minutes for substrate reaction, and the sample absorbance was measured using a microplate reader at 450 nm. $Inhibition = \left(1-\frac{OD value of sample}{OD value of negative control sample}\right) \times 100 %$

### Plaque reduction neutralization test (PRNT) and pseudovirus-based neutralization assay

**PRNT:** In Example 9 and 14, the PRNT assay was conducted by Corgenix, Inc. (Broomfield, CO, USA). In detail, Vero E6 cells were maintained in six-well plates, and SARS-CoV-2 viruses (WA01/2020 isolate) were diluted to approximately 2000 pfu/ mL (after dilution in serum/plasma samples in a 1:1 ratio, the final virus concentration is ~ 1000 pfu/ mL). SARS-CoV-2 vaccine recipient serum/plasma samples were serially diluted in 2-fold using BA-1 medium from 1:5 to 1:10240 (i.e., 1:10 to 1:20480 after mixing with the virus solution). The serum/plasma samples were mixed with virus in a 1:1 ratio, and incubated at 37 °C for 1 hour. The serum/plasma-virus mixtures were added onto Vero E6 cells and incubated at 37 °C for 45 minutes with rocking to distribute the medium every 15 minutes. The cell monolayer was then overlaid with 1% agarose inn cell culture medium and incubated at 37 °C for 24 hours. A second layer containing 1% agarose and 0.005% neutral red was formed on top of the first layer, and the samples were incubated at 37 °C for 24 hours. Count the number of plaques in each well. For each assay, three negative control samples and one positive control samples were included. The inhibition value for each dilution was calculated using the equation below and the 50% inhibition (NT50) was calculated by fitting the inhibition vs dilution fold curve using 4PL sigmoidal fitting. $Inhibition = \left(1-\frac{Plaque number of sample}{Plaque number of negative control sample}\right) \times 100 %$

**Pseudovirus-based neutralization assay:** In Example, 8, pseudovirus (SARS-CoV-2-spike-pseudotyped HIV virions) was produced in house and the neutralization assay was done as described in Si et al. (A human-airway-on-a-chip for the rapid identification of candidate antiviral therapeutics and prophylactics. Nat Biomed Eng 5, 815-829 (2021)). Serum/plasma samples were serially diluted in 2-fold in sample dilution buffer containing 1% BSA in 1× PBS from 1:2 to 1:256 (after mixing the virus and cells, the final dilution fold was 1:20 to 1:2560). 20 µL samples were mixed with 80 µL virus solution (MOI = 0.1), and incubated at 37 °C for 30 minutes. The mixture was then mixed with 100 µL medium containing 20,000 ACE2-293T cells, and incubated at 37 °C for 72 hours. Luciferase activity that reflects the number of pseudovirus particle in host cells was measured using the Bright-Glo^{™} luciferase assay system.

### Example 1. Anti-SARS-Co V-2 Antibody Assay (Antibody R2B17).

FIG. 13 shows anti-SARS-COV-2-RBD antibody assay demonstrated by a monoclonal antibody R2B17. The basic construct of the assay in which two RBD conjugates bind to an anti-RBD mAb produces a PCR-amplifiable sequence d*a*b*c*. Comparing to a conventional ELISA assay using the same RBD protein as the capture antigen, the SPEAR (Successive Proximity Extension Amplification Reaction) assay has achieved 1000 times higher sensitivity with only one percent of the sample volume. Particularly, this binding assay shows a linear range of five orders of magnitude and minimum variation between repeats.

### Example 2. Anti-SARS-CoV-2 Antibody Assay (Multiple Antibodies).

FIG. 14 is a bar-graph showing anti-SARS-COV-2-RBD antibody assay test by 14 monoclonal antibodies. Only one antibody did not have a response due to the RBD recombinant protein sequence used in the assay.

### Example 3. Anti-SARS-CoV-2 Antibody Assay (Single Antibody).

FIG. 15 is a bar graph showing high binding yield can be achieved between human ACE2 conjugate (50nM) and SARS-COV-2 RBD conjugate (5pM/10pM) after incubation at room temperature for 2h.

### Example 4. Anti-SARS-CoV-2 Antibody Assay (Multiple Antibodies).

FIG. 18 is a bar graph showing exemplary assay response to various neutralizing and non-neutralizing antibodies against SARS-Cov-2 spike proteins. In details, 1nM monoclonal antibodies were used to compete with ACE2 proteins to bind SAR-Cov-2 S1 proteins. No antibodies were added in the control samples. The ΔCq refers to the qPCR cycle difference between the antibody-containing samples and control samples. The assay shows high specificity on differentiating neutralization capability of various antibodies.

### Example 5. Anti-SARS-CoV-2 Antibody Assay (R2B17 and R2B12).

FIG. 19A is a line graph showing measurement of the neutralizing capability of two anti-SARS-Cov2 monoclonal antibodies at different concentrations. R2B17 is a neutralizing antibody and R2B12 is a non-neutralizing antibody. The antibodies were diluted from 1 nM to 244 fM, and the control sample contains no antibodies. The ΔCt refers to the qPCR cycle difference between the antibody-containing samples and control samples.

### Example 6. Neutralizing Antibodies in SARS-CoV-2 Negative Donors, Recovered Patients, and Vaccinated Patients.

FIG. 19B is a plot showing assay performance according to an embodiment of the disclosure on Dried Blood Spot Samples collected from unvaccinated health donors (i.e. Negative), recovered patients from SARS-Cov2 infection and vaccinated patients (Moderna, two doses). For each dried blood spot sample, a 6 mm spot was punched and eluted using 500 µL elution buffer. The assay is demonstrated to be able to detect neutralizing antibody from all the recovered patient samples and vaccinated donor samples.

### Example 7. Specificity and Sensitivity of Anti-SARS-CoV-2 Antibody Assay.

FIGS. 21A-21C are line graphs showing a SPEAR-NAb specificity and sensitivity on anti-SARS COV2 neutralizing antibody detection. (FIG. 21A) Six anti-SARS COV2 monoclonal antibodies of different neutralizing capabilities were tested on ELISA and showed good and similar binding affinity with S1. SPEAR-NAb test was performed on these antibodies at 4-fold dilutions from 250nM and was able to differentiate anti-SARS COV2 antibodies with different neutralizing capabilities. (FIG. 21B) Antibodies with high reported neutralization capabilities (R2B12, R2B17 and 5B7D7) showed significantly higher signal inhibition than non-neutralizing counterparts (9B1E8, R1B8 and R2B12). (FIG. 21C) SPEAR has two-order increase in sensitivity in comparison to commercially-available CPASS assay.

### Example 8. Comparison of SPEAR-Nab, CPASS, and PsVNA Assays.

FIGS. 23A-23C are line graphs showing comparison of SPEAR-NAb vs CPASS vs PsVNA on neutralization measurement of negative, infected patient and vaccinated donor DBS samples. FIGS. 23A and 23B demonstrates excellent sensitivity (100%) and specificity (100%) of SPEAR-NAb in clearly differentiating vaccinated and infected patient samples from negative samples (uninfected and unvaccinated), with all vaccinated samples (n=21) above the grand mean of patient samples (n=19) and patient samples above the grand mean of negative subjects (n=22); whereas CPASS and PsVNA poorly differentiate vaccine from patient samples, and vaccine/ patient samples from negative subjects with DBS samples. FIG. 23C is the neutralizing titer measurement at 2-fold dilution on 10 vaccinated DBS samples. SPEAR-Nab is sensitive to measure changes across titers for all vaccine samples (up to 256x) and is capable of quantifying NT50 across vaccine samples. In contrast, CPass and PsVNA show much lower inhibition on 2x dilution and is unable to detect neutralizing antibodies at 8x dilution from all the DBS samples.

### Example 9. Concordance Measurement of SPEAR-NAb on Serum, Plasma, and DBS Samples.

FIG. 24 is a line graph showing concordance measurement of SPEAR-NAb on serum, plasma and DBS samples. Excellent concordance was observed between DBS and serum/plasma samples (R²=0.9788) by SPEAR-Nab. Shaded area shows 90% confidence interval of simple linear regression.

### Example 10. Neutralizing Titers Against Various SARS-CoV-2 Strains.

FIG. 25 is a plot and line graph showing SPEAR-NAb on measuring neutralization titers of vaccinated donor (n=37) against SARS-COV-2 wildtype (WT) and different variants identified from United Kingdom (UK), south Africa (SA) and Brazil (BZ) from a single DBS sample. Neutralizing titer at 50% inhibition (NT50) was interpolated from 8x 2-fold dilution on each sample. Red bar/line shows the geometric mean titer (GMT). The left panel shows that lower NT50 for variants (UK (B.1.1.7) > SA (B.1.351) > BZ (P.1)) in comparison to WT. Variant measurement per individual is shown on the right panel and similar decrease of NT50 on S1 variants is observed. Statistical significance test is Friedman test with subsequent Dunn's multiple comparison.

### Example 11. SPEAR-NAb Versus ELISA Pan-IgG Assays.

FIG. 26 is a line and plot graph showing SARS-COV2 Pan-IgG assay by SPEAR. SPEAR demonstrates high sensitivity in quantifying anti-SARS COV2 monoclonal antibodies (R2B17) by S1 conjugated SPEAR probes at low fM over a wide dynamic range, with four orders improvement in sensitivity in comparison to ELISA (left panel). DBS samples from vaccine subjects and recovered patients are perfectly differentiated (100% sensitivity and 100% specificity) from negative DBS samples (uninfected and unvaccinated donors).

### Example 12. S Protein Expression Assay.

FIG. 27 is plot graphs showing S protein expression assay by SPEAR. SPEAR works well in quantifying S proteins at low fM over a wide dynamic range using probes conjugated to two anti-SARS COV2 monoclonal antibodies of different S1 binding epitopes. SPEAR is able to detect fM copies of S1 from DBS samples of vaccinated donors (Day 2 or Day 3 post first dose) with excellent specificity.

### Example 13. Multiple Assays Conducted on Single DBS Sample.

FIG. 28 is a series of plot graphs showing multiple assays can be conducted on a single DBS sample. Longitudinal study of vaccinated subjects (Moderna/Pfizer) on S protein, Pan-IgG and neutralizing antibodies measurement by SPEAR/ SPEAR-Nab. DBS samples of four vaccinated subjects were measured over time-course before and after first and second inoculation. SPEAR shows distinct profiles of S1 over time where initial peak was observed roughly 3 days post the first dose for all four individuals and flatten when Pan-IgG and neutralizing antibody appeared to rise. Similar profile was observed for Pan-IgG and neutralizing antibody that first peak was observed on Day 14 after the first jab and gradually decrease over time and the second peak was observed roughly 9 days after the second dose.

### Example 14. Measurement of SPEAR-NAb on Serum, Plasma/Serum, and DBS.

FIGS. 29A and 29B are line graphs showing measurement of SPEAR-NAb on serum, plasma/serum (FIG. 29A) and DBS (FIG. 29B) samples. Excellent concordance was observed between plaque reduction neutralization test (PRNT) and SPEAR-Nab for the serum/plasma samples (R²=0.9390). Excellent concordance was observed between PRNT serum/plasma samples and SPEAR-Nab DBS samples (R²=0.9175). Shaded area shows 90% confidence interval of simple linear regression.

### Example 15: Performance of SPEAR non-enzymatic strand displacement approach in the detection of neutralizing and non-neutralizing antibodies

**FIG. 32** is bar graph showing detection of neutralizing and non-neutralizing antibodies according to an embodiment of the workflow shown in **FIGS. 31A and 31B**. The pair of the reporter probes RBD conjugates was allowed to incubate with or without SARS-CoV-2 monoclonal antibodies (R2B17 and R2B12 with high and trivial neutralizing capabilities against ACE2) at 4 °C overnight, followed by the addition of DNA conjugated ACE2 or free-floating displacement DNA strands at 0 nM, 50 nM or 200 nM to incubate at room temperature for 30 minutes before enzymatic reaction. In the absence of ACE2, neutralizing and non-neutralizing antibodies demonstrated comparable signals. By the addition of 50 nM and 200 nM ACE2, the cycle threshold (Ct) for non-neutralizing antibodies was highly declined and dropped almost to the background level, whereas Ct for neutralizing antibody was not as much as affected. Nearly 5 cycles difference, or 28-fold difference in copies, was observed between neutralizing and non-neutralizing antibodies at 200 nM ACE2. Such differentiation was in majority contributed by the colocalization of ACE2 and RBDs proven by the little leakage seen with free floating strands at the same concentrations.

All patents and other publications; including literature references, issued patents, published patent applications, and co-pending patent applications; cited throughout this application are expressly incorporated herein by reference for the purpose of providing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Various non-limiting expressions of the inventive concept are set out in the following clauses.

### CLAUSES

1. A method of detecting a target in a sample, the method comprising:
   a. contacting the sample with a first reporter probe and a blocking probe, wherein:
      i. the first reporter probe comprises a first target binding ligand linked to a first nucleic acid;
      ii. the blocking probe comprises a blocking ligand capable of directly or indirectly forming a complex with the first target binding ligand and linked to a blocking probe nucleic acid,

      wherein a portion of the first nucleic acid and a portion of the blocking probe nucleic acid are capable of hybridizing in the absence of the target,
      wherein binding of the target to the first reporter probe inhibits the formation of the complex, and
      wherein a portion of the first nucleic acid is capable of being extended at least two times to produce a nucleic acid record in the presence of the target and no more than one time in the absence of the target;
   b. providing a polymerase, thereby producing the nucleic acid record of an interaction between the first reporter probe and the target; and
   c. detecting the nucleic acid record.
2. The method of clause 1, wherein
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising a single-stranded toehold domain (a) at a first end of the double stranded nucleic acid; and
         2. a first nucleic acid second strand substantially complementary to the first strand, forming a double-stranded region (duplex region) with the first strand and comprising toehold domains (x and b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid comprising a toehold domain (a*) that is substantially complementary to the toehold domain (a) of the double-stranded nucleic acid of the first reporter probe, and wherein the target can simultaneously bind with the first reporter probe and the second reporter probe;
      iii. the blocking ligand is capable of directly or indirectly forming a complex with the first target binding ligand and/or the second target binding ligand and is linked to the blocking probe nucleic acid, wherein the blocking probe nucleic acid comprises a first toehold domain (a) that is substantially identical to the toehold domain (a) of the first reporter probe, and a second toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
3. The method of clause 1, wherein:
   a. the contacting the sample with the first reporter probe and the blocking probe comprises contacting the sample with the first reporter probe, a second reporter probe, and the blocking probe, wherein:
      i. the first nucleic acid comprises a double stranded nucleic acid, wherein the double-stranded nucleic acid comprises:
         1. a first nucleic acid first strand linked to the first target binding ligand and comprising single-stranded toehold domain (a) at a first end of the double stranded nucleic acid; and
         2. a first nucleic acid second strand substantially complementary to the first nucleic acid first strand, forming a double-stranded region (duplex region) with the first nucleic acid first strand and comprising toe hold domains (x and b) at a second end of the double-stranded nucleic acid, and
         wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the toehold domain (x) of the first reporter probe; a second nucleic acid second strand hybridized to the second nucleic acid first strand and comprising a second nucleic acid second hybridizing domain, substantially complementary to the second nucleic acid first hybridizing domain and linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or the second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
4. The method of clause 1, wherein:
   a. the contacting the sample with the first reporter probe and the blocking probe comprises contacting the sample with the first reporter probe, a second reporter probe, and the blocking probe, wherein:
      i. the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a single-stranded toehold domain (a) at an end distal from the toehold domain (x); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid first strand comprising a second nucleic acid first hybridizing domain linked to a toehold domain (x) that is substantially identical to the pairing domain (x) of the first reporter probe; a second nucleic acid second strand hybridized to the second nucleic acid first strand and comprising a second nucleic acid second hybridizing domain, substantially complementary to the second nucleic acid first hybridizing domain, linked to a primer binding domain (d*) linked to a toehold domain (a*) that is substantially complementary to the toehold domain (a) of the first reporter probe;
      iii. the blocking probe comprises a blocking ligand capable of directly or indirectly forming a complex with the first target binding ligand and/or second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.
5. A method of detecting a target in a sample, the method comprising:
   a. contacting the sample with a first reporter probe, wherein:
      i. the first reporter probe comprises a first target binding ligand linked to a first nucleic acid;
   b. contacting the sample with a blocking probe, wherein:
      i. the blocking probe comprises a blocking ligand capable of directly or indirectly forming a complex with the first target binding ligand and linked to a blocking probe nucleic acid,

      wherein a portion of the first nucleic acid and a portion of the blocking probe nucleic acid are capable of hybridizing in the absence of the target,
      wherein binding of the target to the first reporter probe inhibits the formation of the complex, and
      wherein a portion of the first nucleic acid is capable of being extended at least two times to produce a nucleic acid record in the absence of the target and no more than one time in the presence of the target;
   c. providing a polymerase, thereby producing the nucleic acid record of an interaction between the first nucleic acid and the blocking probe nucleic acid if said interaction is present; and
   d. detecting the presence or absence of the nucleic acid record, and wherein the absence of the nucleic acid record indicates the target is present in the sample.
6. The method of clause 5, wherein
   i. the first nucleic acid comprises a double stranded nucleic acid, wherein the double-stranded nucleic acid comprises:
      1. a first nucleic acid first strand linked to the first target binding ligand and comprising a single-stranded toehold domain (a) at a first end of the double stranded nucleic acid; and
      2. a first nucleic acid second strand substantially complementary to the first strand, forming a double-stranded region (duplex region) with the first nucleic acid first strand and comprising a toehold domain (b) at a second end of the double-stranded nucleic acid, and
         wherein the double-stranded nucleic acid comprises a first stopper molecule in the first nucleic acid first strand and a second stopper molecule in the first nucleic acid second strand in the duplex region;
   ii. the blocking probe nucleic acid comprises: a first toehold domain (a) that is substantially identical to the toehold domain (a) of the first reporter probe; and a primer binding domain (d*).
7. A method of detecting a target in a sample, the method comprising:
   a. contacting the sample with a first reporter probe and a second reporter probe, wherein:
      i. the first reporter probe comprises a first target binding ligand capable of binding the target and linked to first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe, wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a blocking ligand capable of directly or indirectly forming a complex with the first target binding ligand and linked to a blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
   c. providing a polymerase, thereby producing a nucleic acid record of an interaction between the first nucleic acid and the second nucleic acid if said interaction is present; and
   d. detecting the presence or absence of the nucleic acid record, and wherein the presence of the nucleic acid record indicates the target is present in the sample.
8. A method of detecting a target in a sample, the method comprising:
   a. contacting the sample with a first reporter probe and a second reporter probe, wherein:
      i. the first reporter probe comprises first target binding ligand capable of binding the target and linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
         1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
         2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
      ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
         wherein the toehold domain (b) of the first reporter probe comprises a nucleotide that is not present in the hybridizing domain (Lk1) of the first reporter probe and the hybridizing domain (Lk2) of the second probe, and
         wherein a portion of the first nucleic acid and a portion of the second nucleic acid are capable of hybridizing in the presence of the target;
   b. contacting the sample with a first blocking probe and a second blocking probe, wherein:
      i. the first blocking probe comprises a blocking ligand capable of directly or indirectly forming a complex with the first target binding ligand and linked to a blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the first blocking probe from forming a complex, ; and
      ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex;
   c. providing a polymerase and a first dNTP mix, where the first dNTP mix is substantially free of a dNTP complementary to the nucleotide present in the toehold domain (b) of the first reporter probe but not in the hybridizing domain (Lk1) of the first reporter probe and the hybridizing domain (Lk2) of the second reporter probe;
   d. providing a second dNTP mix. and optionally a polymerase, wherein the second dNTP mix comprises a dTNP complementary to the nucleotide present in the toehold domain (a) or (b) of the first reporter probe but not in the hybridizing domain (Lk1) of the first reporter probe and the hybridizing domain (Lk2) of the second reporter probe, thereby producing a nucleic acid record of an interaction between the first nucleic acid and the second nucleic acid if said interaction is present; and
   e. detecting the presence or absence of the nucleic acid record, and wherein the presence of the nucleic acid record indicates the target is present in the sample
9. The method of any one of clauses 1-8, wherein the providing the polymerase extends one or more of the nucleic acids.
10. The method of any one of clauses 1-9, wherein the method further comprises amplifying the nucleic acid record, if present, prior to detecting the presence or the absence of nucleic acid record.
11. The method of any one of clauses 1-10, wherein the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.
12. The method of any one of clauses 1-11, wherein the target is a biomolecule.
13. The method of any one of clauses 1-12, wherein the target is an antibody, a nucleic acid, a small molecule, or an antigen.
14. The method of any one of clauses 1-13, wherein the target is a neutralizing antibody, an antigen, a spike protein, a nucleic acid, or a small molecule.
15. The method of clause 14, wherein the antigen is comprised in a vaccine, or wherein the antigen is encoded by a vaccine.
16. The method of any one of clauses 1-15, wherein the sample is a biological sample.
17. A composition for detecting a target in a sample, the composition comprising:
   a. a first reporter probe comprising a target binding ligand linked to a first nucleic acid; and
   b. a blocking probe comprising a blocking ligand capable of directly or indirectly forming a complex with the target binding ligand of the first reporter probe and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
18. The composition of clause 17, further comprising:
   a second reporter probe comprising a second target binding ligand linked to a second nucleic acid,
   wherein a portion of the first nucleic acid and a portion of the second nucleic acid are capable of hybridizing, and
   wherein a portion of the second nucleic acid and a portion of the blocking probe nucleic acid are capable of hybridizing in the absence of the target.
19. A composition for detecting a target in a sample, the composition comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the absence of the target and no more than one time in presence of the target.
20. A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
21. The composition of clause 20, wherein the composition further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
22. The composition of clause 21, wherein the composition further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
23. A composition for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
24. The composition of clause 24, wherein the composition further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
25. The composition of clause 25, wherein the composition further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.
26. A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
27. The kit of clause 27, further comprising:
   a second reporter probe comprising a second target binding ligand linked to a second nucleic acid, wherein a portion of the first nucleic acid and a portion of the second nucleic acid are capable of hybridizing,
   wherein a portion of the second nucleic acid and a portion of the blocking probe nucleic acid are capable of hybridizing in the absence of the target.
28. A kit for detecting a target in a sample, the kit comprising:
   a. a first reporter probe comprising a first target binding ligand linked to a first nucleic acid; and
   b. a blocking probe comprising a blocking ligand capable of directly or indirectly forming a complex with the first target binding ligand and linked to a blocking probe nucleic acid, and

   wherein a portion of the first nucleic acid and a portion of the blocking probe nucleic acid are capable of hybridizing in the absence of the target,
   wherein binding of the target to the first reporter probe inhibits the formation of the complex, and
   wherein a portion of the first nucleic acid is capable of being extended at least two times to produce a nucleic acid record in the absence of the target and no more than one time in the presence of the target.
29. A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
30. The kit of clause 29, wherein the kit further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
31. The kit of clause 30, wherein the kit further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
32. A kit for detecting a target in a sample, comprising a first reporter probe and a first blocking probe, wherein:
   i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
      1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
      2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
   ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and

   wherein binding of the target to the first reporter probe inhibits the formation of the complex, and
   wherein a portion of the first nucleic acid is capable of being extended at least two times to produce a nucleic acid record in the presence of the target and no more than one time in the absence of the target.
33. The kit of clause 32, wherein the kit further comprises a second reporter probe, and wherein:
   the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.
34. The kit of clause 33, wherein the kit further comprises a second blocking probe, and wherein:
   the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.

## Claims

1. A method of detecting a target in a sample, the method comprising:
I. a first set of steps comprising:
a. contacting the sample with a first reporter probe, wherein:
i. the first reporter probe comprises a first target binding ligand linked to a first nucleic acid;
b. contacting the sample with a blocking probe, wherein:
i. the blocking probe comprises a blocking ligand capable of directly or indirectly forming a complex with the first target binding ligand and linked to a blocking probe nucleic acid,
wherein a portion of the first nucleic acid and a portion of the blocking probe nucleic acid are capable of hybridizing in the absence of the target,
wherein binding of the target to the first reporter probe inhibits the formation of the complex, and
wherein a portion of the blocking probe nucleic acid is capable of being extended at least two times to produce a nucleic acid record in the absence of the target and no more than one time in the presence of the target;
c. providing a polymerase, wherein the polymerase is a strand displacing polymerase, thereby producing the nucleic acid record of an interaction between the first nucleic acid and the blocking probe nucleic acid if said interaction is present; and
d. detecting the presence or absence of the nucleic acid record, and wherein the absence of the nucleic acid record indicates the target is present in the sample; or
II. a second set of steps comprising:
a. contacting the sample with a first reporter probe, wherein:
i. the first reporter probe comprises a first target binding ligand linked to a first nucleic acid, wherein the first nucleic acid comprises a double stranded nucleic acid, wherein the double-stranded nucleic acid comprises:
1. a first nucleic acid first strand linked to the first target binding ligand and comprising a single-stranded toehold domain (a) at a first end of the double stranded nucleic acid; and
2. a first nucleic acid second strand substantially complementary to the first strand, forming a double-stranded region (duplex region) with the first nucleic acid first strand and comprising a toehold domain (b) at a second end of the double-stranded nucleic acid, and
wherein the double-stranded nucleic acid comprises a first stopper molecule in the first nucleic acid first strand and a second stopper molecule in the first nucleic acid second strand in the duplex region;
b. contacting the sample with a blocking probe, wherein:
i. the blocking probe comprises a blocking ligand capable of directly or indirectly forming a complex with the first target binding ligand and linked to a blocking probe nucleic acid, wherein the blocking probe nucleic acid comprises: a first toehold domain (a*) that is substantially complementary to the toehold domain (a) of the first reporter probe; and a primer binding domain (d*),
wherein a portion of the first nucleic acid and a portion of the blocking probe nucleic acid are capable of hybridizing in the absence of the target,
wherein binding of the target to the first reporter probe inhibits the formation of the complex, and
wherein a portion of the blocking probe nucleic acid is capable of being extended at least two times to produce a nucleic acid record in the absence of the target and no more than one time in the presence of the target;
c. providing a polymerase, wherein the polymerase is a strand displacing polymerase, thereby producing the nucleic acid record of an interaction between the first nucleic acid and the blocking probe nucleic acid if said interaction is present; and
d. detecting the presence or absence of the nucleic acid record, and wherein the absence of the nucleic acid record indicates the target is present in the sample; or
III. a third set of steps comprising:
a. contacting the sample with a first reporter probe and a blocking probe, wherein:
i. the first reporter probe comprises a first target binding ligand linked to a first nucleic acid;
ii. the blocking probe comprises a blocking ligand capable of directly or indirectly forming a complex with the first target binding ligand and linked to a blocking probe nucleic acid,
wherein a portion of the first nucleic acid and a portion of the blocking probe nucleic acid are capable of hybridizing in the absence of the target,
wherein binding of the target to the first reporter probe inhibits the formation of the complex, and
wherein a portion of the first nucleic acid is capable of being extended at least two times to produce a nucleic acid record in the presence of the target and no more than one time in the absence of the target;
b. providing a polymerase, wherein the polymerase is a strand displacing polymerase, thereby producing the nucleic acid record of an interaction between the first reporter probe and the target; and
c. detecting the nucleic acid record; or
IV. a fourth set of steps comprising:
a. contacting the sample with a first reporter probe and a second reporter probe, wherein:
i. the first reporter probe comprises a first target binding ligand capable of binding the target and linked to first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
wherein a portion of the first nucleic acid and a portion of the second nucleic acid are capable of hybridizing in the presence of the target;
b. contacting the sample with a first blocking probe and a second blocking probe, wherein:
i. the first blocking probe comprises a blocking ligand capable of directly or indirectly forming a complex with the first target binding ligand and linked to a blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.
c. providing a polymerase, wherein the polymerase is a strand displacing polymerase, thereby producing a nucleic acid record of an interaction between the first nucleic acid and the second nucleic acid if said interaction is present; and
d. detecting the presence or absence of the nucleic acid record, and wherein the presence of the nucleic acid record indicates the target is present in the sample; or
V. a fifth set of steps comprising:
a. contacting the sample with a first reporter probe and a second reporter probe, wherein:
i. the first reporter probe comprises first target binding ligand capable of binding the target and linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the hybridizing domain (Lk1) and wherein the hybridizing domain (Lk1) is substantially complementary to the hybridizing domain (Lk1*); and
2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
ii. the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a toehold domain (a*) that is substantially complementary to the toehold domain (a) of the first reporter probe,
wherein the toehold domain (b) of the first reporter probe comprises a nucleotide that is not present in the hybridizing domain (Lk1) of the first reporter probe and the hybridizing domain (Lk2) of the second reporter probe, and
wherein a portion of the first nucleic acid and a portion of the second nucleic acid are capable of hybridizing in the presence of the target;
b. contacting the sample with a first blocking probe and a second blocking probe, wherein:
i. the first blocking probe comprises a blocking ligand capable of directly or indirectly forming a complex with the first target binding ligand and linked to a blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the first blocking probe from forming a complex; and
ii. the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex;
c. providing a polymerase and a first dNTP mix, wherein the polymerase is a strand displacing polymerase, and the first dNTP mix is substantially free of a dNTP complementary to the nucleotide present in the toehold domain (b) of the first reporter probe but not in the hybridizing domain (Lk1) of the first reporter probe and the hybridizing domain (Lk2) of the second reporter probe;
d. providing a second dNTP mix and optionally a polymerase, wherein the polymerase is a strand displacing polymerase, and the second dNTP mix comprises a dTNP complementary to the nucleotide present in the toehold domain (b) of the first reporter probe but not in the hybridizing domain (Lk1) of the first reporter probe and the hybridizing domain (Lk2) of the second reporter probe, thereby producing a nucleic acid record of an interaction between the first nucleic acid and the second nucleic acid if said interaction is present; and
e. detecting the presence or absence of the nucleic acid record, and wherein the presence of the nucleic acid record indicates the target is present in the sample.

2. The method of claim 1, wherein in the third set of steps:
a. the contacting the sample with the first reporter probe and the blocking probe comprises contacting the sample with the first reporter probe, a second reporter probe, and the blocking probe, wherein:
i. the first nucleic acid comprises a double stranded nucleic acid, wherein the double-stranded nucleic acid comprises:
1. a first nucleic acid first strand linked to the first target binding ligand and comprising a single-stranded toehold domain (a) at a first end of the double stranded nucleic acid; and
2. a first nucleic acid second strand substantially complementary to the first strand, forming a double-stranded region (duplex region) with the first strand and comprising toehold domains (x and b) at a second end of the double-stranded nucleic acid, and
wherein the double stranded nucleic acid comprises a first stopper molecule in the first nucleic acid first strand and a second stopper molecule in the first nucleic acid second strand in the duplex region;
ii. the second reporter probe comprises a second target binding ligand capable of binding the target and linked to a second nucleic acid comprising a toehold domain (a*) that is substantially complementary to the toehold domain (a) of the double-stranded nucleic acid of the first reporter probe, and wherein the target can simultaneously bind with the first reporter probe and the second reporter probe;
iii. the blocking ligand is capable of directly or indirectly forming a complex with the first target binding ligand and/or the second target binding ligand and is linked to the blocking probe nucleic acid, wherein the blocking probe nucleic acid comprises a first toehold domain (a) that is substantially identical to the toehold domain (a) of the first reporter probe, and a second toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.

3. The method of claim 1, wherein in the third set of steps:
a. the contacting the sample with the first reporter probe and the blocking probe comprises contacting the sample with the first reporter probe, a second reporter probe, and the blocking probe, wherein:
i. the first nucleic acid comprises a double stranded nucleic acid, wherein the double-stranded nucleic acid comprises:
1. a first nucleic acid first strand linked to the first target binding ligand and comprising single-stranded toehold domain (a) at a first end of the double stranded nucleic acid; and
2. a first nucleic acid second strand substantially complementary to the first nucleic acid first strand, forming a double-stranded region (duplex region) with the first nucleic acid first strand and comprising toe hold domains (x and b) at a second end of the double-stranded nucleic acid, and
wherein the double stranded nucleic acid comprises a first stopper molecule in the first nucleic acid first strand and a second stopper molecule in the first nucleic acid second strand in the duplex region;
ii. the second reporter probe comprises a second target binding ligand capable of binding the target and linked to a second nucleic acid first strand comprising a second nucleic acid first hybridizing domain linked to a toehold domain (x) that is substantially identical to the toehold domain (x) of the first reporter probe; a second nucleic acid second strand hybridized to the second nucleic acid first strand and comprising a second nucleic acid second hybridizing domain, substantially complementary to the second nucleic acid first hybridizing domain and linked to a primer binding domain (d*) linked to a toehold domain (a*) that is substantially complementary to the toehold domain (a) of the first reporter probe;
iii. the blocking probe comprises a blocking ligand capable of directly or indirectly forming a complex with the first target binding ligand and/or the second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.

4. The method of claim 1, wherein in the third set of steps:
a. the contacting the sample with the first reporter probe and the blocking probe comprises contacting the sample with the first reporter probe, a second reporter probe, and the blocking probe, wherein:
i. the first nucleic acid comprises: a linking strand linked to the first target binding ligand and comprising a first nucleic acid first hybridizing domain linked to a toehold domain (x); and a double stranded nucleic acid hybridized to the linking strand, wherein the double-stranded nucleic acid comprises:
1. a first nucleic acid first strand hybridized to the linking strand linked to the first target binding ligand and comprising a single-stranded toehold domain (a) at an end distal from the toehold domain (x); and
2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
ii. the second reporter probe comprises a second target binding ligand capable of binding the target and linked to a second nucleic acid first strand comprising a second nucleic acid first hybridizing domain linked to a toehold domain (x) that is substantially identical to the pairing domain (x) of the first reporter probe; a second nucleic acid second strand hybridized to the second nucleic acid first strand and comprising a second nucleic acid second hybridizing domain, substantially complementary to the second nucleic acid first hybridizing domain, linked to a primer binding domain (d*) linked to a toehold domain (a*) that is substantially complementary to the toehold domain (a) of the first reporter probe;
iii. the blocking probe comprises a blocking ligand capable of directly or indirectly forming a complex with the first target binding ligand and/or second target binding ligand and linked to the blocking probe nucleic acid, wherein the blocking probe nucleic acid comprises a toehold domain (x*) that is substantially complementary to the toehold domain (x) of the first reporter probe, and wherein binding of the target to the first reporter probe inhibits the first reporter probe and the blocking probe from forming a complex and/or wherein binding of the target to the second reporter probe inhibits the second reporter probe and the blocking probe from forming a complex.

5. The method of any one of claims 1-4, wherein in any one of the first set of steps, the second set of steps, the third set of steps, the fourth set of steps, and the fifth set of steps, the providing the polymerase extends one or more of the nucleic acids.

6. The method of any one of claims 1-5, wherein in any one of the first set of steps, the second set of steps, the third set of steps, the fourth set of steps, and the fifth set of steps the method further comprises amplifying the nucleic acid record, if present, prior to detecting the presence or the absence of nucleic acid record.

7. The method of any one of claims 1-6, wherein in any one of the first set of steps, the second set of steps, the third set of steps, the fourth set of steps, and the fifth set of steps, the first target binding ligand and/or the second target binding ligand and the blocking ligand are a receptor and ligand, nucleic acid and nucleic acid binding protein, antibody and antigen, antigen binding fragment of an antibody and antigen, antibody and Fc receptor, antibody and protein A, aptamer and its binding target, or a drug and its binding target.

8. The method of any one of claims 1-7, wherein in any one of the first set of steps, the second set of steps, the third set of steps, the fourth set of steps, and the fifth set of steps, the target is a biomolecule, optionally
wherein the target is an antibody, a nucleic acid, a small molecule, or an antigen, or optionally
wherein the target is a neutralizing antibody, an antigen, a spike protein, a nucleic acid, or a small molecule.

9. The method of claim 8, wherein the antigen is comprised in a vaccine, or wherein the antigen is encoded by a vaccine.

10. The method of any one of claims 1-9, wherein in any one of the first set of steps, the second set of steps, the third set of steps, the fourth set of steps, and the fifth set of steps, the sample is a biological sample.

11. The method of any one of claims 1-10, wherein the first reporter probe and the blocking probe are not in a complex prior to contacting with the sample and/or the second reporter probe and the blocking probe are not in a complex prior to contacting with the sample.

12. A composition for detecting a target in a sample, the composition comprising a strand displacing polymerase and:
I. a first set of elements comprising:
a. a first reporter probe comprising a first target binding ligand linked to a first nucleic acid; and
b. a blocking probe comprising a blocking ligand capable of directly or indirectly forming a complex with the first target binding ligand and linked to a blocking probe nucleic acid, and
wherein a portion of the first nucleic acid and a portion of the blocking probe nucleic acid are capable of hybridizing in the absence of the target,
wherein binding of the target to the first reporter probe inhibits the formation of the complex, and
wherein a portion of the blocking probe nucleic acid is capable of being extended at least two times to produce a nucleic acid record in the absence of the target and no more than one time in presence of the target; or
II. a second set of elements comprising:
a. a first reporter probe comprising a first target binding ligand linked to a first nucleic acid, wherein the first nucleic acid comprises a double stranded nucleic acid, wherein the double-stranded nucleic acid comprises:
1. a first nucleic acid first strand linked to the first target binding ligand and comprising a single-stranded toehold domain (a) at a first end of the double stranded nucleic acid; and
2. a first nucleic acid second strand substantially complementary to the first strand, forming a double-stranded region (duplex region) with the first nucleic acid first strand and comprising a toehold domain (b) at a second end of the double-stranded nucleic acid, and
wherein the double-stranded nucleic acid comprises a first stopper molecule in the first nucleic acid first strand and a second stopper molecule in the first nucleic acid second strand in the duplex region; and
b. a blocking probe comprising a blocking ligand capable of directly or indirectly forming a complex with the first target binding ligand and linked to a blocking probe nucleic acid, the blocking probe nucleic acid comprises: a first toehold domain (a*) that is substantially complementary to the toehold domain (a) of the first reporter probe; and a primer binding domain (d*), and
wherein a portion of the first nucleic acid and a portion of the blocking probe nucleic acid are capable of hybridizing in the absence of the target,
wherein binding of the target to the first reporter probe inhibits the formation of the complex, and
wherein a portion of the blocking probe nucleic acid is capable of being extended at least two times to produce a nucleic acid record in the absence of the target and no more than one time in presence of the target; or
III. a third set of elements comprising:
a. a first reporter probe comprising a first target binding ligand linked to a first nucleic acid; and
b. a blocking probe comprising a blocking ligand capable of directly or indirectly forming a complex with the first target binding ligand and linked to a blocking probe nucleic acid, and
wherein a portion of the first nucleic acid and a portion of the blocking probe nucleic acid are capable of hybridizing in the absence of the target,
wherein binding of the target to the first reporter probe inhibits the formation of the complex, and
wherein a portion of the first nucleic acid is capable of being extended at least two times to produce a nucleic acid record in the presence of the target and no more than one time in the absence of the target; or
IV. a fourth set of elements comprising a first reporter probe and a first blocking probe, wherein:
i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from the toehold domain (x) and wherein the hybridizing domain (Lk1) is substantially complementary to the hybridizing domain (Lk1*); and
2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region; and
ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is linked to a first blocking probe hybridizing domain (Lk1), where the toehold domain (x*) is substantially complementary to the toehold domain (x) of the first reporter probe, where the hybridizing domain (Lk1) is substantially complementary to the hybridizing domain (Lk1*) of the first reporter probe, and wherein binding of the target to the first reporter probe inhibits the first reporter probe and the first blocking probe from forming a complex; and
wherein binding of the target to the first reporter probe inhibits the formation of the complex, and
wherein a portion of the first nucleic acid is capable of being extended at least two times to produce a nucleic acid record in the presence of the target and no more than one time in the absence of the target; or
V. a fifth set of elements comprising a first reporter probe and a first blocking probe, wherein:
i. the first reporter probe comprises a first target binding ligand linked to a first nucleic acid, wherein the first nucleic acid comprises: a linking strand linked to the first target binding ligand and comprising a first nucleic acid first hybridizing domain (Lk1*) linked to a toehold domain (x); and a double stranded nucleic acid hybridized to the linking strand, wherein the double-stranded nucleic acid comprises:
1. a first nucleic acid first strand hybridized to the linking strand linked to the first target binding ligand and comprising a first nucleic acid second hybridizing domain (Lk1) linked to a single-stranded toehold domain (a), wherein the toehold domain (a) is at an end distal from hybridizing domain (Lk1) and wherein the hybridizing domain (Lk1) is substantially complementary to the hybridizing domain (Lk1*); and
2. a first nucleic acid second strand substantially complementary to the first nucleic acid first strand, forming a double-stranded region (duplex region) with the first nucleic acid first strand and comprising a toehold domain (b) at a second end of the double-stranded nucleic acid, and wherein the double stranded nucleic acid comprises a first stopper molecule in the first nucleic acid first strand and a second stopper molecule in the first nucleic acid second strand in the duplex region;
ii. the first blocking probe comprises a first blocking ligand capable of directly or indirectly forming a complex with the first target binding ligand and linked to a first blocking probe nucleic acid, wherein the first blocking probe nucleic acid comprises a toehold domain (x*) that is substantially complementary to the toehold domain (x) of the first reporter probe, and
wherein binding of the target to the first reporter probe inhibits the formation of the complex, and
wherein a portion of the first nucleic acid is capable of being extended at least two times to produce a nucleic acid record in the presence of the target and no more than one time in the absence of the target.

13. The composition of claim 12, wherein the third set of elements further comprises:
a second reporter probe comprising a second target binding ligand linked to a second nucleic acid,
wherein a portion of the first nucleic acid and a portion of the second nucleic acid are capable of hybridizing, and
wherein a portion of the second nucleic acid and a portion of the blocking probe nucleic acid are capable of hybridizing in the absence of the target.

14. The composition of claim 12, wherein the fourth set of elements further comprises a second reporter probe, and wherein:
the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.

15. The composition of claim 14, wherein the fourth set of elements further comprises a second blocking probe, and wherein:
the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is linked to a second blocking probe hybridizing domain (Lk2), where the toehold domain (x*) is substantially complementary to the toehold domain (x) of the second reporter probe, where the hybridizing domain (Lk2) is substantially complementary to the hybridizing domain (Lk2*) of the second reporter probe, and wherein binding of the target to the second reporter probe inhibits the second reporter probe and the second blocking probe from forming a complex.

16. The composition of claim 12, wherein the fifth set of elements further comprises a second reporter probe, and wherein:
the second reporter probe comprises a second target binding ligand capable of binding the target and linked to a second nucleic acid, wherein the second nucleic acid comprises a second nucleic acid first strand comprising a second nucleic acid first hybridizing domain (Lk2*) linked to a toehold domain (x) that is substantially identical to the toehold domain (x) of the first reporter probe; a second nucleic acid second strand hybridized to the second nucleic acid first strand and comprising a second nucleic acid second hybridizing domain (Lk2), substantially complementary to the second nucleic acid first hybridizing domain (Lk2*), linked to a primer binding domain (d*) linked to a toehold domain (a*) that is substantially complementary to the toehold domain (a) of the first reporter probe, and wherein a portion of the first nucleic acid and a portion of the second nucleic acid are capable of hybridizing in the presence of the target.

17. The composition of claim 16, wherein the fifth set of elements further comprises a second blocking probe, and wherein:
the second blocking probe comprises a second blocking ligand capable of directly or indirectly forming a complex with the second target binding ligand and linked to a second blocking probe nucleic acid, wherein the second blocking probe nucleic acid comprises a toehold domain (x*) that is substantially complementary to the toehold domain (x) of the second reporter probe, and wherein binding of the target to the second reporter probe inhibits the second reporter probe and the second blocking probe from forming a complex.

18. The method or composition of any one of claims 1-17, wherein the first target binding ligand and the second target binding ligand are the same.

19. The method or composition of any one of claims 1-17, wherein the first target binding ligand and the second target binding ligand are different.

20. The method or composition of any one of claims 1-19, wherein the second target binding ligand is a class specific antibody.
